# EUROPEAN PATENT APPLICATION

(11) **EP 4 467 572 A1**
(43) Date of publication of application: **27.11.2024**
(21) Application number: 23743191.1
(22) Date of filing: 13.01.2023
(51) Int. Cl.: C07K 17/00, C07K 17/02, C12N 7/02, C12N 1/21, C12N 15/12, C12N 15/63, C12Q 1/04, C12Q 1/70, C12P 21/00, B01J 20/24, C07K 14/705, C12M 1/34

(54) **MODIFIED RECOMBINANT ADENO-ASSOCIATED VIRUS (AAV)-BINDING PROTEIN AND METHOD OF ANALYSIS OF AAV BASED ON INFECTION ABILITY**

(30) Priority: 21.01.2022 JP 2022007922; 17.03.2022 JP 2022043222
(71) Applicant: Tosoh Corporation, Yamaguchi 746-8501 (JP)
(72) Inventor: KURIHARA, Kento, Ayase-shi, Kanagawa 252-1123 (JP); WATANABE, Kazuya, Ayase-shi, Kanagawa 252-1123 (JP); OMURA, Keita, Ayase-shi, Kanagawa 252-1123 (JP); YOSHIDA, Kouhei, Ayase-shi, Kanagawa 252-1123 (JP); MAKINO, Yuriko, Ayase-shi, Kanagawa 252-1123 (JP); TANAKA, Toru, Ayase-shi, Kanagawa 252-1123 (JP)
(74) Representative: Novagraaf International SA
(86) International application number: PCT/JP2023/000872
(87) International publication number: WO 2023/140197

(57) **Abstract**

The object is to provide a modified adeno-associated virus (AAV)-binding protein having an enhanced binding activity to AAV and a method for analyzing AAV contained in a sample based on a difference in the infectivity.

The object is achieved by substituting an amino acid residue at a specific position in regions corresponding to extracellular region domain 1 (PKD1) and domain 2 (PKD2) of KIAA0319L (UniProt No. Q8IZA0) with a different specific amino acid residue. The object is achieved by an AAV analysis method comprising a step of allowing AAV contained in a sample adsorbed by an adsorbent which comprises an insoluble carrier and a polypeptide comprising at least an amino acid sequence corresponding to extracellular region domain 1 or domain 2 of KIAA0319L immobilized on the carrier; and a step of eluting the AAV adsorbed by the adsorbent using an eluent.

## Description

### FIELD

The present invention relates to a protein having a binding activity to an adeno-associated virus (AAV) and a method for analyzing AAV. More specifically, the present invention relates to an improved AAV-binding protein having an enhanced binding activity to AAV and a method for analyzing AAV contained in a sample based on the strength of the infectivity.

### BACKGROUND

Adeno-associated virus (AAV) is a non-enveloped virus classified into the genus *Dependoparvovirus* of the family *Parvoviridae.* AAV capsid particles are composed of three types of proteins (VP1, VP2, and VP3), in which about 60 protein molecules are mixed in a ratio of VP1:VP2:VP3= about 1:1:10, collectively forming the shape of a regular icosahedron with a diameter of from 20 nm to 30 nm.

Natural AAV lacks autonomous proliferation capacity and replication depends on helper viruses such as adenoviruses and herpesviruses. In the presence of the helper viruses, the AAV genome is replicated within host cells to form complete AAV particles containing the AAV genome, and the AAV particles are released from the host cells. On the other hand, in the absence of the helper viruses, the AAV genome is in a state of being maintained in the episome or integrated into the host chromosome (latent state).

AAV can infect cells of a wide variety of species, including human, and also infect non-dividing cells that have been differentiated such as blood cells, muscles, and nerve cells, AAV is less concerned about side effects because it is not pathogenic to humans, AAV particles are physicochemically stable, and the like. Therefore, AAV is attracting attention for its utility value as a vector for gene introduction for the treatment of congenital genetic diseases.

It is reported that a substitution of an amino acid residue at specific position in AAV with a different amino acid residue makes a difference in the strength of the infectivity to cells (NPL 1). Therefore, when AAV is used as a vector for gene introduction, it is necessary to quickly and easily analyze differences in the strength of the infectivity thereof.

In general, production of gene recombinant AAV vector (hereinafter, also simply referred to as "AAV vector") is carried out by introducing nucleic acids that encode essential elements for AAV particle formation into cells to prepare cells capable of producing AAV (hereinafter also referred to as "AAV-producing cells") and culturing the cells to express the essential elements for AAV particle formation. The produced AAV vector is recovered and purified from AAV producing cells to obtain a therapeutic AAV vector formulation.

As a method for recovering and purifying an AAV vector from AAV producing cells, there is a method with affinity chromatography based on the binding affinity with AAV using an adsorbent comprising an insoluble carrier and an AAV-binding protein immobilized on the carrier, by which the vector can be recovered and purified from the solution comprising the AAV vector in which contaminants coexist. As a specific example, PL 1 achieves purification of the AAV vector with high purify by using a polypeptide with enhanced stability to heat, acid and alkali, comprising the extracellular domain 1 (PKD1) and domain 2 (PKD2) of KIAA0319L (UniProt No. Q8IZA0), wherein an amino acid residue at a specific position within these domains is substituted with a different amino acid residue, as an AAV-binding protein immobilized on the insoluble carrier (hereinafter, also simply referred to as "ligand protein").

It is reported that a substitution of an amino acid residue at a specific position in AAV with a different amino acid residue makes a difference in the strength of the infectivity to cells (NPL 1). Therefore, when AAV is used as a vector for gene introduction, it is necessary to quickly and easily analyze differences in the strength of the infectivity thereof. However, as disclosed in NPL 1, conventional analysis methods use cultured cells, which require a lot of time and effort.

### [CITATION LIST]

### [PATENT LITERATURE]

[PL 1] WO2021/106882

### [NON PATENT LITERATURE]

[NPL 1] Lochrie, M.A., et al., Journal of virology, 80(2), 821, 2006

### SUMMARY

### [TECHNICAL PROBLEM]

As described above, the adeno-associated virus (AAV)-binding protein used as a ligand protein in WO2021/106882 has enhanced stability to heat, acid, and alkali than the wild type (AAV-binding protein without amino acid substitution). On the other hand, further enhancement of the binding activity to AAV is necessary.

In addition, as described above, when AAV is used as a vector for gene introduction, it is necessary to quickly and easily analyze differences in the strength of the infectivity thereof. However, as disclosed in Lochrie, M.A., et al., Journal of virology, 80(2), 821, 2006 (NPL 1), conventional analysis methods use cultured cells, which require a lot of time and effort.

Therefore, the object of the invention is to provide a modified AAV-binding protein having an enhanced binding activity to an AAV, as well as providing a method for analyzing an adeno-associated virus contained in a sample based on the strength of the infectivity.

### [SOLUTION TO PROBLEM]

In order to solve the above problems, the present inventors conducted intensive studies. As a result, the inventors found that a substitution of a specific amino acid residue in the amino acid residues constituting an adeno-associated virus (AAV)-binding protein with a different amino acid residue enhances a binding activity to AAV, and analysis of an adeno-associated virus (AAV) contained in a sample using an adsorbent comprising an insoluble carrier and an AAV-binding protein immobilized on the carrier enables the analysis of the AAV based on the strength of the infectivity. This has led to the completion of the invention.

The present invention encompasses the following aspects of [1] to [14].
[1] An adeno-associated virus (AAV)-binding protein, which is selected from any of the following (i) to (iii):
   (i) an AAV-binding protein comprising at least amino acid residues from serine at position 312 to aspartic acid at position 500 of the amino acid sequence set forth in SEQ ID NO: 1, wherein at least any one of the following amino acid substitutions (1) to (5) is present at the amino acid residues from position 312 to position 500;
      (1) a substitution of alanine with valine at position 330 of SEQ ID NO: 1,
      (2) a substitution of alanine with any one of cysteine, phenylalanine, leucine, arginine, tryptophan, or tyrosine at position 330 of SEQ ID NO: 1,
      (3) a substitution of tyrosine with cysteine at position 331 of SEQ ID NO: 1,
      (4) a substitution of valine with tryptophan or tyrosine at position 332 of SEQ ID NO: 1,
      (5) a substitution of leucine with cysteine at position 333 of SEQ ID NO: 1;
   (ii) an AAV-binding protein comprising at least amino acid residues from serine at position 312 to aspartic acid at position 500 of the amino acid sequence set forth in SEQ ID NO: 1, wherein the at least any one of the amino acid substitutions (1) to (5) is present at the amino acid residues from position 312 to position 500, wherein one or more substitutions, deletions, insertions, and additions of one or several amino acid residues are further present at one or several positions other than the amino acid substitutions set forth in (1) to (5), and wherein the AAV-binding protein has AAV-binding activity; and
   (iii) an AAV-binding protein comprising an amino acid sequence having 70% or more homology to an entire amino acid sequence in which the at least any one of the amino acid substitutions (1) to (5) is present in an amino acid sequence ranging from serine at position 312 to aspartic acid at position 500 of the amino acid sequence set forth in SEQ ID NO: 1, wherein the at least any one of the amino acid substitutions remains in the amino acid sequence, and wherein the AAV-binding protein has AAV-binding activity.
[2] The AAV-binding protein according to [1], which is selected from any of the following (iv) to (vi):
   (iv) an AAV-binding protein comprising at least amino acid residues from serine at position 312 to aspartic acid at position 500 of the amino acid sequence set forth in SEQ ID NO: 1, wherein at least the following amino acid substitution (1) is present at the amino acid residues from position 312 to position 500:
      (1) a substitution of alanine with valine at position 330 of SEQ ID NO: 1;
   (v) an AAV-binding protein comprising at least amino acid residues from serine at position 312 to aspartic acid at position 500 of the amino acid sequence set forth in SEQ ID NO: 1, wherein the at least amino acid substitution (1) is present at the amino acid residues from position 312 to position 500, wherein one or more substitutions, deletions, insertions, and additions of one or several amino acid residues are further present at one or several positions other than the amino acid substitution set forth in (1), and wherein the AAV-binding protein has AAV-binding activity; and
   (vi) an AAV-binding protein comprising an amino acid sequence having 70% or more homology to an entire amino acid sequence in which the at least the amino acid substitution (1) is present in an amino acid sequence ranging from serine at position 312 to aspartic acid at position 500 of the amino acid sequence set forth in SEQ ID NO: 1, wherein the amino acid substitution (1) remains in the amino acid sequence, and wherein the AAV-binding protein has AAV-binding activity.
[3] The AAV-binding protein according to [1], wherein the following 10 amino acid substitutions are further present:
   a substitution of valine with aspartic acid at position 317 of SEQ ID NO: 1,
   a substitution of tyrosine with serine at position 342 of SEQ ID NO: 1,
   a substitution of lysine with glutamic acid at position 362 of SEQ ID NO: 1,
   a substitution of lysine with asparagine at position 371 of SEQ ID NO: 1,
   a substitution of valine with alanine at position 381 of SEQ ID NO: 1,
   a substitution of isoleucine with valine at position 382 of SEQ ID NO: 1,
   a substitution of glycine with serine at position 390 of SEQ ID NO: 1,
   a substitution of lysine with glutamic acid at position 399 of SEQ ID NO: 1,
   a substitution of serine with arginine at position 476 of SEQ ID NO: 1, and
   a substitution of asparagine with aspartic acid at position 487 of SEQ ID NO: 1.
[4] The AAV-binding protein according to [3], which is selected from any of the following (vii) to (ix):
   (vii) an AAV-binding protein comprising at least amino acid residues from serine at position 25 to aspartic acid at position 213 of the amino acid sequence set forth in any one of SEQ ID NOS: 15 to 25;
   (viii) an AAV-binding protein comprising at least amino acid residues from serine at position 25 to aspartic acid at position 213 of the amino acid sequence set forth in any one of SEQ ID NOS: 15 to 25, wherein one or more substitutions, deletions, insertions, and additions of one or several amino acid residues are further present at one or several positions other than said amino acid substitution(s) of said amino acid sequence in the amino acid residues from position 25 to position 213, and wherein the AAV-binding protein has AAV-binding activity; and
   (ix) an AAV-binding protein comprising at least amino acid residues from serine at position 25 to aspartic acid at position 213 of the amino acid sequence set forth in any one of SEQ ID NOS: 15 to 25, except for having 70% or more homology to said amino acid sequence from serine at position 25 to aspartic acid at position 213, wherein said amino acid substitution(s) of said amino acid sequence remain, and wherein the AAV-binding protein has AAV-binding activity.
[5] The AAV-binding protein according to [3], wherein at least any one of the following amino acid substitutions (I) to (LXXXVII) is further present:
   (I) a substitution of serine with proline at position 312 of SEQ ID NO: 1,
   (II) a substitution of alanine with valine at position 313 of SEQ ID NO: 1,
   (III) a substitution of glutamic acid with glycine or valine at position 315 of SEQ ID NO: 1,
   (IV) a substitution of glutamine with arginine at position 318 of SEQ ID NO: 1,
   (V) a substitution of isoleucine with valine at position 319 of SEQ ID NO: 1,
   (VI) a substitution of lysine with methionine or asparagine at position 323 of SEQ ID NO: 1,
   (VII) a substitution of asparagine with histidine at position 324 of SEQ ID NO: 1,
   (VIII) a substitution of glutamic acid with lysine at position 325 of SEQ ID NO: 1,
   (IX) a substitution of valine with glutamic acid or alanine at position 326 of SEQ ID NO: 1,
   (X) a substitution of glutamine with leucine at position 327 of SEQ ID NO: 1,
   (XI) a substitution of leucine with any one of glutamine, arginine and proline at position 328 of SEQ ID NO: 1,
   (XII) a substitution of asparagine with tyrosine or serine at position 329 of SEQ ID NO: 1,
   (XIII) a substitution of valine with glutamic acid or alanine at position 332 of SEQ ID NO: 1,
   (XIV) a substitution of leucine with proline or methionine at position 333 of SEQ ID NO: 1,
   (XV) a substitution of glutamine with leucine or histidine at position 334 of SEQ ID NO: 1,
   (XVI) a substitution of glutamic acid with valine at position 335 of SEQ ID NO: 1,
   (XVII) a substitution of proline with glutamine at position 337 of SEQ ID NO: 1,
   (XVIII) a substitution of lysine with glutamic acid or arginine at position 338 of SEQ ID NO: 1,
   (XIX) a substitution of glycine with glutamic acid at position 339 of SEQ ID NO: 1,
   (XX) a substitution of glutamic acid with aspartic acid at position 340 of SEQ ID NO: 1,
   (XXI) a substitution of threonine with alanine at position 341 of SEQ ID NO: 1,
   (XXII) a substitution of threonine with any one of proline, alanine and serine at position 343 of SEQ ID NO: 1,
   (XXIII) a substitution of aspartic acid with glycine at position 345 of SEQ ID NO: 1,
   (XXIV) a substitution of isoleucine with threonine at position 349 of SEQ ID NO: 1,
   (XXV) a substitution of threonine with alanine at position 350 of SEQ ID NO: 1,
   (XXVI) a substitution of aspartic acid with valine at position 354 of SEQ ID NO: 1,
   (XXVII) a substitution of tyrosine with histidine at position 355 of SEQ ID NO: 1,
   (XXVIII) a substitution of methionine with any one of isoleucine, leucine and valine at position 359 of SEQ ID NO: 1,
   (XXIX) a substitution of glutamic acid with aspartic acid at position 360 of SEQ ID NO: 1,
   (XXX) a substitution of glycine with glutamic acid at position 361 of SEQ ID NO: 1,
   (XXXI) a substitution of isoleucine with threonine or proline at position 366 of SEQ ID NO: 1,
   (XXXII) a substitution of leucine with valine at position 367 of SEQ ID NO: 1,
   (XXXIII) a substitution of lysine with any one of asparagine, arginine and glutamic acid at position 368 of SEQ ID NO: 1,
   (XXXIV) a substitution of leucine with glutamine or proline at position 369 of SEQ ID NO: 1,
   (XXXV) a substitution of leucine with proline at position 376 of SEQ ID NO: 1,
   (XXXVI) a substitution of phenylalanine with tyrosine at position 379 of SEQ ID NO: 1,
   (XXXVII) a substitution of lysine with asparagine or arginine at position 380 of SEQ ID NO: 1,
   (XXXVIII) a substitution of glutamic acid with valine at position 384 of SEQ ID NO: 1,
   (XXXIX) a substitution of alanine with threonine at position 388 of SEQ ID NO: 1,
   (XL) a substitution of histidine with any one of asparagine, aspartic acid, arginine and leucine at position 389 of SEQ ID NO: 1,
   (XLI) a substitution of glycine with arginine at position 392 of SEQ ID NO: 1,
   (XLII) a substitution of valine with alanine or aspartic acid at position 394 of SEQ ID NO: 1,
   (XLII) a substitution of asparagine with aspartic acid at position 395 of SEQ ID NO: 1,
   (XLIII) a substitution of valine with glycine at position 396 of SEQ ID NO: 1,
   (XLIV) a substitution of valine with alanine at position 398 of SEQ ID NO: 1,
   (XLV) a substitution of arginine with serine or histidine at position 403 of SEQ ID NO: 1,
   (XLVI) a substitution of lysine with alanine at position 404 of SEQ ID NO: 1,
   (XLVII) a substitution of arginine with serine at position 406 of SEQ ID NO: 1,
   (XLVIII) a substitution of isoleucine with asparagine at position 409 of SEQ ID NO: 1,
   (XLIX) a substitution of isoleucine with threonine or leucine at position 411 of SEQ ID NO: 1, (L) a substitution of valine with isoleucine at position 412 of SEQ ID NO: 1,
   (LI) a substitution of phenylalanine with serine at position 416 of SEQ ID NO: 1,
   (LII) a substitution of isoleucine with phenylalanine or valine at position 419 of SEQ ID NO: 1,
   (LIII) a substitution of serine with threonine at position 420 of SEQ ID NO: 1,
   (LIV) a substitution of leucine with proline at position 421 of SEQ ID NO: 1,
   (LV) a substitution of proline with serine at position 422 of SEQ ID NO: 1,
   (LVI) a substitution of threonine with alanine at position 423 of SEQ ID NO: 1,
   (LVII) a substitution of serine with glycine at position 425 of SEQ ID NO: 1,
   (LVIII) a substitution of isoleucine with valine at position 428 of SEQ ID NO: 1,
   (LIX) a substitution of glycine with alanine at position 430 of SEQ ID NO: 1,
   (LX) a substitution of threonine with alanine at position 434 of SEQ ID NO: 1,
   (LXI) a substitution of aspartic acid with asparagine at position 437 of SEQ ID NO: 1,
   (LXII) a substitution of valine with alanine at position 440 of SEQ ID NO: 1,
   (LXIII) a substitution of histidine with tyrosine or arginine at position 443 of SEQ ID NO: 1,
   (LXIV) a substitution of glutamic acid with aspartic acid at position 446 of SEQ ID NO: 1,
   (LXV) a substitution of lysine with glutamic acid or asparagine at position 448 of SEQ ID NO: 1,
   (LXVI) a substitution of leucine with isoleucine at position 451 of SEQ ID NO: 1,
   (LXVII) a substitution of glutamic acid with lysine at position 453 of SEQ ID NO: 1,
   (LXVIII) a substitution of glutamic acid with glycine at position 454 of SEQ ID NO: 1,
   (LXIX) a substitution of lysine with glutamic acid or arginine at position 455 of SEQ ID NO: 1,
   (LXX) a substitution of lysine with asparagine or glutamic acid at position 464 of SEQ ID NO: 1,
   (LXXI) a substitution of lysine with glutamine or glutamic acid at position 467 of SEQ ID NO: 1,
   (LXXII) a substitution of valine with glutamic acid at position 469 of SEQ ID NO: 1,
   (LXXIII) a substitution of glycine with cysteine at position 471 of SEQ ID NO: 1,
   (LXXIV) a substitution of asparagine with tyrosine or aspartic acid at position 472 of SEQ ID NO: 1,
   (LXXV) a substitution of tyrosine with histidine at position 473 of SEQ ID NO: 1,
   (LXXVI) a substitution of threonine with alanine at position 478 of SEQ ID NO: 1,
   (LXXVII) a substitution of valine with alanine at position 479 of SEQ ID NO: 1,
   (LXXVIII) a substitution of valine with aspartic acid or alanine at position 480 of SEQ ID NO: 1,
   (LXXIX) a substitution of serine with threonine at position 482 of SEQ ID NO: 1,
   (LXXX) a substitution of glycine with serine at position 484 of SEQ ID NO: 1,
   (LXXXI) a substitution of threonine with serine at position 486 of SEQ ID NO: 1,
   (LXXXII) a substitution of serine with threonine or phenylalanine at position 488 of SEQ ID NO: 1,
   (LXXXIII) a substitution of threonine with alanine or serine at position 489 of SEQ ID NO: 1,
   (LXXXIV) a substitution of alanine with serine at position 491 of SEQ ID NO: 1,
   (LXXXV) a substitution of asparagine with aspartic acid at position 492 of SEQ ID NO: 1,
   (LXXXVI) a substitution of asparagine with tyrosine or aspartic acid at position 496 of SEQ ID NO: 1, and
   (LXXXVII) a substitution of aspartic acid with glycine at position 500 of SEQ ID NO: 1.
[6] The AAV-binding protein according to [5], which is selected from any of the following (x) to (xii):
   (x) an AAV-binding protein comprising at least amino acid residues from serine at position 25 to aspartic acid at position 213 of the amino acid sequence set forth in any one of SEQ ID NOS: 54 to 63, 65, 83, 85, 87, 88, and 90 to 92;
   (xi) an AAV-binding protein comprising at least amino acid residues from serine at position 25 to aspartic acid at position 213 of the amino acid sequence set forth in any one of SEQ ID NOS: 54 to 63, 65, 83, 85, 87, 88, and 90 to 92, wherein one or more substitutions, deletions, insertions, and additions of one or several amino acid residues are further present at one or several positions other than said amino acid substitution(s) of said amino acid sequence in the amino acid residues from position 25 to position 213, and wherein the AAV-binding protein has AAV-binding activity; and
   (xii) an AAV-binding protein comprising at least amino acid residues from serine at position 25 to aspartic acid at position 213 of the amino acid sequence set forth in any one of SEQ ID NOS: 54 to 63, 65, 83, 85, 87, 88, and 90 to 92, except for having 70% or more homology to said amino acid sequence from serine at position 25 to aspartic acid at position 213, wherein said amino acid substitution(s) of said amino acid sequence remain, and wherein the AAV-binding protein has AAV-binding activity.
[7] A polynucleotide encoding the AAV-binding protein according to any of [1] to [6].
[8] An expression vector comprising the polynucleotide according to [7].
[9] A transformant obtained by transforming *Escherichia coli* with the expression vector according to [8].
[10] A method for producing an AAV-binding protein, comprising a step of allowing an AAV-binding protein to be expressed by culturing the transformant according to [9] and a step of recovering the AAV-binding protein expressed from the obtained culture.
[11] An AAV adsorbent comprising an insoluble carrier and the AAV-binding protein according to any one of [1] to [6] which is immobilized on the carrier.
[12] A column comprising the AAV adsorbent according to [11].
[13] A method for purifying AAV, comprising a step of adding a solution containing AAV to the column according to [12] to allow the AAV to be adsorbed by said adsorbent and a step of eluting the AAV adsorbed by said adsorbent using an eluate.
[14] A method for analyzing AAV contained in a sample based on the strength of the infectivity, comprising a step of adding the sample containing AAV to the column according to [12] to allow the AAV to be adsorbed by said adsorbent, and a step of eluting the AAV adsorbed by said adsorbent using an eluate.

Hereinafter, the invention will be described in detail.

The term infectivity as used herein refers to the ability of a virus such as AAV to infect host cells. Infectivity may be expressed by using a known index indicating the ability to infect, as a proportion or ratio of the index of the variant (amino acid substitution product) to that of the wild type. Examples of the index indicating the ability to infect include the infection rate when a virus infects host cells, the ability of a virus to bind to a receptor on the cell membrane of host cells, the level of the activity of a gene contained therein, the intensity of luminescence inside or outside an animal of the protein encoded by the gene, such as GFP (for example, Fig.6 of Amanda M. Dudek, et al., Journal of virology, 92(7), e02213-17,2018), and such expression levels of markers indicating the effectiveness of a virus such as AAV. Host cells generally refer to various cells such as HEK293 and HT-1080, cells of animals such as mice and monkeys, which can take any form, including floating cells, adherent cells, and living cells. The concentration for infecting a virus such as AAV can be changed as appropriate so that the difference can be easily seen. Although the infectivity of an AAV variant is not limited, for example, as described in NPL 1, the infectivity can be obtained by infecting host cells such as human HepG2 cells with the AAV variant and the wild type AAV, measuring the infection rates thereof, and calculating the ratio of the infection rate of the AAV variant relative to the infection rate of the wild type AAV being set to 100. Alternatively, the infectivity of the variant may also be determined as a relative value to the infectivity of the wild type.

The AAV binding protein described herein is a protein comprising at least amino acid residues from serine (Ser) at position 312 to aspartic acid (Asp) at position 500, which are the regions corresponding to the extracellular domain 1 (PKD1) and domain 2 (PKD2) in the amino acid sequence of KIAA0319L (UniProt No. Q8IZA0) set forth in SEQ ID NO: 1, wherein amino acid substitution(s) is(are) present at specific position(s) of the amino acid residues from position 312 to position 500. Therefore, the AAV-binding protein of the invention may comprise: all or part of the other extracellular domains (domain 3 (PKD3), domain 4 (PKD4), and domain 5 (PKD5)) on the C-terminal side of the protein; all or part of a signal sequence such as the MANSC (motif at N terminus with seven cysteines) domain or cysteine-rich region on the N-terminal side of PKD1; and all or part of the transmembrane and intracellular domains on the N-terminal and/or C-terminal side of the extracellular domain.

The amino acid substitutions at specific position(s) is(are) specifically at least any one of amino acid substitutions of:
(1) Ala330Val (this notation indicates a substitution of alanine at position 330 of SEQ ID NO: 1 with valine; the same applies hereinafter),
(2) any one of Ala330Cys, Ala330Phe, Ala330Leu, Ala330Arg, Ala330Trp, and Ala330Tyr,
(3) Tyr331Cys,
(4) Val332Trp or Val332Tyr, and
(5) Leu333Cys,
in the amino acid sequence set forth in SEQ ID NO: 1. Such amino acid substitutions would lead to the enhancement of the binding activity to AAV.

Among them, (1) Ala330Val is an amino acid substitution which significantly enhances the binding activity to AAV, and significantly suppresses the generation of decomposition products derived from an AAV-binding protein at the time of the production of the AAV-binding protein with genetic engineering techniques using recombinant E. coli, and significantly improves the production efficiency of the AAV-binding protein using the recombinant E. coli. For that reason, preferable aspect of the AAV-binding protein of the invention is an AAV binding protein wherein at least the amino acid substitution of (1) Ala330Val is present.

The number of amino acid substitutions set forth in (1) to (5) is not particularly limited. In other words, only any one of the amino acid substitutions set forth in the (1) to (5) may be present, or two or more of the amino acid substitutions set forth in the (1) to (5) may be present. However, since the amino acid substitutions set forth in (1) and (2) are substitutions that occur at the same position, even if two or more amino acid substitutions occur, either (1) or (2) can occur.

The AAV to enhance the binding activity is not particularly limited, and it may be a naturally occurring AAV or an artificially produced AAV. Examples of the naturally occurring AAV include serotype 1 (AAV1), serotype 2 (AAV2), serotype 3 (AAV3), serotype 5 (AAV5), serotype 6 (AAV6), serotype 7 (AAV7), serotype 8 (AAV8), serotype 9 (AAV9), serotype 10 (AAV10), serotype 11 (AAV11), serotype 12 (AAV12), and serotype 13 (AAV13). In addition, examples of the artificially produced AAV include AAVrh8, AAVrh10, and chimeric AAVs having two or more characteristics (cell tropism and infectivity) of these serotypes.

In (ii), (v), (viii), and (xi) described above, the expression "one or several" refers to, for example, from 1 to 50, from 1 to 30, from 1 to 20, from 1 to 10, from 1 to 9, from 1 to 8, from 1 to 7, from 1 to 6, from 1 to 5, from 1 to 4, from 1 to 3, from 1 to 2, or 1, although it depends on positions of amino acid substitutions and types of amino acid residues in the protein conformation of the AAV-binding protein.

Examples of the substitutions, deletions, insertions, or additions described in (ii), (v), (viii), and (xi) above include substitutions of amino acid residues disclosed in WO2021/106882. Among them, particularly preferable aspect of the AAV-binding protein of the invention is an AAV-binding protein wherein at least amino acid substitutions of Val317Asp, Tyr342Ser, Lys362Glu, Lys371Asn, Val381Ala, Ile382Val, Gly390Ser, Lys399Glu, Ser476Arg and Asn487Asp are present, as the stability to heat, acid and alkali is particularly enhanced.

Examples of the particularly preferable aspect described above include AAV-binding proteins set forth in the following (A) to (K).
(A) An AAV-binding protein comprising amino acid residues from serine at position 312 to aspartic acid at position 500 of the amino acid sequence set forth in SEQ ID NO: 1, wherein at least amino acid substitutions of Val317Asp, Ala330Val, Tyr342Ser, Lys362Glu, Lys371Asn, Val381Ala, Ile382Val, Gly390Ser, Lys399Glu, Ser476Arg and Asn487Asp are present at the amino acid residues from position 312 to position 500.
(B) An AAV-binding protein comprising amino acid residues from serine at position 312 to aspartic acid at position 500 of the amino acid sequence set forth in SEQ ID NO: 1, wherein at least amino acid substitutions of Val317Asp, Ala330Cys, Tyr342Ser, Lys362Glu, Lys371Asn, Val381Ala, Ile382Val, Gly390Ser, Lys399Glu, Ser476Arg and Asn487Asp are present at the amino acid residues from position 312 to position 500.
(C) An AAV-binding protein comprising amino acid residues from serine at position 312 to aspartic acid at position 500 of the amino acid sequence set forth in SEQ ID NO: 1, wherein at least amino acid substitutions of Val317Asp, Ala330Phe, Tyr342Ser, Lys362Glu, Lys371Asn, Val381Ala, Ile382Val, Gly390Ser, Lys399Glu, Ser476Arg and Asn487Asp are present at the amino acid residues from position 312 to position 500.
(D) An AAV-binding protein comprising amino acid residues from serine at position 312 to aspartic acid at position 500 of the amino acid sequence set forth in SEQ ID NO: 1, wherein at least amino acid substitutions of Val317Asp, Ala330Leu, Tyr342Ser, Lys362Glu, Lys371Asn, Val381Ala, Ile382Val, Gly390Ser, Lys399Glu, Ser476Arg and Asn487Asp are present at the amino acid residues from position 312 to position 500.
(E) An AAV-binding protein comprising amino acid residues from serine at position 312 to aspartic acid at position 500 of the amino acid sequence set forth in SEQ ID NO: 1, wherein at least amino acid substitutions of Val317Asp, Ala330Arg, Tyr342Ser, Lys362Glu, Lys371Asn, Val381Ala, Ile382Val, Gly390Ser, Lys399Glu, Ser476Arg and Asn487Asp are present at the amino acid residues from position 312 to position 500.
(F) An AAV-binding protein comprising amino acid residues from serine at position 312 to aspartic acid at position 500 of the amino acid sequence set forth in SEQ ID NO: 1, wherein at least amino acid substitutions of Val317Asp, Ala330Trp, Tyr342Ser, Lys362Glu, Lys371Asn, Val381Ala, Ile382Val, Gly390Ser, Lys399Glu, Ser476Arg and Asn487Asp are present at the amino acid residues from position 312 to position 500.
(G) An AAV-binding protein comprising amino acid residues from serine at position 312 to aspartic acid at position 500 of the amino acid sequence set forth in SEQ ID NO: 1, wherein at least amino acid substitutions of Val317Asp, Ala330Tyr, Tyr342Ser, Lys362Glu, Lys371Asn, Val381Ala, Ile382Val, Gly390Ser, Lys399Glu, Ser476Arg and Asn487Asp are present at the amino acid residues from position 312 to position 500.
(H) An AAV-binding protein comprising amino acid residues from serine at position 312 to aspartic acid at position 500 of the amino acid sequence set forth in SEQ ID NO: 1, wherein at least amino acid substitutions of Val317Asp, Tyr331Cys, Tyr342Ser, Lys362Glu, Lys371Asn, Val381Ala, Ile382Val, Gly390Ser, Lys399Glu, Ser476Arg and Asn487Asp are present at the amino acid residues from position 312 to position 500.
(I) An AAV-binding protein comprising amino acid residues from serine at position 312 to aspartic acid at position 500 of the amino acid sequence set forth in SEQ ID NO: 1, wherein at least amino acid substitutions of Val317Asp, Val332Trp, Tyr342Ser, Lys362Glu, Lys371Asn, Val381Ala, Ile382Val, Gly390Ser, Lys399Glu, Ser476Arg and Asn487Asp are present at the amino acid residues from position 312 to position 500.
(J) An AAV-binding protein comprising amino acid residues from serine at position 312 to aspartic acid at position 500 of the amino acid sequence set forth in SEQ ID NO: 1, wherein at least amino acid substitutions of Val317Asp, Val332Tyr, Tyr342Ser, Lys362Glu, Lys371Asn, Val381Ala, Ile382Val, Gly390Ser, Lys399Glu, Ser476Arg and Asn487Asp are present at the amino acid residues from position 312 to position 500.
(K) An AAV-binding protein comprising amino acid residues from serine at position 312 to aspartic acid at position 500 of the amino acid sequence set forth in SEQ ID NO: 1, wherein at least amino acid substitutions of Val317Asp, Leu333Cys, Tyr342Ser, Lys362Glu, Lys371Asn, Val381Ala, Ile382Val, Gly390Ser, Lys399Glu, Ser476Arg and Asn487Asp are present at the amino acid residues from position 312 to position 500.

Specific examples of the particularly preferable aspect described above include AAV-binding proteins as follows.
An AAV-binding protein comprising at least an amino acid sequence ranging from serine at position 25 to aspartic acid at position 213 of the amino acid sequence set forth in SEQ ID NO: 19, which is one aspect of the AAV-binding protein set forth in (A).
An AAV-binding protein comprising at least an amino acid sequence ranging from serine at position 25 to aspartic acid at position 213 of the amino acid sequence set forth in SEQ ID NO: 15, which is one aspect of the AAV-binding protein set forth in (B).
An AAV-binding protein comprising at least an amino acid sequence ranging from serine at position 25 to aspartic acid at position 213 of the amino acid sequence set forth in SEQ ID NO: 16, which is one aspect of the AAV-binding protein set forth in (C).
An AAV-binding protein comprising at least an amino acid sequence ranging from serine at position 25 to aspartic acid at position 213 of the amino acid sequence set forth in SEQ ID NO: 17, which is one aspect of the AAV-binding protein set forth in (D).
An AAV-binding protein comprising at least an amino acid sequence ranging from serine at position 25 to aspartic acid at position 213 of the amino acid sequence set forth in SEQ ID NO: 18, which is one aspect of the AAV-binding protein set forth in (E).
An AAV-binding protein comprising at least an amino acid sequence ranging from serine at position 25 to aspartic acid at position 213 of the amino acid sequence set forth in SEQ ID NO: 20, which is one aspect of the AAV-binding protein set forth in (F).
An AAV-binding protein comprising at least an amino acid sequence ranging from serine at position 25 to aspartic acid at position 213 of the amino acid sequence set forth in SEQ ID NO: 21, which is one aspect of the AAV-binding protein set forth in (G).
An AAV-binding protein comprising at least an amino acid sequence ranging from serine at position 25 to aspartic acid at position 213 of the amino acid sequence set forth in SEQ ID NO: 22, which is one aspect of the AAV-binding protein set forth in (H).
An AAV-binding protein comprising at least an amino acid sequence ranging from serine at position 25 to aspartic acid at position 213 of the amino acid sequence set forth in SEQ ID NO: 23, which is one aspect of the AAV-binding protein set forth in (I).
An AAV-binding protein comprising at least an amino acid sequence ranging from serine at position 25 to aspartic acid at position 213 of the amino acid sequence set forth in SEQ ID NO: 24, which is one aspect of the AAV-binding protein set forth in (J).
An AAV-binding protein comprising at least an amino acid sequence ranging from serine at position 25 to aspartic acid at position 213 of the amino acid sequence set forth in SEQ ID NO: 25, which is one aspect of the AAV-binding protein set forth in (K).

In (ii), (v), (viii), and (xi) described above, the expression "substitutions of one or several amino acid residues" may include a conservative substitution between amino acids having similar physical and/or chemical properties, in addition to the amino acid substitution(s) at specific position(s) described above. Those skilled in the art know that in the case of conservative substitution, the protein function is usually maintained between a protein having a substitution and a protein lacking the substitution. One example of a conservative substitution is a substitution that occurs between glycine and alanine, serine and proline, or glutamic acid and alanine (Protein Structure and Function, Medical Sciences International, Ltd., 9, 2005). Another example of the amino acid substitution is a substitution for monomerizing the AAV-binding protein of the invention. Specific examples thereof include amino acid substitutions in which a cysteine residue that easily constitutes a higher-order structure is substituted with a serine residue or a methionine residue.

Further, the expression "one or more substitutions, deletions, insertions, and additions" in (ii), (v), (viii), and (xi) described above may also include naturally occurring mutations due to the difference in the origin of the AAV-binding protein, the difference in species, and the like (mutants or variants).

In (iii), (vi), (ix), and (xii) described above, the homology of the amino acid sequences may be 70% or more, and the homology may be more than that (e.g., 80% or more, 85% or more, 90% or more, or 95% or more). The term "homology" used herein may refer to similarity or identity and may particularly refer to identity. The "amino acid sequence homology" means homology to the entire amino acid sequence.

The "identity" between amino acid sequences means the proportion of amino acid residues of the same type in those amino acid sequences (Experimental Medicine, February 2013, Vol. 31, No. 3, YODOSHA CO., LTD.). The "similarity" between amino acid sequences means the sum of the proportion of amino acid residues of the same type in those amino acid sequences and the proportion of amino acid residues having similar side chain properties (Experimental Medicine, February 2013, Vol. 31, No. 3, YODOSHA CO., LTD.). The homology of the amino acid sequence can be determined by using an alignment program such as BLAST (Basic Local Alignment Search Tool) or FASTA.

It is also possible to further add a useful oligopeptide to the N-terminal side or the C-terminal side of the AAV-binding protein of the invention for separation from a solution with the presence of contaminants. Examples of the oligopeptide include polyhistidine, polylysine, polyarginine, polyglutamic acid, and polyaspartic acid. In addition, a cysteine-containing oligopeptide useful for immobilizing the AAV-binding protein of the invention to a solid phase such as a support for chromatography may be further added to the N-terminal side or the C-terminal side of the AAV-binding protein of the invention.

The length of an oligopeptide to be added to the N-terminal side or the C-terminal side of the AAV-binding protein is not particularly limited as long as the AAV-binding property and stability of the AAV-binding protein of the invention are not impaired. When adding the oligopeptide to the AAV-binding protein of the invention, it is possible to prepare a polynucleotide encoding the oligopeptide, and then, add the polynucleotide to the N-terminal side or the C-terminal side of the AAV-binding protein by a genetic engineering method known to those skilled in the art. Alternatively, it is also possible to chemically synthesize the oligopeptide and add the oligopeptide to the N-terminal side or the C-terminal side of the AAV-binding protein of the invention via chemical binding.

It is also possible to further add a signal peptide for promoting efficient expression in *Escherichia coli* (*E. coli*) used as a host, to the N-terminal side of the AAV-binding protein of the invention. Examples of the signal peptide can include signal peptides that promotes protein secretion in a periplasmic space, such as PelB, OmpA, DsbA, DsbC, MalE, and TorT (Japanese Unexamined Patent Publication (Kokai) No. 2011-097898).

Examples of a method for producing a polynucleotide encoding the AAV-binding protein of the invention (hereinafter referred to as "polynucleotide of the invention") can include:
(I) a method for artificially synthesizing a polynucleotide comprising a nucleotide sequence that is converted from the amino acid sequence of the AAV-binding protein of the invention; and
(II) a method in which a polynucleotide comprising the entire or partial sequence of the AAV-binding protein is prepared directly in an artificial manner or by a DNA amplification method such as a PCR method using cDNA or the like of the AAV-binding protein, and the prepared polynucleotide is ligated in a suitable manner.

In the method (I) described above, when converting from an amino acid sequence to a nucleotide sequence, it is preferable to perform the conversion in consideration of the frequency of use of codons in *E. coli* which is the host to be transformed. Specifically, the conversion may be performed to avoid codons such as AGA/AGG/CGG/CGA for arginine (Arg), ATA for isoleucine (Ile), CTA for leucine (Leu), GGA for glycine (Gly), and CCC for proline (Pro) because those codons are used infrequently (they are so-called rare codons). Analysis of the frequency of codon usage is also possible by using a public database (e.g., the Codon Usage Database on the website of Kazusa DNA Research Institute).

When introducing a mutation into the polynucleotide of the invention, the error-prone PCR method can be used. The reaction conditions in the error-prone PCR method are not particularly limited as long as the desired mutation can be introduced into the polynucleotide encoding the AAV-binding protein. For example, the mutation can be introduced by, making the concentration of four kinds of deoxynucleotides (dATP/dTTP/dCTP/dGTP) serving as substrates different, adding MnCl₂ at a concentration of from 0.01 to 10 mM (preferably from 0.1 to 1 mM) to a PCR reaction solution, and performing PCR. In addition, examples of a mutagenesis method other than the error prone PCR method include a method for producing a mutant by allowing an agent serving as a mutagen to come into contact with or act on a polynucleotide comprising the entire or partial sequence of an AAV-binding protein or irradiating the polynucleotide with ultraviolet rays to introduce a mutation into the polynucleotide. As an agent used as a mutagen in the method, a mutagenic agent commonly used by those skilled in the art such as hydroxylamine, N-methyl-N'-nitro-N-nitrosoguanidine, nitrite, sulfurous acid, or hydrazine may be used.

When transforming *E. coli* as the host with the polynucleotide of the invention, the polynucleotide of the invention itself may be used. However, it is more preferable to use a polynucleotide obtained by inserting the polynucleotide of the invention at an appropriate position in an expression vector (e.g., bacteriophage, cosmid, plasmid, or the like commonly used for transformation of prokaryotic or eukaryotic cells). The expression vector is not particularly limited as long as it exists stably and can be replicated in the host to be transformed (*E. coli*)*.* Examples of the expression vector can include a pET plasmid vector, a pUC plasmid vector, a pTrc plasmid vector, and a pCDF plasmid vector.

In addition, the appropriate position means a position that does not destroy the replication function of the expression vector, the desired antibiotic marker, or the transmissible region. When inserting the polynucleotide of the invention into the expression vector, it is preferable to insert it in a state of being linked to a functional polynucleotide such as a promoter required for expression. Examples of the promoter include a trp promoter, a tac promoter, a trc promoter, a lac promoter, a T7 promoter, a recA promoter, and an lpp promoter.

*E. coli* as the host may be transformed using the expression vector produced by the method in which the polynucleotide of the invention is inserted (hereinafter referred to as "expression vector of the invention"), by a method usually used by those skilled in the art. Specifically, E. *coli* may be transformed by a method described in a known document (e.g., Molecular Cloning, Cold Spring Harbor Laboratory, 256, 1992) or the like. Transformants obtained via transformation by the method described above are screened by an appropriate method such that a transformant capable of expressing the AAV-binding protein of the invention (hereinafter referred to as "transformant of the invention") can be obtained.

The expression vector of the invention from the transformant of the invention may be prepared from the culture obtained by culturing the transformant of the invention by an alkaline extraction method or a commercially available extraction kit such as a QIAprep Spin Miniprep kit (manufactured by QIAGEN).

The AAV-binding protein of the invention can be produced by culturing the transformant of the invention and recovering the AAV-binding protein of the invention from the obtained culture. The culture described herein includes not only the cultured cells of the transformant of the invention itself, but also the medium used for the culture.

The transformant used in the method for producing the protein of the invention may be cultured in a medium suitable for culturing a target host (*E. coli*). Examples of a preferable medium include LB (Luria-Bertani) medium supplemented with necessary nutrient sources. To selectively grow the transformant of the invention depending on whether or not the vector of the invention is introduced, it is preferable to add a drug corresponding to a drug resistance gene contained in the vector to the medium and culture the transformant. For example, when the vector contains a kanamycin resistance gene, kanamycin may be added to the medium.

In addition to carbon, nitrogen, and inorganic salt sources, suitable nutrient sources may be added to the medium. The medium may contain one or more reducing agents optionally selected from the group consisting of glutathione, cysteine, cystamine, thioglycolate, and dithiothreitol. The culture temperature is generally from 10°C to 40°C, preferably from 20°C to 37°C, more preferably around 25°C, but may be selected depending on the characteristics of the protein to be expressed. The pH of the medium is pH 6.8 to pH 7.4, preferably around pH 7.0. When the vector of the invention contains an inducible promoter, it is preferable to induce the vector under conditions that allow favorable expression of the AAV-binding protein of the invention.

As the inducer, isopropyl-β-D-thiogalactopyranoside (IPTG) can be exemplified. The turbidity of the culture solution (absorbance at 600 nm) is measured, and when it becomes about 0.5 to 1.0, an appropriate amount of IPTG is added and then the culture is continued, thereby achieving the expression of the AAV-binding protein. The concentration of IPTG added may be appropriately selected from a range of from 0.005 to 1.0 mM, preferably a range of from 0.01 to 0.5 mM. Various conditions relating to IPTG induction may be performed under conditions well known in the art.

To recover the AAV-binding protein of the invention from the culture obtained by culturing the transformant of the invention, the AAV-binding protein of the invention may be recovered by separation/purification from the culture by a method suitable for the expression form of the AAV-binding protein of the invention in the transformant of the invention. For example, when the AAV-binding protein of the invention is expressed in the culture supernatant, the cells may be separated by centrifugation and the AAV-binding protein may be purified from the obtained culture supernatant. In addition, when the AAV-binding protein of the invention is expressed intracellularly (in a periplasmic space), after collecting the cells by centrifugation, the cells are disrupted by adding an enzyme treatment agent, a surfactant, or the like to extract the AAV-binding protein, and then the AAV-binding protein may be purified.

To purify the AAV-binding protein of the invention, a method known in the art may be used, and one example thereof is separation/purification using liquid chromatography. Examples of liquid chromatography include on ion exchange chromatography, hydrophobic interaction chromatography, gel filtration chromatography, and affinity chromatography. By performing a purification operation in combination with these chromatography methods, the AAV-binding protein of the invention can be prepared with high purity.

As a method for measuring the binding activity of the obtained AAV-binding protein of the invention to AAV, for example, the binding activity to AAV may be measured by using an enzyme-linked immunosorbent assay method (hereinafter referred to as "ELISA method"), a surface plasmon resonance method, or the like. The AAV used for measuring the binding activity may be an AAV vector or a VLP (virus-like particle). In addition, any serotype of the AAV vector and VLP may be used as long as it exhibits the binding activity to the AAV-binding protein of the invention.

The AAV-binding protein of the invention can be used, for example, for purification or analysis of AAV. The AAV-binding protein of the invention can be used, for example, by immobilizing it on an insoluble carrier. In other words, purification or analysis of AAV can be specifically performed using, an AAV adsorbent comprising, for example, an insoluble carrier and the AAV-binding protein of the invention immobilized on the insoluble carrier. The AAV adsorbent of the invention described herein also refers to an AAV adsorbent comprising the insoluble carrier and the AAV-binding protein of the invention immobilized on the insoluble carrier. The purification of AAV is not limited to the purification of AAV from a solution in which contaminants coexist, but also includes the purification of AAV based on its structure, properties, activity, and the like. The material, shape, and the like of the insoluble carrier are not particularly limited, examples of which include those disclosed in WO2021/106882. Further, the insoluble carrier may be, for example, porous or non-porous.

The AAV-binding protein of the invention can be immobilized on an insoluble carrier, for example, via a covalent bond. Specifically, the AAV-binding protein of the invention can be immobilized on an insoluble carrier by covalently binding the AAV-binding protein of the invention and the insoluble carrier, for example, via an active group of the insoluble carrier. The immobilization of the AAV-binding protein of the invention on the insoluble carrier may be performed, for example, based on the disclosure in WO2021/106882.

The AAV adsorbent of the invention can be used, for example, by filling it into a column to purify AAV. Specifically, for example, by adding a solution containing AAV to a column filled with the AAV adsorbent of the invention (hereinafter, also simply referred to as "the column of the invention"), the AAV is adsorbed by the adsorbent, and the AAV adsorbed by the adsorbent is eluted such that the AAV can be purified. In other words, the present invention provides a method for purifying AAV comprising a step of adding a solution containing AAV to a column filled with the AAV adsorbent of the invention to allow the AAV to be adsorbed by the adsorbent and a step of eluting the AAV adsorbed by the adsorbent. The AAV purification using the column of the invention may be performed, for example, based on the disclosure in WO2021/106882.

By purifying AAV with the AAV adsorbent of the invention, for example, purified AAV can be obtained. In other words, the method for purifying AAV may be, in one aspect, a method for producing AAV, and specifically, a method for producing purified AAV. AAV is obtained, for example, as an elution fraction containing AAV. This means that the fraction containing the eluted AAV can be obtained. For example, the AAV fraction can be obtained by a conventional method. As a method for obtaining the AAV fraction, a method comprising replacing a collection container every fixed time or fixed capacity, a method comprising replacing a collection container according to the shape of an eluate chromatogram, and a method using an automated fraction collector such as autosampler can be exemplified. Furthermore, AAV can also be recovered from the fraction containing AAV. Recovery of AAV from the fraction containing AAV can be performed, for example, by known methods used for protein purification.

The present invention also encompasses the following aspects [15] to [17]:
[15] A method for analyzing an adeno-associated virus (AAV) contained in a sample based on the strength of the infectivity, wherein the analysis is performed by a method comprising a step of allowing the AAV to be adsorbed by an AAV adsorbent comprising an insoluble carrier and the AAV-binding protein which is immobilized on the carrier, and a step of eluting the AAV adsorbed by said adsorbent using an eluate, and wherein the AAV-binding protein is a polypeptide selected from any of the following (xiii) to (xv):
   (xiii) a polypeptide comprising at least amino acid residues from serine at position 312 to glutamic acid at position 401 or amino acid residues from isoleucine at position 409 to aspartic acid at position 500 of the amino acid sequence set forth in SEQ ID NO: 94;
   (xiv) a polypeptide comprising at least amino acid residues from serine at position 312 to glutamic acid at position 401 or amino acid residues from isoleucine at position 409 to aspartic acid at position 500 of the amino acid sequence set forth in SEQ ID NO: 94, wherein one or more substitutions, deletions, insertions, and additions of one or several amino acid residues are present at one or several positions in these amino acid residues, and wherein the polypeptide has AAV-binding activity;
   (xv) a polypeptide comprising at least amino acid residues from serine at position 312 to glutamic acid at position 401 or amino acid residues from isoleucine at position 409 to aspartic acid at position 500 of the amino acid sequence set forth in SEQ ID NO: 94, except for having 70% or more homology to said amino acid sequence consisting of said amino acid residues, wherein the polypeptide has AAV-binding activity.
[16] The method according to [15], wherein the AAV-binding protein is a polypeptide selected from any of the following (xvi) to (xviii):
   (xvi) a polypeptide comprising at least amino acid residues from serine at position 312 to aspartic acid at position 500 of the amino acid sequence set forth in SEQ ID NO: 94;
   (xvii) a polypeptide comprising at least amino acid residues from serine at position 312 to aspartic acid at position 500 of the amino acid sequence set forth in SEQ ID NO: 94, wherein one or more substitutions, deletions, insertions, and additions of one or several amino acid residues are present at one or several positions in the amino acid residues from position 312 to position 500, and wherein the polypeptide has AAV-binding activity;
   (xviii) a polypeptide comprising at least amino acid residues from serine at position 312 to aspartic acid at position 500 of the amino acid sequence set forth in SEQ ID NO: 94, except for having 70% or more homology to said amino acid sequence consisting of said amino acid residues from serine at position 312 to aspartic acid at position 500, wherein the polypeptide has AAV-binding activity.
[17] The method according to [15] or [16], wherein the AAV adsorbent is filled in a column.

The AAV-binding protein which is a ligand protein (a protein to be immobilized on the insoluble carrier) for the adsorbent for use in the AAV analysis in the present invention (hereinafter, also referred to as "AAV adsorbent") is a polypeptide comprising at least a region corresponding to the extracellular domain 1 (PKD1) (amino acid residues from serine (Ser) at position 312 to glutamic acid (Glu) at position 401 of SEQ ID NO: 94) or the extracellular domain 2 (PKD2) (amino acid residues from isoleucine (Ile) at position 409 to aspartic acid (Asp) at position 500 of SEQ ID NO: 94) in KIAA0319L (UniProt; Accession Number: Q8IZA0, SEQ ID NO: 94) which is one aspect of the AAV receptor (AAVR), having AAV binding activity. Specific examples thereof include a polypeptide selected from any of the following (xiii) to (xv):
(xiii) a polypeptide comprising at least amino acid residues from serine at position 312 to glutamic acid at position 401 or amino acid residues from isoleucine at position 409 to aspartic acid at position 500 of the amino acid sequence set forth in SEQ ID NO: 94;
(xiv) a polypeptide comprising at least amino acid residues from serine at position 312 to glutamic acid at position 401 or amino acid residues from isoleucine at position 409 to aspartic acid at position 500 of the amino acid sequence set forth in SEQ ID NO: 94, wherein one or more substitutions, deletions, insertions, and additions of one or several amino acid residues are present at one or several positions in these amino acid residues, and wherein the polypeptide has AAV-binding activity;
(xv) a polypeptide comprising at least amino acid residues from serine at position 312 to glutamic acid at position 401 or amino acid residues from isoleucine at position 409 to aspartic acid at position 500 of the amino acid sequence set forth in SEQ ID NO: 94, except for having 70% or more homology to said amino acid sequence consisting of said amino acid residues, wherein the polypeptide has AAV-binding activity.

In addition, preferable aspects of the AAV-binding protein includes a polypeptide comprising at least regions corresponding to the PKD1 and the PKD2 (amino acid residues from serine (Ser) at position 312 to aspartic acid (Asp) at position 500 of SEQ ID NO: 94) in KIAA0319L (SEQ ID NO: 94). Specific examples thereof include a polypeptide selected from any of the following (xvi) to (xviii):
(xvi) a polypeptide comprising at least amino acid residues from serine at position 312 to aspartic acid at position 500 of the amino acid sequence set forth in SEQ ID NO: 94;
(xvii) a polypeptide comprising at least amino acid residues from serine at position 312 to aspartic acid at position 500 of the amino acid sequence set forth in SEQ ID NO: 94, wherein one or more substitutions, deletions, insertions, and additions of one or several amino acid residues are present at one or several positions in the amino acid residues from position 312 to position 500 of the amino acid sequence, and wherein the polypeptide has AAV-binding activity;
(xviii) a polypeptide comprising at least amino acid residues from serine at position 312 to aspartic acid at position 500 of the amino acid sequence set forth in SEQ ID NO: 94, except for having 70% or more homology to said amino acid sequence consisting of said amino acid residues from serine at position 312 to aspartic acid at position 500, wherein the polypeptide has AAV-binding activity.

The polypeptide described in any of (xiii) to (xviii) described above may suffice as long as it comprises at least a region corresponding to PKD1 and/or PKD2 in KIAA0319L. For example, it may comprise: all or part of a region corresponding to the other extracellular domains (domain 3 (PKD3), domain 4 (PKD4), and domain 5 (PKD5)) on the C-terminal side of PKD2; all or part of a region corresponding to a signal sequence such as the MANSC (motif at N terminus with seven cysteines) domain on the N-terminal side of PKD1 or cysteine-rich region; and all or part of the transmembrane and intracellular domains on the N-terminal and/or C-terminal side of the extracellular domain.

Examples of the (xiv) and (xvii) described above includes polypeptides comprising at least amino acid residues from serine at position 25 (Ser) to aspartic acid (Asp) at position 213 of the amino acid sequence set forth in SEQ ID NO: 97, and AAV-binding proteins disclosed in WO2021/106882. Examples of the substitutions, deletions, insertions, or additions described in (xiv) and (xvii) described above include substitutions of amino acid residues disclosed in WO2021/106882.

In (xiv) and (xvii) described above, the expression "one or several" refers to, for example, from 1 to 50, from 1 to 30, from 1 to 20, from 1 to 10, from 1 to 9, from 1 to 8, from 1 to 7, from 1 to 6, from 1 to 5, from 1 to 4, from 1 to 3, from 1 to 2, or 1, although it depends on positions of amino acid substitutions and types of amino acid residues in the conformation of the AAVR. Substitutions of "one or several" amino acid residues may be present at positions other than the substitutions of the amino acid residues disclosed in WO2021/106882, as long as it has AAV binding activity.

In the "substitutions of one or several amino acid residues" in (xiv) and (xvii) described above, a conservative substitution between amino acids having similar physical and/or chemical properties may be present in addition to the substitution(s) of amino acid residue(s) at specific position(s) described above. Those skilled in the art know that in the case of conservative substitution, the protein function is usually maintained between a protein having a substitution and a protein lacking the substitution. One example of a conservative substitution is a substitution that occurs between glycine and alanine, serine and proline, or glutamic acid and alanine (Protein Structure and Function, Medical Sciences International, Ltd., 9, 2005). Further, "substitutions, deletions, insertions, and additions of one or several amino acid residues" in (xiv) and (xvii) described above also include naturally occurring mutations due to the difference in the origin of AAV-binding protein, the difference in species, and the like (mutants or variants).

The homology of the amino acid sequences in (xv) and (xviii) described above may be 70% or more, and the homology may be more than that (e.g., 80% or more, 85% or more, 90% or more, or 95% or more). The term "homology" used herein may refer to similarity or identity and may particularly refer to identity. The "amino acid sequence homology" means homology to the entire amino acid sequence. The "identity" between amino acid sequences means the proportion of amino acid residues of the same type in those amino acid sequences (Experimental Medicine, February 2013, Vol. 31, No. 3, YODOSHA CO., LTD.). The "similarity" between amino acid sequences means the sum of the proportion of amino acid residues of the same type in those amino acid sequences and the proportion of amino acid residues having similar side chain properties (Experimental Medicine, February 2013, Vol. 31, No. 3, YODOSHA CO., LTD.). The homology of the amino acid sequence can be determined by using an alignment program such as BLAST (Basic Local Alignment Search Tool) or FASTA.

In the present invention, the insoluble carrier for use in the immobilization of the AAV-binding protein is not particularly limited as long as it is insoluble in AAV-containing samples and solutions used for the analysis or purification (such as eluate, equilibration solution, and washing solution). Examples thereof include carriers made with polysaccharides such as agarose, alginate (alginic acid salt), carrageenan, chitin, cellulose, dextrin, dextran, and starch, carriers made with synthetic polymers such as polyvinyl alcohol, polymethacrylate, poly(2-hydroxyethyl methacrylate), and polyurethane, and carriers made with ceramics such as silica. Of these, carriers made with polysaccharides and carriers made with synthetic polymers are preferable as insoluble carriers. Examples of the preferable carrier include hydroxylated polymethacrylate gel such as TOYOPEARL (manufactured by Tosoh Corporation), agarose gel such as Sepharose (manufactured by Cytiva), and cellulose gel such as Cellufine (manufactured by JNC). The shape of insoluble carrier is not particularly limited, but a shape that can be filled in a column is preferred. The insoluble carrier may be, for example, a granular substance, a monolith-like substance, a film-like substance, a fibrous substance, or the like. Further, the insoluble carrier may be, for example, porous or non-porous.

The immobilization of the AAV-binding protein on the insoluble carrier can be carried out by immobilization, for example, via a covalent bond. Specifically, the AAV adsorbent for use in the present invention can be produced by immobilization of the AAV-binding protein on the insoluble carrier by covalently binding therebetween, for example, via an active group of the insoluble carrier. Examples of an active group include an N-hydroxy succinimide (NHS)-activated ester group, an epoxy group, a carboxy group, a maleimide group, a haloacetyl group, a tresyl group, a formyl group, and a haloacetamide group. As the insoluble carrier having an active group, for example, a commercially available insoluble carrier having an active group may be used as it is, or an active group may be introduced into the insoluble carrier for use. Examples of a commercially available carrier having an active group may include TOYOPEARL AF-Epoxy-650M, TOYOPEARL AF-Tresyl-650M (each manufactured by Tosoh Corporation), HiTrap NHS-activated HP Columns, NHS-activated Sepharose 4 Fast Flow, Epoxy-activated Sepharose 6B (each manufactured by Cytiva), and SulfoLink Coupling Resin (manufactured by Thermo Fisher Scientific).

As a method for introducing an active group on the carrier surface, a method in which a compound having two or more active sites is allowed to react, at one of the active sites, with a hydroxy group, an epoxy group, a carboxy group, an amino group, or the like present on the carrier surface can be exemplified.

Examples of a compound for introducing an epoxy group to a hydroxy group or an amino group present on the carrier surface may include epichlorohydrin, ethanediol diglycidyl ether, butanediol diglycidyl ether, hexanediol diglycidyl ether, and polyethylene glycol diglycidyl ether. Specific examples of polyethylene glycol diglycidyl ether may include Denacol EX-810 (n=1), Denacol EX-811 (n=1), Denacol EX-850 (n=2), Denacol EX-851 (n=2), Denacol EX-821 (n=4), Denacol EX-830 (n=9), Denacol EX-832 (n=9), Denacol EX-841 (n=13), and Denacol EX-861 (n=22) (all manufactured by Nagase ChemteX).

Examples of a compound for introducing a carboxy group to an epoxy group present on the carrier surface may include 2-mercaptoacetic acid, 3-mercaptopropionic acid, 4-mercaptobutyric acid, 6-mercaptobutyric acid, glycine, 3-aminopropionic acid, 4-aminobutyric acid, and 6-aminohexanoic acid.

Examples of a compound for introducing a maleimide group to a hydroxy group, an epoxy group, a carboxy group, or an amino group present on the carrier surface may include N-(ε-maleimidocaproic acid)hydrazide, N-(s-maleimidopropionic acid)hydrazide, 4-(4-N-maleimidophenyl)acetic acid hydrazide, 2-aminomaleimide, 3-aminomaleimide, 4-aminomaleimide, 6-aminomaleimide, 1-(4-aminophenyl)maleimide, 1-(3-aminophenyl)maleimide, 4-(maleimide)phenyl isocyanate, 2-maleimidoacetic acid, 3-maleimidopropionic acid, 4-maleimidobutyric acid, 6-maleimidohexanoic acid, N-(α-maleimidoacetoxy)succinimide ester, (m-maleimidobenzoyl)N-hydroxy succinimide ester, succinimidyl-4-(maleimidomethyl)cyclohexane-1-carbonyl-(6-aminohexanoic acid), succinimidyl-4-(maleimidomethyl)cyclohexane-1-carboxylic acid, (p-maleimidobenzoyl)N-hydroxy succinimide ester, and (m-maleimidobenzoyl)N-hydroxy succinimide ester.

Examples of a compound for introducing a haloacetyl group to a hydroxy group or an amino group present on the carrier surface may include chloroacetic acid, bromoacetic acid, iodoacetic acid, chloroacetic acid chloride, bromoacetic acid chloride, bromoacetic acid bromide, chloroacetic acid anhydride, bromoacetic acid anhydride, iodoacetic acid anhydride, 2-(iodoacetamido)acetic acid-N-hydroxysuccinimide ester, 3-(bromoacetamido)propionic acid-N-hydroxysuccinimide ester, and 4-(iodoacetyl)aminobenzoic acid-N-hydroxysuccinimide ester.

As a method for introducing an active group on the carrier surface, a method in which ω-alkenyl alkane glycidyl ether is reacted with a hydroxy group or an amino group present on the carrier surface, and then, ω-alkenyl is halogenated with a halogenating agent for activation can also be exemplified. Examples of ω-alkenyl alkane glycidyl ether may include allyl glycidyl ether, 3-butenyl glycidyl ether, and 4-pentenyl glycidyl ether. Examples of a halogenating agent may include N-hlorosuccinimide, N-bromosuccinimide, and N-iodosuccinimide.

Another example of a method for introducing an active group on the carrier surface is a method for introducing an active group to a carboxy group present on the carrier surface using a condensing agent and an additive. Examples of a condensing agent may include 1-ethyl-3-(3-dimethylaminopropyl)carbodiimide (EDC), dicyclohexylcarbodiamide, and carbonyldiimidazole. Examples of an additive may include N-hydroxysuccinimide (NHS), 4-nitrophenol, and 1-hydroxybenzotriazole.

Immobilization of the AAV-binding protein on an insoluble carrier can be carried out, for example, in a buffer. Examples of a buffer may include acetic acid buffer, phosphoric acid buffer, MES (2-morpholinoethanesulfonic acid) buffer, HEPES (4-(2-hydroxyethyl)-1-piperazineethanesulfonic acid) buffer, Tris (Tris(hydroxymethyl)aminomethane) buffer, and boric acid buffer. The reaction temperature for immobilization may be set as appropriate in consideration of reactivity of an active group and stability of an AAV-binding protein. The reaction temperature at the time of immobilization may be, for example, from 4°C to 50°C, preferably from 10°C to 35°C.

The present invention is also characterized in that AAV contained in a sample is analyzed based on the strength of the infectivity, by a method comprising a step of allowing the AAV contained in the sample by an AAV adsorbent produced by the above-mentioned method (hereinafter, also simply referred to as "adsorption step"), and a step of eluting the AAV adsorbed by said adsorbent using an eluate (hereinafter, also simply referred to as "elution step"). It is preferable that the AAV adsorbent for use in the adsorption step and the elution step is filled in a column (hereinafter, also referred to as "AVR column"), since these steps can be easily performed. A detailed explanation of aspects using an AVR column will be given below as an example.

The sample containing AAV can be added (applied) to an AVR column using, for example, a liquid transfer means such as a pump. Adding a liquid to a column described herein is also referred to as "transferring the liquid to the column." The sample containing AAV may be treated by solvent substitution with an appropriate buffer before being added to the AVR column. Also, the AVR column may be equilibrated with an appropriate buffer (equilibrating solution) before adding the sample containing AAV to the AVR column. It is expected that the equilibration will allow, for example, purification of AAV to a higher degree of purity. Examples of the buffer used for solvent substitution and equilibration may include a phosphate buffer, an acetate buffer, a succinate buffer, a citrate buffer, a Tris buffer, a HEPES buffer and an MES buffer which have a buffering capacity in the neutral region (herein refers to the pH 4.0 to 9.0 region). An inorganic salt such as 10 mM to 600 mM sodium chloride and calcium chloride may be further added to such buffer, for example. The buffer used for solvent substitution and the equilibrating solution may be the same or different. In addition, if components other than AAV such as contaminants remain in the AVR column after passing the sample containing AAV through the AVR column, such components may be removed (washed) from the AVR column before the AAV adsorbed by the AAV adsorbent is eluted with an eluate (i.e., before the elution step). Components other than AAV can be removed from the AVR column by using, for example, a suitable buffer as a washing solution. Regarding the washing solution, for example, the descriptions regarding the buffer solutions used for solvent replacement and equilibration can be applied mutatis mutandis.

In the elution step, in order to elute AAV based on the strength of the infectivity, elution is performed using a gradient. The gradient may be changed in two or more steps (stepwise) (stepwise gradient), or may be changed with a linear gradient (linear gradient). Examples of gradient elution include a gradient in which the pH is lowered from the neutral region to an acidic region (a region more acidic than the neutral region). The pH of the eluate at the beginning of the gradient may be in the neutral range, that is, pH 4.0 to 9.0 region, preferably pH 4.5 to 6.0. The pH of the eluate after gradient, which is an acidic region, may be 3.5 or lower, preferably 2.0 to 2.5. The lower limit of the flow rate is not particularly limited, and the upper limit depends on the back pressure of the insoluble carrier used in the AAV adsorbent. Typically, the flow rate is preferably 0.5 mL/min to 2.0 mL/min. The gradient time is sufficient as long as differences in the strength of the infectivity can be identified as a difference in the elution time. Specifically, the gradient time is preferably 5 minutes or longer, more preferably 20 minutes or longer, and even more preferably 45 minutes or longer.

The AAV that can be analyzed by the method of the invention may be a naturally occurring AAV or an artificially produced AAV. Examples thereof include AAVs of respective serotypes (such as AAV1, AAV2, AAV3, AAV4, AAV5, AAV6, AAV7, AAV8, AAV9, AAV10, AAV1 1, AAV12, and AAV13), AAV with amino acid substitution(s), AAV fused with peptide or protein, and AAV modified with polymer(s).

With use of the method of the invention, for example, it is possible to easily screen for AAV amino acid substitution product (AAV mutants) having desired infectivity. Specifically, screening for AAV mutants having desired infectivity can be performed by creating a mutant library of plasmids encoding AAV capsid using genetic modification such as error-prone PCR, then transforming the host with other plasmids required for AAV formation, and thereafter applying the AAV mutants expressed from the transformant to an AVR column.

According to the present invention, AAV contained in a sample can be analyzed depending on the strength of the infectivity. As a result, for example, AAV having high infectivity can be purified and used as a highly efficient gene therapy drug. Furthermore, if AAVs having different infectivity are mixed, the ratio thereof can be checked. Furthermore, it may also be used for quality control in the AAV production process to check for differences between lots and uniformity.

### ADVANTAGEOUS EFFECTS OF INVENTION

The adeno-associated virus (AAV)-binding protein of the invention is a protein in which amino acid residue(s) at specific positions within domains corresponding to the extracellular domain 1 (PKD1) and domain 2 (PKD2) of KIAA0319L (UniProt No. Q8IZA0) is(are) substituted with different amino acid residue(s). The AAV-binding protein of the invention has enhanced binding activity to AAV than conventional AAV-binding proteins. Furthermore, when the AAV-binding protein of the invention is produced by genetic engineering using recombinant E. coli, the production of decomposition products derived from the protein is suppressed compared to conventional AAV-binding proteins. Therefore, the AAV-binding protein can be efficiently produced, which is useful for industrial production of the protein. In addition, the present invention is characterized by that an adeno-associated virus (AAV) contained in a sample is analyzed based on the strength of the infectivity by a method comprising a step of allowing the AAV to be adsorbed by an adsorbent comprising an insoluble carrier and a polypeptide which is immobilized on the carrier and which comprises at least an amino acid sequence corresponding to the extracellular domain 1 or domain 2 of KIAA0319L, and a step of eluting the AAV adsorbed by said adsorbent using an eluate. According to the present invention, analysis of AAV contained in a sample based on the strength of the infectivity can be quickly and easily carried out, for which conventional methods require a lot of time and effort using cultured cells.

### BRIEF DESCRIPTION OF DRAWINGS

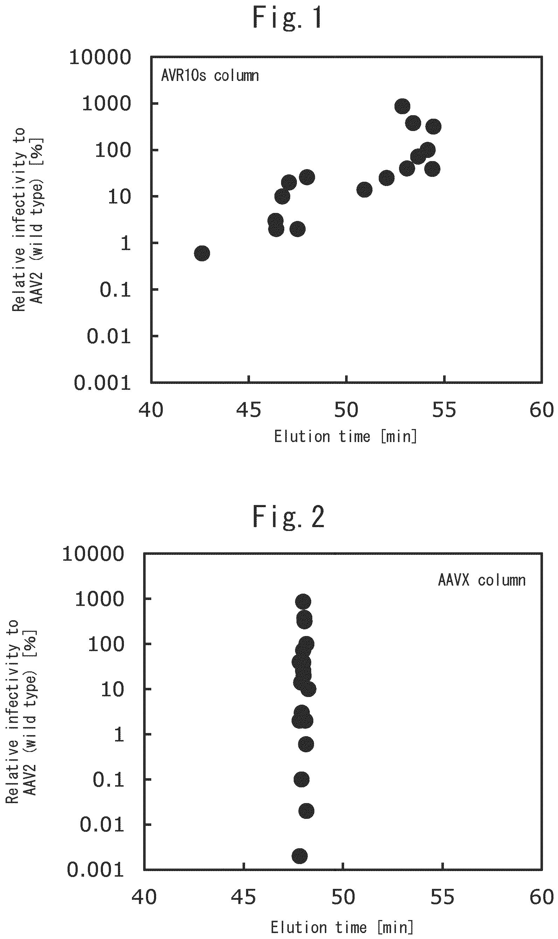

FIG. 1 is a diagram summarizing the analysis results of AAV2 (wild type and its amino acid substitution product) using an AVR10s column. AAV was eluted by applying a gradient of buffer pH from 4.5 to 2.2. The infectivity (relative value to the wild type) of each AAV2 is plotted on the vertical axis, and the elution time of the AAV2 is plotted on the horizontal axis. Among the AAV2 amino acid substitution products, G383A, H271F, and D529A are not shown because they were not adsorbed to the AVR10s immobilization gel and passed through the AVR10s column.
FIG. 2 is a diagram summarizing the analysis results of AAV2 (wild type and its amino acid substitution product) using an AAVX column. AAV was eluted by applying a gradient of buffer pH from 4.5 to 2.2. The infectivity (relative value to the wild type) of each AAV2 is plotted on the vertical axis, and the elution time of the AAV2 is plotted on the horizontal axis.

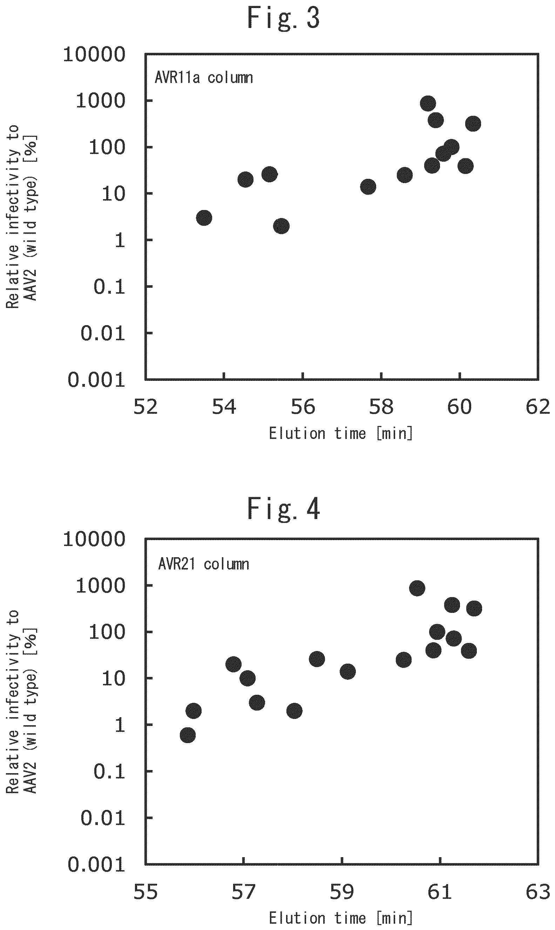

FIG. 3 is a diagram summarizing the analysis results of AAV2 (wild type and its amino acid substitution product) using an AVR11a column. AAV was eluted by applying a gradient of buffer pH from 6.0 to 2.0. The infectivity (relative value to the wild type) of each AAV2 is plotted on the vertical axis, and the elution time of the AAV2 is plotted on the horizontal axis. Among the AAV2 amino acid substitution products, H271A, H271Q, H271T, G383A, H271F, and D529A are not shown because they were not adsorbed to the AVR11a immobilization gel and passed through the AVR11a column.
FIG. 4 is a diagram summarizing the analysis results of AAV2 (wild type and its amino acid substitution product) using an AVR21 column. AAV was eluted by applying a gradient of buffer pH from 6.0 to 2.0. The infectivity (relative value to the wild type) of each AAV2 is plotted on the vertical axis, and the elution time of the AAV2 is plotted on the horizontal axis. Among the AAV2 amino acid substitution products, G383A, H271F, and D529A are not shown because they were not adsorbed to the AVR21 immobilization gel and passed through the AVR21 column.

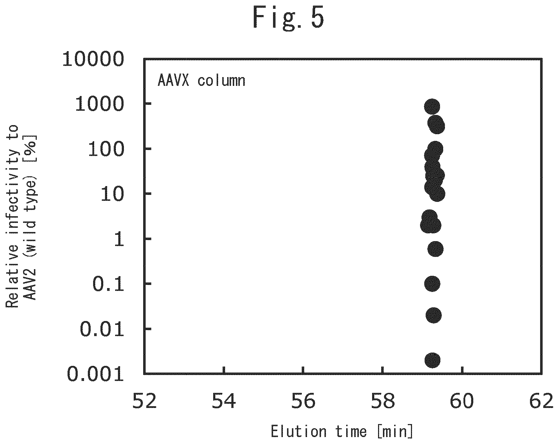

FIG. 5 is a diagram summarizing the analysis results of AAV2 (wild type and its amino acid substitution product) using an AAVX column. AAV was eluted by applying a gradient of buffer pH from 6.0 to 2.0. The infectivity (relative value to the wild type) of each AAV2 is plotted on the vertical axis, and the elution time of the AAV2 is plotted on the horizontal axis.

### EXAMPLES

The invention will now be explained in greater detail using Examples and Comparative Examples, with the understanding that the invention is not limited to Examples.

### Example 1: Preparation of Adeno-Associated Virus (AAV) Vector (Part 1)

Serotype 5 (AAV5) was selected among AAVs to prepare a virus vector used for the following examples.

(1) A nucleotide sequence (SEQ ID NO: 3) was designed, in which a restriction enzyme EcoRI recognition sequence (GAATTC) was added to the 5' end of a polynucleotide encoding an enhanced green fluorescent protein (EGFP) consisting of the amino acid sequence set forth in SEQ ID NO: 2 and a stop codon (TAG) and a BamHI recognition sequence (GGATCC) were added to the 3' end of the same.
(2) A polynucleotide consisting of the sequence set forth in SEQ ID NO: 3 was totally synthesized and cloned into a plasmid (consigned to Fasmac Co., Ltd., named "pUC-EGFP"). The E. coli JM109 strain was transformed with pUC-EGFP, and the resulting transformant was cultured. pUC-EGFP was extracted from the culture solution using a QIAprep Spin Miniprep Kit (manufactured by QIAGEN).
(3) pUC-EGFP obtained in (2) was digested with restriction enzymes EcoRI and BamHI and then ligated to an expression vector pAAV-CMV (manufactured by Takara Bio Inc.), which had previously been digested with restriction enzymes EcoRI and BamHI. The E. coli JM109 strain was transformed using the ligation product.
(4) The transformant obtained in (3) was subjected to shaking culture overnight at 37°C in a 5-L baffled flask containing 1L of a 2×YT medium (1.6% (w/v) tryptone, 1% (w/v) yeast extract, 0.5% (w/v) sodium chloride) containing 100 µg/mL carbenicillin. After the culture was completed, the cells were collected by centrifugation, and a vector pAAV-EGFP expressing EGFP was prepared in a large quantity from the collected cells using a Plasmid Mega Kit (manufactured by QIAGEN).
(5) The E. coli JM109 strain was transformed with a plasmid comprising a polynucleotide encoding the AAV5 capsid (hereinafter referred to as "pRC5 Vector") and a pHelper Vector (manufactured by Takara Bio Inc.). The pRC5 Vector and pHelper were prepared in large quantities by performing the same operation as in (4) using the obtained transformant.
(6) HEK293T cells were cultured in surface-treated CellSTACK Cell Culture Chamber - 5 Chamber, 8 per Case (manufactured by Corning Incorporated) containing 500 mL of a D-MEM medium (manufactured by FUJIFILM Wako Pure Chemical Corporation) containing 5% (v/v) bovine serum. Gene introduction was carried out by adding a complex of pAAV-EGFP prepared in (4), the pRC5 Vector and pHelper prepared in (5), and polyethyleneimine (manufactured by Polysciences, Inc.). The cells were statically cultured for 3 days in 5% (v/v) carbon dioxide at 37°C.
(7) Triton X-100 (manufactured by Sigma-Aldrich) and Benzonase (manufactured by Merck Millipore) were added at final concentrations of 0.1% (w/v) and 1 U/mL, respectively, to the culture solution obtained in (6) and then left standing at 37°C for 3 hours.
(8) After standing, the solution was centrifuged to collect the supernatant. Then, the supernatant was concentrated using a 300 kD-cutoff membrane cassette for a tangential flow filtration system (manufactured by Pall Corporation). The solvent was substituted with 20 mM Tris-HCl buffer (pH 8.0) containing 0.5 M sodium chloride (hereinafter also referred to as "equilibration solution A"). The obtained concentrate was passed through a filter having a pore size of 0.22 µm, thereby removing suspended matter.
(9) The solution from which suspended matter was removed was applied to a 7 mL POROS AAVX column (manufactured by Thermo Fisher Scientific).
(10) After washing with the equilibration solution A, elution was performed with 0.1M acetate buffer (pH 2.5) containing 0.5M sodium chloride. An AAV vector (AAV5-EGFP) solution was obtained via neutralization by adding 1/4 volume of 1 M Tris-HCl buffer (pH 8.5) containing 20 mM magnesium chloride to the resulting elution fraction containing the AAV vector.
(11) The AAV vector concentration in the solution obtained in (10) was quantified by qPCR using an AAVpro Titration Kit (manufactured by Takara Bio Inc.). Further, the solution obtained in (10) was subjected to SDS polyacrylamide gel electrophoresis (SDS-PAGE) and silver-stained using a Pierce Silver Stain Kit (manufactured by Thermo Fisher Scientific), thereby confirming the purity of the AAV vector contained in the solution.

As a result of qPCR, the concentration of the AAV vector (AAV5-EGFP) contained in the solution was 2.5 × 10¹³ cp/mL (cp: number of AAV particles). Furthermore, as a result of SDS-PAGE (silver staining), only bands corresponding to the three types of capsid proteins (VP1, VP2, VP3) constituting the AAV vector were observed, confirming that there was no problem with the purity.

### Example 2: Site-Specific Amino Acid Substitution to AAV-Binding Protein and Library Preparation (Part 1)

A saturation substitution product library for amino acid residues corresponding to alanine (Ala330) at position 330 of SEQ ID NO: 1 (position 43 of SEQ ID NO: 4), tyrosine (Tyr331) at position 331 (position 44 of SEQ ID NO: 4), valine (Val332) at position 332 (position 45 of SEQ ID NO: 4), and leucine (Leu333) at position 333 (position 46 of SEQ ID NO: 4) was created using the vector pET-AVR10s (WO2021/106882) capable of expressing a polypeptide (SEQ ID NO: 4) containing the AAV-binding protein AVR10s as a template. A PelB signal peptide ranges from methionine (Met) at position 1 to alanine (Ala) at position 22, an AAV-binding protein A VR10s ranges from serine (Ser) at position 25 to aspartic acid (Asp) at position 213, and a tag sequence ranges from histidine (His) at position 214 to histidine (His) at position 219 in the polypeptide consisting of the amino acid sequence set forth in SEQ ID NO: 4. AVR10s is a polypeptide with the following 10 amino acid substitutions in amino acid residues from position 312 to position 500 of SEQ ID NO: 1 corresponding to extracellular region domain 1 (PKD1) and domain 2 (PKD2) of KIAA0319L (UniProt No. Q8IZA0):
a substitution of valine with aspartic acid at position 317 of SEQ ID NO: 1 (position 30 of SEQ ID NO: 4),
a substitution of tyrosine with serine at position 342 of SEQ ID NO: 1 (position 55 of SEQ ID NO: 4),
a substitution of lysine with glutamic acid at position 362 of SEQ ID NO: 1 (position 75 of SEQ ID NO: 4),
a substitution of lysine with asparagine at position 371 of SEQ ID NO: 1 (position 84 of SEQ ID NO: 4),
a substitution of valine with alanine at position 381 of SEQ ID NO: 1 (position 94 of SEQ ID NO: 4),
a substitution of isoleucine with valine at position 382 of SEQ ID NO: 1 (position 95 of SEQ ID NO: 4),
a substitution of glycine with serine at position 390 of SEQ ID NO: 1 (position 103 of SEQ ID NO: 4),
a substitution of lysine with glutamic acid at position 399 of SEQ ID NO: 1 (position 112 of SEQ ID NO: 4),
a substitution of serine with arginine at position 476 of SEQ ID NO: 1 (position 189 of SEQ ID NO: 4), and
a substitution of asparagine with aspartic acid at position 487 of SEQ ID NO: 1 (position 200 of SEQ ID NO: 4).

(1) A PCR primer set for creating the saturation substitution library was designed. Specifically,
   SEQ ID NOS: 5 (Forward) and 6 (Reverse) for PCR primers for amino acid substitution of Ala330,
   SEQ ID NOS: 7 (Forward) and 8 (Reverse) for PCR primers for amino acid substitution of Tyr331,
   SEQ ID NOS: 9 (Forward) and 10 (Reverse) for PCR primers for amino acid substitution of Val332, and
   SEQ ID NOS: 11 (Forward) and 12 (Reverse) for PCR primers for amino acid substitution of Leu333
      were designed.
(2) A reaction solution having the composition shown in Table 1 was prepared. The reaction solution was then heat treated at 98°C for 5 minutes. PCR was performed by carrying out 30 cycles of a reaction consisting of a first step at 98°C for 10 seconds, a second step at 55°C for 5 seconds, and a third step at 72°C for 6 minutes per cycle, followed by heat treatment at 72°C for 5 minutes. For the amino acid substitution of Ala330, PCR primers consisting of the sequences set forth in SEQ ID NOS: 5 and 6 were used. For the amino acid substitution of Tyr331, PCR primers consisting of the sequences set forth in SEQ ID NOS: 7 and 8 were used. For the amino acid substitution of Val332, PCR primers consisting of the sequences set forth in SEQ ID NOS: 9 and 10 were used. For the amino acid substitution of Leu333, PCR primers consisting of the sequences set forth in SEQ ID NOS: 11 and 12 were used.

### [Table 1]

**Table 1**

| Composition | Volume |
|---|---|
| 10 ng/µLTemplate | 1 µL |
| 10 µM PCR forward primer | 1.5 µL |
| 10 µM PCR reverse primer | 1.5 µL |
| 10×KOD buffer(manufactured by TOYOBO CO., LTD.) | 5 µL |
| 2 mM dNTPs | 5 µL |
| 25 mM MgSO₄ | 3 µL |
| 1 U/µL KOD Plus (manufactured by TOYOBO CO., LTD.) | 1 µL |
| H₂O | up to 50 µL |

(3) The amplified PCR product was subjected to agarose gel electrophoresis and purified from the gel using a QIAquick Gel Extraction Kit (manufactured by QIAGEN).

(4) The resulting PCR product was treated with restriction enzyme DpnI (manufactured by NEB) at 37°C for 1.5 hours and then at 80°C for 20 minutes. The obtained polynucleotide was subjected to agarose gel electrophoresis and purified from the gel using a QIAquick Gel Extraction Kit (manufactured by QIAGEN).

(5) A reaction solution having the composition shown in Table 2 was prepared, and the DpnI-treated PCR product purified in (4) was subjected to ligation reaction at 25°C for 1.0 hour using T4 DNA ligase (manufactured by NEB) while being digested with restriction enzyme BsaI (manufactured by NEB).

### [Table 2]

**Table 2**

| Composition | Volume |
|---|---|
| 20 ng/µL PCR product | 5 µL |
| 10×CutSmart buffer(manufactured by NEB) | 1.5 µL |
| Bsa I(manufactured by NEB) | 1 µL |
| T4 DNA ligase buffer(manufactured by NEB) | 1.5 µL |
| T4 DNA ligase(manufactured by NEB) | 1 µL |
| H₂O | up to 15 µL |

(6) The E. coli BL21 (DE3) strain was transformed using the obtained ligation product and cultured in an LB plate medium containing 50 µg/mL kanamycin (37°C, 16 hours). The resulting colonies formed on the plate were used as a site-specific mutant library.

### Example 3: Screening of AVR10s Amino Acid Substitution Product (Part 1)

(1) The site-specific mutant library (transformants) prepared in Example 2 was inoculated into 300 µL of a 2×YT liquid medium containing 50 µg/mL kanamycin and subjected to shaking culture at 37°C overnight using a 96-well deep well plate.
(2) After culturing, 5 µL of the culture solution from (1) was subcultured into 500 µL of a 2×YT liquid medium containing 50 µg/mL kanamycin and 0.1 mM isopropyl-β-D-thiogalactopyranoside (IPTG) using a 96-well deep well plate. Further, the shaking culture was continued at 25°C overnight.
(3) After culturing, the culture supernatant obtained by centrifugation was diluted 10 times with 20 mM Tris-HCl buffer (pH 7.4) containing 150 mM sodium chloride.
(4) The binding between the AAV-binding protein in the culture supernatant prepared in (3) and the AAV vector (AAV5-EGFP) prepared in Example 1 was evaluated by the enzyme-linked immunosorbent assay (ELISA) method shown below.
   (4-1) AAV5-EGFP prepared in Example 1 was diluted 200 times with a 20 mM Tris-HCl buffer (pH 7.4) containing 150 mM sodium chloride, added at 100 µL/well to a 96-well microplate (manufactured by Thermo Fisher Scientific), and immobilized (at 4°C for 18 hours). After the immobilization was completed, blocking was performed with a 20 mM Tris-HCl buffer (pH 7.4) containing 2% (w/v) SKIM MILK (manufactured by Becton, Dickinson and Company) and 150 mM sodium chloride.
   (4-2) After washing with a washing buffer (0.05% [w/v] Tween 20 (trade name), 20 mM Tris-HCl buffer (pH 7.4) containing 150 mM sodium chloride), the culture supernatant prepared in (3) was added, thereby allowing the AAV-binding protein and AAV5-EGFP to react (at 30°C for 1 hour).
   (4-3) After the reaction was completed, the microplate was washed with the washing buffer, and an Anti-6His antibody (manufactured by Bethyl Laboratories) diluted to 100 ng/mL was added at 100 µL/well.
   (4-4) After reacting at 30°C for 1 hour and washing with the washing buffer, TMB Peroxidase Substrate (manufactured by KPL) was added at 50 µL/well. Color development was stopped by adding 1M phosphoric acid at 50 µL/well, and absorbance at 450 nm was measured using a microplate reader (manufactured by Tecan).
(5) About 400 transformants were evaluated, and transformants expressing AAV-binding proteins with enhanced binding activity to AAV5 compared with A VR10s (SEQ ID NO: 4) were selected.
(6) The selected transformants were cultured, and an expression vector was prepared using a QIAprep Spin Miniprep Kit (manufactured by QIAGEN).
(7) For a polynucleotide encoding an AAV-binding protein and its surrounding region in the expression vector prepared in (6), the nucleotide sequence was analyzed using a fully automatic DNA sequencer Genetic Analyzer 3500 (manufactured by Thermo Fisher Scientific), and the amino acid substitution site was identified. An oligonucleotide consisting of the sequence set forth in SEQ ID NO: 13 (5'-TAATACGACTCACTATAGGG-3') or SEQ ID NO: 14 (5'-ATGCTAGTTATTGCTCAGCGG-3') was used as a sequence primer upon the analysis.

Table 3 shows a list of the amino acid substitution sites for AVR10s and the binding activities to AAV5 (ratio when the above-described binding activity for AVR10s is set to 1) of the AAV-binding proteins expressed by the transformants selected in (5) above.

It is understood that the AAV-binding proteins, in which at least any one of amino acid substitutions of Ala330Cys (this notation indicates a substitution of alanine at position 330 of SEQ ID NO: 1 (position 43 of SEQ ID NO: 4) with cysteine; the same applies hereinafter), Ala330Phe, Ala330Leu, Ala330Arg, Ala330Val, Ala330Trp, Ala330Tyr, Tyr331Cys, Val332Trp, Val332Tyr, and Leu333Cys is present at the amino acid residues from serine (Ser) at position 312 to aspartic acid (Asp) at position 500 in the amino acid sequence set forth in SEQ ID NO: 1, have enhanced binding activity to AAV5, compared with the AAV-binding protein (AVR10s) without such an amino acid substitution.

Among them, AAV-binding proteins with any of the amino acid substitutions of Ala330Cys, Ala330Val, Ala330Tyr, and Val332Trp were found to have particularly enhanced binding activity to AAV5.

### [Table 3]

**Table 3**

| AAV-binding protein | | | Binding activity for AAV5 (vs AVR10s) |
|---|---|---|---|
| Name | Amino acid substitution site (vs AVR10s) | SEQ ID NO | |
| AVR10s_A330C | Ala330Cys | 15 | 1.94 |
| AVR10s_A330F | Ala330Phe. | 16 | 1.68 |
| AVR10s_A330L | Ala330Leu | 17 | 1.53 |
| AVR10s_A330R | Ala330Arg | 18 | 1.50 |
| AVR10s_A330V | Ala330Val | 19 | 1.83 |
| AVR10s_A330W | Ala330Trp | 20 | 1.46 |
| AVR10s_A330Y | Ala330Tyr | 21 | 2.19 |
| AVR10s_Y331C | Tyr331Cys | 22 | 1.30 |
| AVR10s_V332W | Val332Trp | 23 | 3.87 |
| AVR10s_V332Y | Val332Tyr | 24 | 1.50 |
| AVR10s_L333C | leu333Cys | 25 | 1.51 |
| AVR10s | (Reference) | 4 | 1 |

Among AAV-binding proteins with enhanced binding activity to AAV5 obtained in this Example, an AAV-binding protein with an amino acid substitution of Ala330Cys in AVR10s is named "AVR10s_A330C (SEQ ID NO: 15),"
an AAV-binding protein with an amino acid substitution of Ala330Phe in AVR10s is named "AVR10s_A330F (SEQ ID NO: 16),"
an AAV-binding protein with an amino acid substitution of Ala330Leu in AVR10s is named "AVR10s_A330L (SEQ ID NO: 17),"
an AAV-binding protein with an amino acid substitution of Ala330Arg in AVR10s is named "AVR10s_A330R (SEQ ID NO: 18),"
an AAV-binding protein with an amino acid substitution of Ala330Val in AVR10s is named "AVR10s_A330V (SEQ ID NO: 19),"
an AAV-binding protein with an amino acid substitution of Ala330Trp in AVR10s is named "AVR10s_A330W(SEQ ID NO: 20),"
an AAV-binding protein with an amino acid substitution of Ala330Tyr in AVR10s is named "AVR10s_A330Y (SEQ ID NO: 21),"
an AAV-binding protein with an amino acid substitution of Tyr331Cys in AVR10s is named "AVR10s_Y331C (SEQ ID NO: 22),"
an AAV-binding protein with an amino acid substitution of Val332Trp in AVR10s is named "AVR10s_V332W (SEQ ID NO: 23),"
an AAV-binding protein with an amino acid substitution of Val332Tyr in AVR10s is named "AVR10s_V332Y (SEQ ID NO: 24)," and
an AAV-binding protein with an amino acid substitution of Leu333Cys in AVR10s is named "AVR10s_L333C (SEQ ID NO: 25)."

A PelB signal peptide ranges from methionine (Met) at position 1 to alanine (Ala) at position 22, an AAV-binding protein (AVR10s amino acid substitution product) ranges from serine (Ser) at position 25 to aspartic acid (Asp) at position 213, and a tag sequence ranges from histidine (His) at position 214 to histidine (His) at position 219 in the polypeptides consisting of the amino acid sequences set forth in SEQ ID NOS: 15 to 25.

Example 4: Preparation of AAV-Binding Protein and Calculation of Decomposition Product Ratio
(1) A transformant capable of expressing the AAV-binding protein, which was obtained by transforming the E. coli BL21 (DE3) strain with a plasmid comprising a polynucleotide encoding any one of 11 kinds of AAV-binding proteins (AVR10s_A330C, AVR10s_A330F, AVR10s_A330L, AVR10s_A330R, AVR10s_A330V, AVR10s_A330W, AVR10s_A330Y, AVR10s_Y331C, AVR10s_V332W, AVR10s_V332Y, and AVR10s_L333C) obtained in Example 3 and AVR10s, was inoculated into 3 mL of a 2×YT liquid medium containing 50 µg/mL of kanamycin and precultured by aerobic shaking culture at 37°C overnight.
(2) Each preculture solution from (1) was inoculated at 2 mL into 200 mL of a 2×YT liquid medium supplemented with 50 µg/mL kanamycin in a 1-L baffled flask and subjected to aerobic shaking culture at 37°C.
(3) After 2.0 hours from the start of the culture, each flask was cooled on ice, IPTG was added to a final concentration of 0.1 mM, and then the aerobic shaking culture was continued at 25°C for 20 hours.
(4) After the culture was completed, bacterial cells were collected by centrifuging each culture solution at 4°C and 8000 rpm for 20 minutes.
(5) The bacterial cells collected in (4) were suspended in 20 mM Tris-HCl buffer (pH 7.4) containing 150 mM sodium chloride and 20 mM imidazole (hereinafter also referred to as "equilibration solution B") to 5 mL/1 g (bacterial cells). following which the bacterial cells were disrupted at 8°C for about 10 minutes with an output of about 150 W using an ultrasonic generator (Insonator 201M [manufactured by KUBOTA CORPORATION]). The cell disruption solutions were centrifuged twice at 8000 rpm for 20 minutes at 4°C, and each supernatant was collected.
(6) Each supernatant obtained in (5) was applied to an open column packed with 1.5 mL of Ni Sepharose 6 Fast Flow (manufactured by Cytiva), which had previously been equilibrated with the equilibration solution B. After washing with the equilibration solution B, elution was performed with 20 mM Tris-HCl buffer (pH 7.4) containing 0.5 M imidazole and 150 mM sodium chloride.
(7) Each eluate obtained in (6) was dialyzed with 20 mM HEPES (2-[4-(2-hydroxyethyl)-1-piperazinyl]ethanesulfonic acid) buffer (pH 7.4) containing 150 mM sodium chloride, thereby preparing an AAV-binding protein.
(8) The AAV-binding proteins obtained in (7) were subjected to a capillary electrophoresis system (manufactured by SCIEX), and the decomposition product rate [%] of each AAV-binding protein was calculated.

Table 4 shows the results. The AAV-binding proteins, in which at least any one of amino acid substitutions of Ala330Cys, Ala330Phe, Ala330Leu, Ala330Arg, Ala330Val, Ala330Trp, Ala330Tyr, Tyr331Cys, Val332Trp, Val332Tyr, and Leu333Cys is present at the amino acid residues from serine (Ser) at position 312 to aspartic acid (Asp) at position 500 in the amino acid sequence set forth in SEQ ID NO: 1, have a decreased proportion of an AAV-binding protein-derived decomposition product, compared with the AAV-binding protein without the amino acid substitution (AVR10s with a decomposition rate of 30.0%), suggesting enhanced efficiency of producing an AAV-binding protein using recombinant E. coli.

Among them, the AAV-binding protein (AVR10s_A330V or AVR10s_Y331C), in which an amino acid substitution of Ala330Val or Tyr331Cys is present, has a significantly decreased proportion of an AAV-binding protein-derived decomposition product (AVR10s_A330V with a decomposition rate of 1.9%, AVR10s_Y331C with a decomposition rate of 2.0%), promising that the efficiency of producing an AAV-binding protein using recombinant E. coli will be remarkably enhanced.

### [Table 4]

**Table 4**

| AAV-binding protein | | | Decomposition product rate [%] |
|---|---|---|---|
| Name | Amino acid substitution site (vs AVR10s) | SEQ ID NO | |
| AVR10s_A330C | Ala330Cys | 15 | 11.6 |
| AVR10s_A330F | Ala330Phe | 16 | 5.4 |
| AVR10s_A330L | Ala330Leu | 17 | 4.8 |
| AVR10s_A330R | Ala330Arg | 18 | 15.0 |
| AVR10s_A330V | Ala330Val | 19 | 1.9 |
| AVR10s_A330W | Ala330Trp | 20 | 6.1 |
| AVR10s_A330Y | Ala330Tyr | 21 | 17.2 |
| AVR10s_Y331C | Tyr331Cys | 22 | 2.0 |
| AVR10s_V332W | Val332Trp | 23 | 9.5 |
| AVR10s_V332Y | Val332Tyr | 24 | 11.5 |
| AVR10s_L333C | Leu333Cys | 25 | 7.0 |
| AVR10s | (Reference) | 4 | 30.0 |

### Example 5: Site-Specific Amino Acid Substitution to AAV-Binding Protein and Library Preparation (Part 2)

A saturation substitution product library for amino acid residues corresponding to glutamine (Gln347) at position 347 (position 60 of SEQ ID NO: 4), leucine (Leu348) at position 348 (position 61 of SEQ ID NO: 4), and isoleucine (Ile349) at position 349 (position 62 of SEQ ID NO: 4) was created using the vector pET-AVR10s (WO2021/106882) capable of expressing a polypeptide (SEQ ID NO: 4) comprising the AAV-binding protein AVR10s as a template.
(1) A PCR primer set for creating the saturation substitution library was designed. Specifically,
   SEQ ID NOS: 26 (Forward) and 27 (Reverse) for PCR primers for amino acid substitution of Gln347,
   SEQ ID NOS: 28 (Forward) and 29 (Reverse) for PCR primers for amino acid substitution of Leu348, and
   SEQ ID NOS: 30 (Forward) and 31 (Reverse) for PCR primers for amino acid substitution of Ile349,
      were designed.
(2) PCR was performed in the same manner as in Example 2 (2) except that for the amino acid substitution of Gln347, PCR primers consisting of the sequences set forth in SEQ ID NOS: 26 and 27 were used, for the amino acid substitution of Leu348, PCR primers consisting of the sequences set forth in SEQ ID NOS: 28 and 29 were used, and for the amino acid substitution of Ile349, PCR primers consisting of the sequences set forth in SEQ ID NOS: 30 and 31 were used.
(3) After purifying the PCR products amplified by the method described in Example 2 (3) and (4), a ligation reaction was performed by the method described in Example 2 (5).
(4) The E. coli BL21 (DE3) strain was transformed using the obtained ligation product and cultured in an LB plate medium containing 50 µg/mL kanamycin (37°C, 16 hours). The resulting colonies formed on the plate were used as a site-specific mutant library.

Example 6: Screening of AVR10s Amino Acid Substitution Product (Part 2)
(1) After culturing the site-specific mutant library (transformants) created in Example 5 using the method described in Example 3 (1) and (2), the culture supernatant obtained by centrifugation was diluted 10 times with 20 mM Tris-HCl buffer (pH 7.4) containing 150 mM sodium chloride.
(2) The binding between the AAV-binding protein in the culture supernatant diluted solution prepared in (1) and the AAV vector (AAV5-EGFP) prepared in Example 1 was evaluated by the ELISA method described in Example 3 (4).
(3) About 400 transformants were evaluated, and transformants expressing AAV-binding proteins with enhanced binding activity to AAV5 compared with A VR10s (SEQ ID NO: 4) were selected.
(4) The selected transformants were cultured, and an expression vector was prepared using a QIAprep Spin Miniprep Kit (manufactured by QIAGEN).
(5) For a polynucleotide encoding an AAV-binding protein and its surrounding region in the expression vector prepared in (4), the nucleotide sequence was analyzed using the method described in Example 3 (7), and the amino acid substitution site was identified.

Table 5 shows a list of the amino acid substitution sites for AVR10s and the binding activities to AAV5 (ratio when the above-described binding activity for AVR10s is set to 1) of the AAV-binding proteins expressed by the transformants selected in (3) above.

It is understood that the AAV-binding proteins, in which at least any one of amino acid substitutions of Gln347Ser, Leu348Cys, and Ile349Asn is present at the amino acid residues from serine (Ser) at position 312 to aspartic acid (Asp) at position 500 in the amino acid sequence set forth in SEQ ID NO: 1, have enhanced binding activity to AAV5, compared with the AAV-binding protein without the amino acid substitution (AVR10s).

### [Table 5]

**Table 5**

| AAV-binding protein | | | Binding activity for AAV5 (vs AVR10s) |
|---|---|---|---|
| Name | Amino acid substitution site (vs AVR10s) | SEQ ID NO | |
| AVR10s_Q347S | Gln347Ser | 32 | 1.73 |
| AVR10s_L348C | Leu348Cys | 33 | 1.19 |
| AVR10s_I349N | Ile349Asn | 34 | 1.13 |
| AVR10s | (Reference) | 4 | 1 |

Among AAV-binding proteins with enhanced binding activity to AAV5 obtained in this Example,
an AAV-binding protein with an amino acid substitution of Gln347Ser in AVR10s is named "AVR10s_Q347S (SEQ ID NO: 32),"
an AAV-binding protein with an amino acid substitution of Leu348Cys in AVR10s is named "AVR10s_L348C (SEQ ID NO: 33)," and
an AAV-binding protein with an amino acid substitution of Ile349Asn in AVR10s is named "AVR10s_I349N (SEQ ID NO: 34)."

A PelB signal peptide ranges from methionine (Met) at position 1 to alanine (Ala) at position 22, an AAV-binding protein (AVR10s amino acid substitution product) ranges from serine (Ser) at position 25 to aspartic acid (Asp) at position 213, and a tag sequence ranges from histidine (His) at position 214 to histidine (His) at position 219 in the polypeptides consisting of the amino acid sequences set forth in SEQ ID NOS: 32 to 34.

Example 7: Preparation of AAV-Binding Protein and Calculation of Decomposition Product Ratio (Part 2)
(1) From a transformant capable of expressing the AAV-binding protein, which was obtained by transforming the E. coli BL21 (DE3) strain with a plasmid comprising a polynucleotide encoding any one of three kinds of AAV-binding proteins (AVR10s_Q347S, AVR10s_L348C, and AVR10s_I349N) obtained in Example 6 and AVR10s, the protein was prepared by the method described in Example 4 (1) to (7).
(2) The AAV-binding proteins obtained in (1) were subjected to a capillary electrophoresis system (manufactured by SCIEX), and the decomposition product rate [%] of each AAV-binding protein was calculated.

Table 6 shows the results. The AAV-binding proteins, in which at least any one of amino acid substitutions of Gln347Ser, Leu348Cys, and Ile349Asn is present at the amino acid residues from serine (Ser) at position 312 to aspartic acid (Asp) at position 500 in the amino acid sequence set forth in SEQ ID NO: 1, have a decreased proportion of an AAV-binding protein-derived decomposition product, compared with the AAV-binding protein without the amino acid substitution (AVR10s with a decomposition rate of 18.4%), suggesting enhanced efficiency of producing an AAV-binding protein using recombinant E. coli.

### [Table 6]

**Table 6**

| AAV-binding protein | | | Decomposition product rate [%] |
|---|---|---|---|
| Name | Amino acid substitution site (vs, AVR10s) | SEQ ID NO | |
| AVR10s_Q3475 | Gln347Ser | 32 | 5.98 |
| AVR10s_L348C | Leu348Cys | 33 | 4.63 |
| AVR10s_I349N | Ile349Asn | 34 | 4.57 |
| AVR10s | (Reference) | 4 | 18.4 |

Example 8: Preparation of AAV Vector (Part 2)
(1) The E. coli JM109 strain was transformed with a plasmid pRC2-mi342 Vector (manufactured by Takara Bio Inc.) containing a polynucleotide encoding the AAV serotype 2 (AAV2) and a pHelper Vector (manufactured by Takara Bio Inc.). The obtained transformant was cultured by the method described in Example 1 (4).
(2) After collecting bacterial cells by centrifuging the culture solution in (1), the pRC2-mi342 Vector and pHelper Vector were prepared in large quantities from the collected bacterial cells using a Plasmid Mega Kit (manufactured by QIAGEN).
(3) HEK293T cells were cultured in five T-225 flasks (manufactured by Thermo Fisher Scientific) containing 40 mL of a D-MEM medium (manufactured by FUJIFILM Wako Pure Chemical Corporation) containing 10% (v/v) bovine serum.
(4) Using a TransIT-VirusGEN Transfection Reagent (manufactured by Takara Bio Inc.), the pRC2-mi342 Vector and pHelper Vector prepared in (2) were introduced into HEK293T cells cultured in (3). The cells were statically cultured for 3 days in 5% carbon dioxide at 37°C.
(5) The cells cultured in (4) were collected, extracted using an AAVpro Purification Kit (manufactured by Takara Bio Inc.), and purified, thereby obtaining VLP2 (virus-like particles, capsid protein particles of AAV serotype 2). About 1 mL of a VLP2 purification solution was prepared from the five T-225 flasks.

### Example 9: Creation and Screening of Mutation Library of AAV-Binding Protein (Part 1)

Among the AAV-binding proteins evaluated in Examples 2 and 3, a protein with an amino acid substitution of Ala330Val (SEQ ID NO: 19, hereinafter also referred to as "AVR11a") was selected, and a mutation was randomly introduced into the polynucleotide portion encoding the protein using error-prone PCR.
(1) Error-prone PCR was performed using the vector pET-AVR11a capable of expressing the polypeptide (SEQ ID NO: 19) comprising AVR11a prepared in Example 2 as a template. A reaction solution having the composition shown in Table 7 was prepared. The reaction solution was then heat treated at 98°C for 2 minutes. Error-prone PCR was performed by carrying out 30 cycles of a reaction consisting of a first step at 98°C for 30 seconds, a second step at 55°C for 20 seconds, and a third step at 72°C for 90 seconds per cycle, followed by heat treatment at 72°C for 5 minutes. Mutations were successfully introduced into the polynucleotide encoding the AAV-binding protein by error-prone PCR, and the average mutation introduction rate was 1.0 amino acid mutations per molecule.

### [Table 7]

**Table 7**

| Composition | Volume |
|---|---|
| 5 ng/µL Template | 1 µL |
| 10 µM PCR primer(SEQ ID NO: 13) | 2 µL |
| 10 µM PCR primer(SEQ ID NO: 14) | 2 µL |
| 10 mM MnCl₂ | 1.5 µL |
| 2.5 mM dNTPs | 4 µL |
| 10×Ex Taq Buffer(manufacured by Takara Bio Inc.) | 5 µL |
| GoTaq polymerase(manufactured by Promega Corporation) | 0.5 µL |
| H₂O | up to 50 µL |

(2) The PCR product obtained in (1) was purified, digested with restriction enzymes NcoI and XhoI, and ligated into an expression vector pET26b (manufactured by Merck Millipore), which had previously been digested with the same restriction enzymes.

(3) After the ligation reaction was completed, the E. coli BL21 (DE3) strain was transformed with the reaction solution and cultured in an LB plate medium containing 50 µg/mL kanamycin (37°C, 18 hours). The resulting colonies formed on the plate were used as a random mutant library.

(4) The random mutant library (transformants) prepared in (3) was inoculated into 200 µL of a 2YT liquid medium containing 50 µg/mL kanamycin and subjected to shaking culture at 37°C overnight using a 96-well deep well plate.

(5) The culture solution in (4) was centrifuged, and the resulting culture supernatant was diluted twice with ultrapure water. The diluted culture solution in an amount of 60 µL was mixed with 60 µL of a 0.1 M sodium hydroxide aqueous solution and alkali-treated at 61.2°C for 15 minutes.

(6) The binding activity between the AAV-binding protein and VLP2 when the treatment in (5) was performed and the binding activity between the AAV-binding protein and VLP2 when the treatment in (5) was not performed were measured using the ELISA method described in Example 3 (4). The residual activity was calculated by dividing the binding activity between the AAV-binding protein and VLP2 when heat treatment was performed by the binding activity between the AAV-binding protein and VLP2 when heat treatment was not performed.

(7) A random mutant library of about 1800 strains was evaluated using the method described in (6). Transformants expressing an AAV-binding protein with enhanced residual activity compared with the parent molecule AVR11a were selected from the library. The selected transformants were cultured, and an expression vector was prepared using a QIAprep Spin Miniprep Kit (manufactured by QIAGEN).

(8) The nucleotide sequence of the polynucleotide region encoding the AAV-binding protein inserted into the obtained expression vector was analyzed by the method described in Example 3 (7), and the amino acid mutation site was identified.

Tables 8 and 9 show lists of the amino acid substitution sites and the residual activities (%) after alkali treatment of the AAV-binding proteins expressed by the transformants selected in (7) above to AVR11a.

It can be said that the AAV-binding proteins, in which at least any one of amino acid substitutions of Ser312Pro, Ala313Val, Glu315Gly, Glu315Val, Gln318Arg, Lys323Met, Asn324His, Val326Glu, Gln327Leu, Leu328Gln, Leu328Arg, Leu328Pro, Asn329Tyr, Val332Glu, Val332Ala, Leu333Pro, Gln334Leu, Gln334His, Glu335Val, Pro337Gln, Lys338Glu, Glu340Asp, Thr341Ala, Thr343Pro, Thr343Ala, Thr343Ser, Asp345Gly, Ile349Thr, Thr350Ala, Asp354Val, Tyr355His, Met359Ile, Met359Leu, Glu360Asp, Ile366Thr, Leu367Val, Lys368Asn, Leu369Gln, Leu376Pro, Phe379Tyr, Lys380Asn, Lys380Arg, Ile(Val)382Asp (this notation indicates that isoleucine at position 382 of SEQ ID NO: 1 has been replaced with valine and then with aspartic acid; the same applies hereinafter), Ile(Val)382Ala, Glu384Val, Ala388Thr, His389Asp, His389Arg, His389Asn, His389Leu, Gly(Ser)390Asn, Gly392Arg, Val394Ala, Val394Asp, Asn395Asp, Val396Gly, Val398Ala, Arg403Ser, Arg403His, Lys404Ala, Arg406Ser, Ile409Asn, Ile411Thr, Ile411Leu, Val412Ile, Phe416Ser, Ile419Phe, Ile419Val, Ser420Thr, Thr423Ala, Ser425Gly, Ile428Val, Gly430Ala, Thr434Ala, Asp437Asn, Val440Ala, Glu446Asp, Lys448Glu, Lys448Asn, Leu451Ile, Glu453Lys, Lys455Glu, Lys455Arg, Lys464Asn, Lys464Glu, Val469Glu, Gly471Cys, Asn472Tyr, Asn472Asp, Tyr473His, Ser(Arg)476Gly, Thr478Ala, Val479Ala, Val480Asp, Val480Ala, Gly484Ser, Thr486Ser, Ser488Thr, Thr489Ala, Thr489Ser, Asn496Tyr, and Asn496Asp is present at the amino acid residues from serine (Ser) at position 312 to aspartic acid (Asp) at position 500 in the amino acid sequence set forth in SEQ ID NO: 1, have enhanced stability to alkali compared with AVR11a.

### [Table 8]

**Table 8**

| Amino acid substitution | Residual activity [%] | Amino acid substitution | **Residual** activity [%] |
|---|---|---|---|
| Ala313Val | 146.9 | Lys404Ala | 28.9 |
| Gln334Leu | 70.6 | Ile419Phe | 26.4 |
| Thr341Ala | 21.1 | Val440Ala | 28.3 |
| Met359Ile | 32.0 | Glu446Asp | 79.6 |
| Ile366Thr | 111.2 | Lys448Glu | 23.7 |
| Leu367Val | 27.5 | Glu453Lys | 21.6 |
| Leu369Gln | 37.6 | Lys464Asn | 100.7 |
| Phe379Tyr | 30.3 | Lys464Glu | 26.5 |
| Lys380Asn | 40.6 | Asn472Tyr | 22.3 |
| Val394Ala | 137.5 | Thr478Ala | 41.7 |
| Val398Ala | 45.6 | Val480Asp | 82.6 |
| | | AVR11a | 15.0 |

### [Table 9]

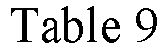

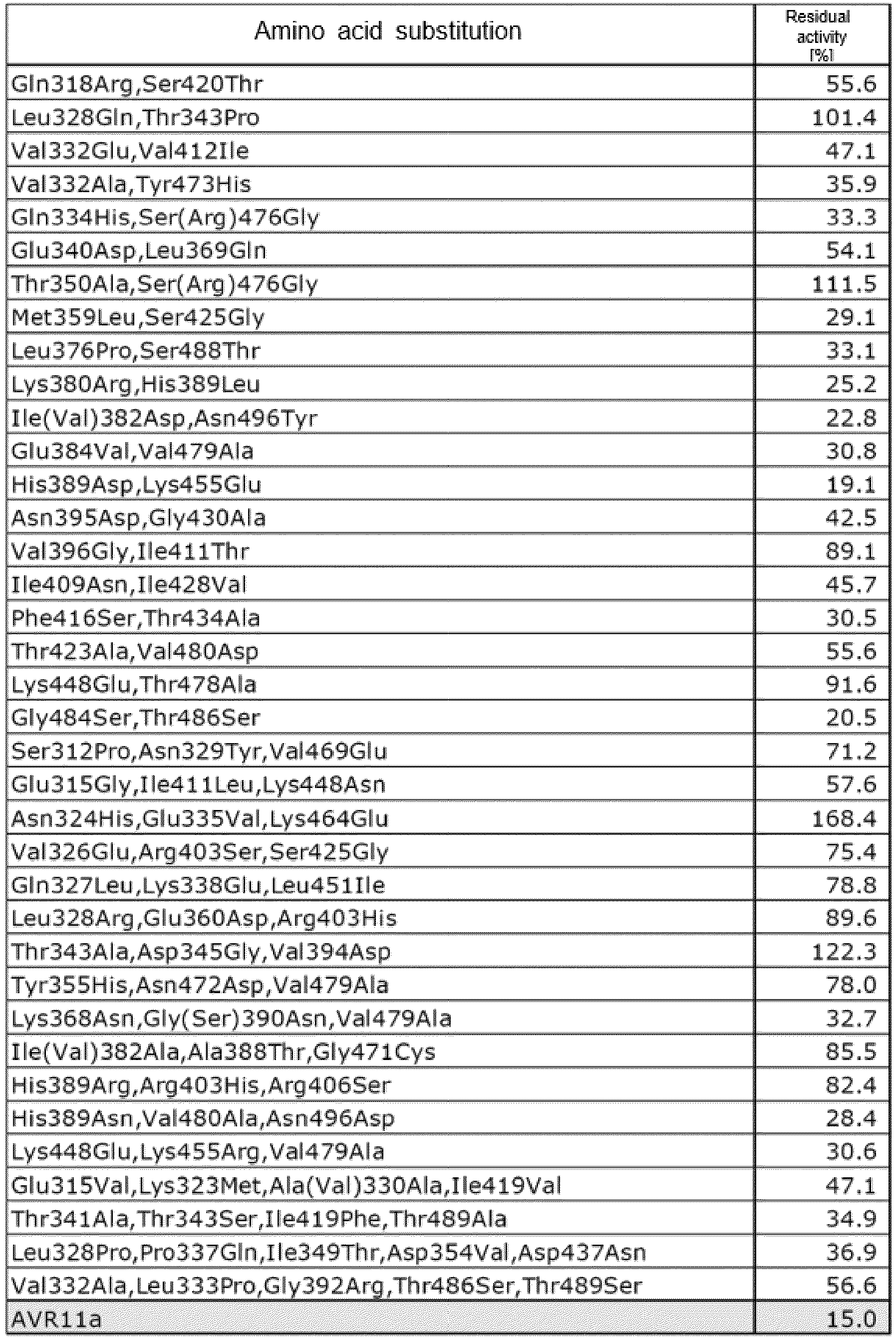

### Example 10: Preparation of Aggregate of Amino Acid Substitutions in AVR1 1a

It was attempted to aggregate amino acid substitutions, which were found in Example 9 to be involved in enhancing the alkali stability of AAV-binding proteins, in AVR1 1a (SEQ ID NO: 19), thereby further enhancing the alkali stability. Specifically, 11 types of AAV-binding proteins shown in (a) to (k) below were designed and prepared.
(a) Protein with the Asn324His amino acid substitution introduced into AVR1 1a (SEQ ID NO: 54, named "AVR12a"),
(b) Protein with the Glu335Val amino acid substitution introduced into AVR1 1a (SEQ ID NO: 55, named "AVR12b"),
(c) Protein with the Asn324His and Glu335Val amino acid substitutions introduced into AVR1 1a (SEQ ID NO: 56, named "AVR13"),
(d) Protein with the Asn324His, Thr341Ala, and Glu335Val amino acid substitutions introduced into AVR1 1a (SEQ ID NO: 57, named "AVR14a"),
(e) Protein with the Asn324His, Glu335Val, and Gln334Leu amino acid substitutions introduced into AVR1 1a (SEQ ID NO: 58, named "AVR14b"),
(f) Protein with the Asn324His, Glu335Val, and Met359Ile amino acid substitutions introduced into AVR1 1a (SEQ ID NO: 59, named "AVR14c"),
(g) Protein with the Asn324His, Glu335Val, and Phe379Tyr amino acid substitutions introduced into AVR1 1a (SEQ ID NO: 60, named "AVR14d"),
(h) Protein with the Asn324His, Gln334Leu, Glu335Val, and Thr341Ala amino acid substitutions introduced into AVR1 1a (SEQ ID NO: 61, named "AVR15a"),
(i) Protein with the Asn324His, Gln334Leu, Glu335Val, and Met359Ile amino acid substitutions introduced into AVR1 1a (SEQ ID NO: 62, named "AVR15b"),
(j) Protein with the Asn324His, Gln334Leu, Glu335Val, and Phe379Tyr amino acid substitutions introduced into AVR1 1a (SEQ ID NO: 63, named "AVR15c2), and
(k) Protein with the Asn324His, Gln334Leu, Glu335Val, Thr341Ala, and Phe379Tyr amino acid substitutions introduced into AVR1 1a (SEQ ID NO: 65, named "AVR16").

Hereinafter, a method of preparing the 11 types of AAV-binding proteins shown in (a) to (k) above will be explained.

### (a) AVR12a

This protein was prepared by selecting Asn324His from among the amino acid substitutions involved in alkali stability revealed in Example 9 and introducing the amino acid substitution into AVR1 1a (SEQ ID NO: 19).
(a-1) PCR was performed in the same manner as in Example 2 (2), except that a vector pET-AVR11a capable of expressing a polypeptide (SEQ ID NO: 19) comprising AVR1 1a was used as a template, and oligonucleotides consisting of the sequences set forth in SEQ ID NO: 44 (5'-TTTT[GGTCTC]ACATTCAGTTGTACTTCGTGCTTCGGCAG-3') and SEQ ID NO: 45 (5'-TTTT[GGTCTC]AAATGTTTATGTGCTGCAGGAACCACCGAAAG-3') (the square brackets in SEQ ID NOS: 44 and 45 indicate the restriction enzyme BsaI recognition sequence) were used as PCR primers (Forward: SEQ ID NO: 44; Reverse: SEQ ID NO: 45).
(a-2) After purifying the PCR products amplified by the method described in Example 2 (3) and (4), a ligation reaction was performed by the method described in Example 2 (5).
(a-3) The E. coli BL21 (DE3) strain was transformed using the obtained ligation product. After the resulting transformant was cultured in an LB medium supplemented with 50 µg/mL kanamycin, the plasmid was extracted from the bacterial cells (transformants) recovered by centrifugation, thereby obtaining a plasmid (expression vector) pET-AVR12a comprising a polynucleotide encoding AVR12a with 12 amino acid substitutions in the wild-type AAV-binding protein.
(a-4) The nucleotide sequence of pET-AVR12a was analyzed and confirmed in the same manner as in Example 3 (7).

The amino acid sequence of AVR12a with a signal sequence and a polyhistidine tag is set forth in SEQ ID NO: 54. A PelB signal peptide ranges from methionine (Met) at position 1 to alanine (Ala) at position 22, an AAV-binding protein AVR12a (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 54. In addition, aspartic acid of Val317Asp is present at position 30, histidine of Asn324His is present at position 37, valine of Ala330Val is present at position 43, serine of Tyr342Ser is present at position 55, glutamic acid of Lys362Glu is present at position 75, asparagine of Lys371Asn is present at position 84, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200 in SEQ ID NO: 54.

### (b) AVR12b

This protein was prepared by selecting Glu335Val from among the amino acid substitutions involved in enhancing alkali stability revealed in Example 9 and introducing the amino acid substitution into AVR11a (SEQ ID NO: 19).
(b-1) PCR was performed in the same manner as in Example 2 (2), except that pET-AVR11a described above was used as a template and, oligonucleotides consisting of the sequences set forth in SEQ ID NO: 46 (5'-TTTT[GGTCTC]ACATTCAGTTGTACTTCGTTCTTCGGCAG-3') and SEQ ID NO: 47 (5'-TTTT[GGTCTC]AAATGTTTATGTGCTGCAGGTACCACCGAAAG-3') (the square brackets in SEQ ID NOS: 46 and 47 indicate the restriction enzyme BsaI recognition sequence) were used as PCR primers (Forward: SEQ ID NO: 46; Reverse: SEQ ID NO: 47).
(b-2) A plasmid (expression vector) pET-AVR12b comprising a polynucleotide encoding AVR12b with 12 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR12b with a signal sequence and a polyhistidine tag is set forth in SEQ ID NO: 55. A PelB signal peptide ranges from methionine (Met) at position 1 to alanine (Ala) at position 22, an AAV-binding protein AVR12b (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 55. In addition, aspartic acid of Val317Asp is present at position 30, valine of Ala330Val is present at position 43, valine of Glu335Val is present at position 48, serine of Tyr342Ser is present at position 55, glutamic acid of Lys362Glu is present at position 75, asparagine of Lys371Asn is present at position 84, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200 in SEQ ID NO: 55.

### (c) AVR13

This protein was prepared by selecting Asn324His and Glu335Val from among the amino acid substitutions involved in enhancing alkali stability revealed in Example 9 and introducing the amino acid substitutions into AVR11a (SEQ ID NO: 19).
(c-1) PCR was performed in the same manner as in Example 2 (2), except that pET-AVR11a described above was used as a template, and oligonucleotides consisting of the sequences set forth in SEQ ID NO: 48 (5'-TTTT[GGTCTC]ACATTCAGTTGTACTTCGTGCTTCGGCAG-3') and SEQ ID NO: 49 (5'-TTTT[GGTCTC]AAATGTTTATGTGCTGCAGGTACCACCGAAAG-3') (the square brackets in SEQ ID NOS: 48 and 49 indicate the restriction enzyme BsaI recognition sequence) were used as PCR primers (Forward: SEQ ID NO: 48; Reverse: SEQ ID NO: 49).
(c-2) A plasmid (expression vector) pET-AVR13 comprising a polynucleotide encoding AVR13 with 13 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR13 with a signal sequence and a polyhistidine tag is set forth in SEQ ID NO: 56. A PelB signal peptide ranges from methionine (Met) at position 1 to alanine (Ala) at position 22, an AAV-binding protein AVR13(corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 56. In addition, aspartic acid of Val317Asp is present at position 30, histidine of Asn324His is present at position 37, valine of Ala330Val is present at position 43, valine of Glu335Val is present at position 48, serine of Tyr342Ser is present at position 55, glutamic acid of Lys362Glu is present at position 75, asparagine of Lys371Asn is present at position 84, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200 in SEQ ID NO: 56.

### (d) AVR14a

This protein is a protein for which Asn324His, Glu335Val, and Thr341Ala were selected from among the amino acid substitutions involved in enhancing alkali stability revealed in Example 9, and the amino acid substitutions were aggregated into AVR11a (SEQ ID NO: 19). Specifically, AVR14a was prepared by introducing mutations resulting in amino acid substitutions of Asn324His and Glu335Val into a polynucleotide (SEQ ID NO: 38) encoding the protein obtained in Example 9, in which an amino substitution of Thr341Ala was introduced into AVR11a (named "AVR11a_T341A," SEQ ID NO: 39).
(d-1) PCR was performed in the same manner as in (c-1), except that the vector pET-AVR11a_T341A capable of expressing the polypeptide (SEQ ID NO: 39) comprising AVR11a_T341A was used as a template.
(d-2) A plasmid (expression vector) pET-AVR14a comprising a polynucleotide encoding AVR14a with 14 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR14a with a signal sequence and a polyhistidine tag is set forth in SEQ ID NO: 57. A PelB signal peptide ranges from methionine (Met) at position 1 to alanine (Ala) at position 22, an AAV-binding protein AVR14a(corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 57. In addition, aspartic acid of Val317Asp is present at position 30, histidine of Asn324His is present at position 37, valine of Ala330Val is present at position 43, valine of Glu335Val is present at position 48, alanine of Thr341Ala is present at position 54, serine of Tyr342Ser is present at position 55, glutamic acid of Lys362Glu is present at position 75, asparagine of Lys371Asn is present at position 84, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200 in SEQ ID NO: 57.

### (e) AVR14b

This protein is a protein for which Asn324His, Gln334Leu, and Glu335Val were selected from among the amino acid substitutions involved in enhancing alkali stability revealed in Example 9, and the amino acid substitutions were aggregated in AVR11a (SEQ ID NO: 19). Specifically, AVR14b was prepared by introducing mutations resulting in amino acid substitutions of Asn324His and Glu335Val into a polynucleotide (SEQ ID NO: 36) encoding the protein obtained in Example 9, in which an amino substitution of Gln334Leu was introduced into AVR11a (named "AVR11a_Q334L," SEQ ID NO: 37).
(e-1) PCR was performed in the same manner as in (c-1), except that the vector pET-AVR_Q334L capable of expressing the polypeptide (SEQ ID NO: 37) comprising AVR11a_Q334L was used as a template.
(e-2) A plasmid (expression vector) pET-AVR14b comprising a polynucleotide encoding AVR14b with 14 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR14b with a signal sequence and a polyhistidine tag is set forth in SEQ ID NO: 58. A PelB signal peptide ranges from methionine (Met) at position 1 to alanine (Ala) at position 22, an AAV-binding protein AVR14b(corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 58. In addition, aspartic acid of Val317Asp is present at position 30, histidine of Asn324His is present at position 37, valine of Ala330Val is present at position 43, leucine of Gln334Leu is present at position 47, valine of Glu335Val is present at position 48, serine of Tyr342Ser is present at position 55, glutamic acid of Lys362Glu is present at position 75, asparagine of Lys371Asn is present at position 84, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200 in SEQ ID NO: 58.

### (f) AVR14c

This protein is a protein for which Asn324His, Glu335Val, and Met359Ile were selected from among the amino acid substitutions involved in enhancing alkali stability revealed in Example 9, and the amino acid substitutions were aggregated in AVR11a (SEQ ID NO: 19). Specifically, AVR14c was prepared by introducing mutations resulting in amino acid substitutions of Asn324His and Glu335Val into a polynucleotide (SEQ ID NO: 40) encoding the protein obtained in Example 9, in which an amino substitution of Met359Ile was introduced into AVR11a (named "AVR11a_M359I," SEQ ID NO: 41).
(f-1) PCR was performed in the same manner as in (c-1), except that the vector pET-AVR_M359I capable of expressing the polypeptide (SEQ ID NO: 41) comprising AVR11a _M3 591 was used as a template.
(f-2) A plasmid (expression vector) pET-AVR14c comprising a polynucleotide encoding AVR14c with 14 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR14c with a signal sequence and a polyhistidine tag is set forth in SEQ ID NO: 59. A PelB signal peptide ranges from methionine (Met) at position 1 to alanine (Ala) at position 22, an AAV-binding protein AVR14c(corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 59. In addition, aspartic acid of Val317Asp is present at position 30, histidine of Asn324His is present at position 37, valine of Ala330Val is present at position 43, valine of Glu335Val is present at position 48, serine of Tyr342Ser is present at position 55, isoleucine of Met359Ile is present at position 72, glutamic acid of Lys362Glu is present at position 75, asparagine of Lys371Asn is present at position 84, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200 in SEQ ID NO: 59.

### (g) AVR14d

This protein is a protein for which Asn324His, Glu335Val, and Phe379Tyr were selected from among the amino acid substitutions involved in enhancing alkali stability revealed in Example 9, and the amino acid substitutions were aggregated in AVR11a (SEQ ID NO: 19). Specifically, AVR14d was prepared by introducing mutations resulting in amino acid substitutions of Asn324His and Glu335Val into a polynucleotide (SEQ ID NO: 42) encoding the protein obtained in Example 9, in which an amino substitution of Phe379Tyr was introduced into AVR11a (named "AVR11a_F379Y," SEQ ID NO: 43).
(g-1) PCR was performed in the same manner as in (c-1), except that the vector pET-AVR_F379Y capable of expressing the polypeptide (SEQ ID NO: 41) comprising AVR11a_F379Y was used as a template.
(g-2) A plasmid (expression vector) pET-AVR14d comprising a polynucleotide encoding AVR14d with 14 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR14d with a signal sequence and a polyhistidine tag is set forth in SEQ ID NO: 60. A PelB signal peptide ranges from methionine (Met) at position 1 to alanine (Ala) at position 22, an AAV-binding protein AVR14d(corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 60. In addition, aspartic acid of Val317Asp is present at position 30, histidine of Asn324His is present at position 37, valine of Ala330Val is present at position 43, valine of Glu335Val is present at position 48, serine of Tyr342Ser is present at position 55, glutamic acid of Lys362Glu is present at position 75, asparagine of Lys371Asn is present at position 84, tyrosine of Phe379Tyr is present at position 92, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200 in SEQ ID NO: 60.

### (h) AVR15a

This protein is a protein for which Asn324His, Gln334Leu, Glu335Val, and Thr341Ala were selected from among the amino acid substitutions involved in enhancing alkali stability revealed in Example 9, and the amino acid substitutions were aggregated in AVR11a (SEQ ID NO: 19). Specifically, AVR15a was prepared by introducing mutations resulting in amino acid substitutions of Asn324His, Gln334Leu, and Glu335Val into a polynucleotide (SEQ ID NO: 38) encoding AVR11a_T341A (SEQ ID NO: 39).
(h-1) PCR was performed in the same manner as in Example 2 (2), except that pET-AVR_T341A described above was used as a template, and oligonucleotides consisting of the sequences set forth in SEQ ID NO: 50 (5'-TTTT[GGTCTC]ACATTCAGTTGTACTTCGTGCTTCGGCAG-3') and SEQ ID NO: 51 (5'-TTTT[GGTCTC]AAATGTTTATGTGCTGCTGGTACCACCGAAAG-3') (the square brackets in SEQ ID NOS: 50 and 51 indicate the restriction enzyme BsaI recognition sequence) were used as PCR primers (Forward: SEQ ID NO: 50; Reverse: SEQ ID NO: 51).
(h-2) A plasmid (expression vector) pET-AVR15a comprising a polynucleotide encoding AVR15a with 15 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR15a with a signal sequence and a polyhistidine tag is set forth in SEQ ID NO: 61. A PelB signal peptide ranges from methionine (Met) at position 1 to alanine (Ala) at position 22, an AAV-binding protein AVR15a(corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 61. In addition, aspartic acid of Val317Asp is present at position 30, histidine of Asn324His is present at position 37, valine of Ala330Val is present at position 43, leucine of Gln334Leu is present at position 47, valine of Glu335Val is present at position 48, alanine of Thr341Ala is present at position 54, serine of Tyr342Ser is present at position 55, glutamic acid of Lys362Glu is present at position 75, asparagine of Lys371Asn is present at position 84, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200 in SEQ ID NO: 61.

### (i) AVR15b

This protein is a protein for which Asn324His, Gln334Leu, Glu335Val, and Met359Ile were selected from among the amino acid substitutions involved in enhancing alkali stability revealed in Example 9, and the amino acid substitutions were aggregated in AVR11a (SEQ ID NO: 19). Specifically, AVR15b was prepared by introducing mutations resulting in amino acid substitutions of Asn324His, Gln334Leu, and Glu335Val into a polynucleotide (SEQ ID NO: 40) encoding AVR11a_M359I (SEQ ID NO: 41).
(i-1) PCR was performed in the same manner as in (h-1), except that the above-described pET-AVR11a_M359I was used as a template.
(i-2) A plasmid (expression vector) pET-AVR15b comprising a polynucleotide encoding AVR15b with 15 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR15b with a signal sequence and a polyhistidine tag is set forth in SEQ ID NO: 62. A PelB signal peptide ranges from methionine (Met) at position 1 to alanine (Ala) at position 22, an AAV-binding protein AVR15b (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 62. In addition, aspartic acid of Val317Asp is present at position 30, histidine of Asn324His is present at position 37, valine of Ala330Val is present at position 43, leucine of Gln334Leu is present at position 47, valine of Glu335Val is present at position 48, serine of Tyr342Ser is present at position 55, isoleucine of Met359Ile is present at position 72, glutamic acid of Lys362Glu is present at position 75, asparagine of Lys371Asn is present at position 84, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200 in SEQ ID NO: 62.

### (j) AVR15c

This protein is a protein for which Asn324His, Gln334Leu, Glu335Val, and Phe379Tyr were selected from among the amino acid substitutions involved in enhancing alkali stability revealed in Example 9, and the amino acid substitutions were aggregated in AVR11a (SEQ ID NO: 19). Specifically, AVR15c was prepared by introducing mutations resulting in amino acid substitutions of Asn324His, Gln334Leu, and Glu335Val into a polynucleotide (SEQ ID NO: 42) encoding AVR11a_F379Y (SEQ ID NO: 43).
(j-1) PCR was performed in the same manner as in (h-1), except that the above-described pET-AVR11a_F379Y was used as a template.
(j-2) A plasmid (expression vector) pET-AVR15c comprising a polynucleotide encoding AVR15c with 15 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR15c with a signal sequence and a polyhistidine tag is set forth in SEQ ID NO: 63. A PelB signal peptide ranges from methionine (Met) at position 1 to alanine (Ala) at position 22, an AAV-binding protein AVR15c (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 63. In addition, aspartic acid of Val317Asp is present at position 30, histidine of Asn324His is present at position 37, valine of Ala330Val is present at position 43, leucine of Gln334Leu is present at position 47, valine of Glu335Val is present at position 48, serine of Tyr342Ser is present at position 55, glutamic acid of Lys362Glu is present at position 75, asparagine of Lys371Asn is present at position 84, tyrosine of Phe379Tyr is present at position 92, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200 in SEQ ID NO: 63.

### (k) AVR16

This protein is a protein for which Asn324His, Gln334Leu, Glu335Val, Thr341Ala, and Phe379Tyr were selected from among the amino acid substitutions involved in enhancing alkali stability revealed in Example 9, and the amino acid substitutions were aggregated in AVR11a (SEQ ID NO: 19). Specifically, AVR16 was prepared by introducing mutations resulting in an amino acid substitution of Thr341Ala into a polynucleotide (SEQ ID NO: 64) encoding AVR15c (SEQ ID NO: 63).
(k-1) PCR was performed in the same manner as in Example 2 (2), except that a vector pET-AVR 15c capable of expressing a polypeptide (SEQ ID NO: 63) comprising AVR15c prepared in (j) was used as a template, and oligonucleotides consisting of the sequences set forth in SEQ ID NO: 52 (5'-TTTT[GGTCTC]ATTTCGGTGGTACCAGCAGCACATAAAC-3') and SEQ ID NO: 53 (5'-TTTT[GGTCTC]AGAAAGGGGAAGCCTCGACGTATG-3') (the square brackets in SEQ ID NOS: 52 and 53 indicate the restriction enzyme BsaI recognition sequence) were used as PCR primers (Forward: SEQ ID NO: 52; Reverse: SEQ ID NO: 53).
(k-2) A plasmid (expression vector) pET-AVR16 comprising a polynucleotide encoding AVR16 with 16 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR16 with a signal sequence and a polyhistidine tag is set forth in SEQ ID NO: 65. A PelB signal peptide ranges from methionine (Met) at position 1 to alanine (Ala) at position 22, an AAV-binding protein AVR16 (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 65. In addition, aspartic acid of Val317Asp is present at position 30, histidine of Asn324His is present at position 37, valine of Ala330Val is present at position 43, leucine of Gln334Leu is present at position 47, valine of Glu335Val is present at position 48, alanine of Thr341Ala is present at position 54, serine of Tyr342Ser is present at position 55, glutamic acid of Lys362Glu is present at position 75, asparagine of Lys371Asn is present at position 84, tyrosine of Phe379Tyr is present at position 92, alanine of Val381Ala is present at position 94, valine of Ile382Val is present at position 95, serine of Gly390Ser is present at position 103, glutamic acid of Lys399Glu is present at position 112, arginine of Ser476Arg is present at position 189, and aspartic acid of Asn487Asp is present at position 200 in SEQ ID NO: 65.

### Example 11: Evaluation of Alkali Stability of Aggregate of Amino Acid Substitutions in AVR11a

(1) Culture was performed in the same manner as in Example 4 (1) to (3), except that a transformant, which was obtained by transforming the E. coli BL21 (DE3) strain with a plasmid comprising a polynucleotide encoding any one of the 11 types of AAV-binding proteins (an aggregate of amino acid substitutions in AVR11a, specifically, AVR12a [SEQ ID NO: 54], AVR12b [SEQ ID NO: 55], AVR13 [SEQ ID NO: 56], AVR14a [SEQ ID NO: 57], AVR14b [SEQ ID NO: 58], AVR14c [SEQ ID NO: 59], AVR14d [SEQ ID NO: 60], AVR15a [SEQ ID NO: 61], AVR15b [SEQ ID NO: 62], AVR15c [SEQ ID NO: 63], and AVR16 [SEQ ID NO: 65]) obtained in Example 10 and AVR11a (SEQ ID NO: 19), was used as a transformant capable of expressing an AAV-binding protein.
(2) After the culture, bacterial cells were collected using the method described in Example 4 (4), and an AAV-binding protein was prepared from the collected bacterial cells using the method described in Examples 4 (5) to (7).
(3) The binding activity between the AAV-binding protein prepared in (2) and the VLP2 prepared in Example 8 was measured using the ELISA method described in Example 3 (4). Based on the absorbance at 450 nm as the measurement result, the AAV-binding protein obtained in (2) was diluted with pure water such that the measurement value would be the same.
(4) Each AAV-binding protein solution diluted in (3) was divided into two fractions. One of the fractions was mixed with an equal amount of a 0.1 M aqueous sodium hydroxide solution and left standing at a constant temperature for a definite period for alkali treatment (treatment temperature: 30°C; treatment time: 3 hours). The other fraction was not subjected to the alkali treatment (corresponding to the "starting time" of the alkali treatment).
(5) The fraction after the treatment in (4) was mixed with 0.5M MES buffer (pH 6.0) at a ratio of 7:3 to adjust the pH to around 6. The binding activity with VLP2 was then measured by the ELISA method described in Example 3 (4).
(6) The residual activity was calculated by dividing the absorbance at 450 nm when the alkali treatment in (4) was performed by the absorbance at 450 nm when the treatment time was 0 hours.

Table 10 shows the results. It is understood that each aggregate of amino acid substitutions in AVR11a obtained in Example 10 has higher residual activity and enhanced alkali stability compared with AVR11a (SEQ ID NO: 19).

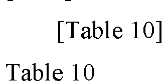

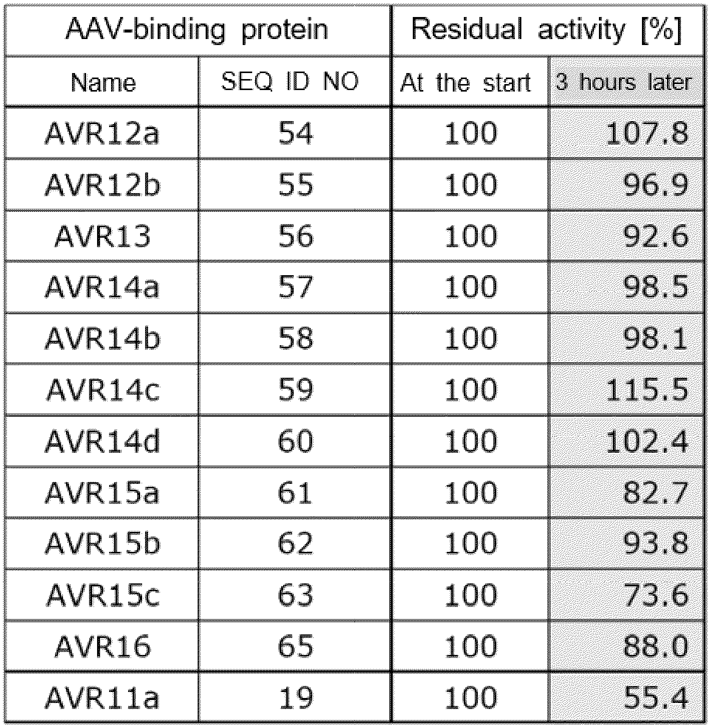

### Example 12: Creation and Screening of Mutation Library of AAV-Binding Protein (Part 2)

Among the AAV-binding proteins evaluated in Example 11, AVR16 (SEQ ID NO: 65) was selected, and a mutation was randomly introduced into the polynucleotide (SEQ ID NO: 66) portion encoding the protein using error-prone PCR.
(1) Error-prone PCR was performed in the same manner as in Example 9 (1), except that a vector pET-AVR16 capable of expressing a polypeptide (SEQ ID NO: 65) comprising AVR16 prepared in Example 10(k) was used as a template. Mutations were successfully introduced into the polynucleotide encoding the AAV-binding protein by the error-prone PCR, and the average mutation introduction rate was 0.8 amino acid mutations per molecule.
(2) A random mutant library was created from the PCR product obtained in (1) by the method described in Examples 9 (2) to (3).
(3) The random mutant library (transformants) prepared in (2) was inoculated into 200 µL of a 2YT liquid medium containing 50 µg/mL kanamycin and subjected to shaking culture at 37°C overnight using a 96-well deep well plate.
(4) The culture solution in (3) was centrifuged, and the resulting culture supernatant was diluted 16 times with ultrapure water. The diluted culture solution in an amount of 60 µL was mixed with 60 µL of a 0.1 M sodium hydroxide aqueous solution and alkali-treated at 42.1°C for 15 minutes.
(5) The binding activity between the AAV-binding protein and VLP2 when the treatment in (4) was performed and the binding activity between the AAV-binding protein and VLP2 when the treatment in (4) was not performed were measured using the ELISA method described in Example 3 (4). The residual activity was calculated by dividing the binding activity between the AAV-binding protein and VLP2 when heat treatment was performed by the binding activity between the AAV-binding protein and VLP2 when heat treatment was not performed.
(6) A random mutant library of about 1800 strains was evaluated using the method described in (5). Transformants expressing an AAV-binding protein with enhanced residual activity compared with the parent molecule AVR16 were selected from the library. The selected transformants were cultured, and an expression vector was prepared using a QIAprep Spin Miniprep Kit (manufactured by QIAGEN).
(7) The nucleotide sequence of the polynucleotide region encoding the AAV-binding protein inserted into the obtained expression vector was analyzed by the method described in Example 3 (7), and the amino acid mutation site was identified.

Tables 11 and 12 show lists of the amino acid substitution sites and the residual activities (%) after alkali treatment of the AAV-binding proteins expressed by the transformants selected in (6) above to AVR11a.

It can be said that the AAV-binding proteins, in which at least any one of amino acid substitutions of Val(Asp)317Asn, Ile319Val, Lys323Asn, Glu325Lys, Val326Ala, Asn329Ser, Leu333Met, Lys338Arg, Gly339Glu, Met359Val, Gly361Glu, Lys(Glu)362Asp, Ser366Pro, Lys368Arg, Lys368Glu, Leu369Pro, Lys380Arg, Val(Ala)381Thr, Ile(Val)382Asp, Lys(Glu)399Gly, Leu421Pro, Pro422Ser, Ser425Gly, His443Tyr, His443Arg, Glu454Gly, Lys455Arg, Lys467Glu, Lys467Gln, Ser(Arg)476Gly, Ser482Thr, Ser488Phe, Ala491Ser, Asn492Asp, Asn496Tyr, and Asp500Gly is present at the amino acid residues from serine (Ser) at position 312 to aspartic acid (Asp) at position 500 in the amino acid sequence set forth in SEQ ID NO: 1, have enhanced stability to alkali even compared with AVR16 (SEQ ID NO: 65) with more enhanced alkali stability than AVR11a (SEQ ID NO: 19).

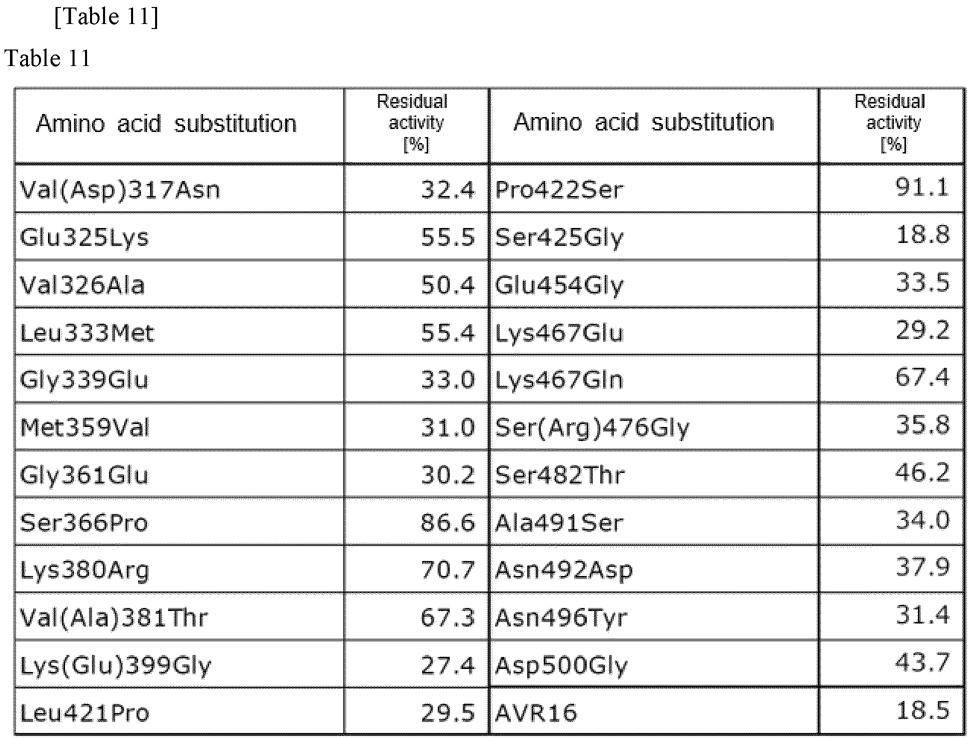

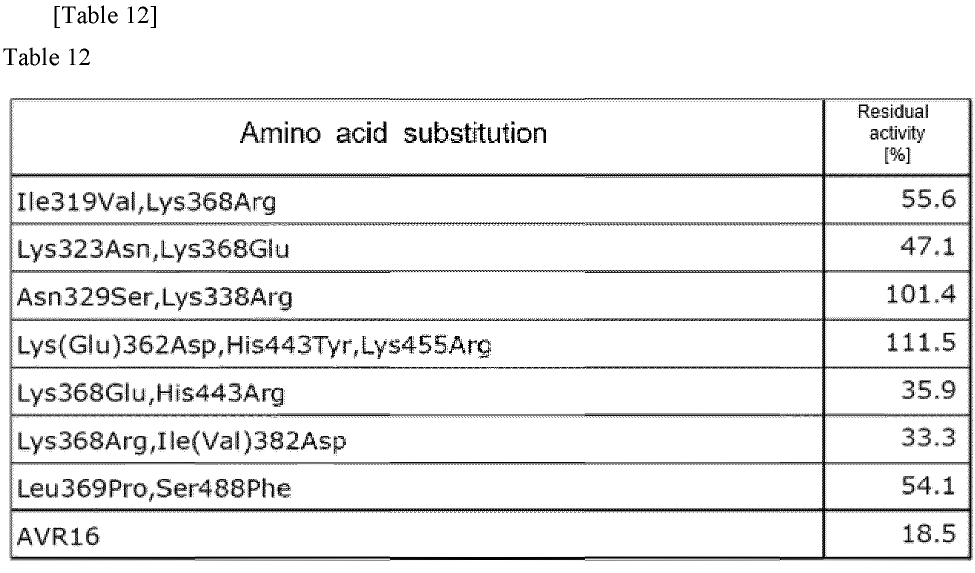

### Example 13: Preparation of Aggregate of Amino Acid Substitutions in AVR16

It was attempted to aggregate amino acid substitutions, which were found in Example 12 to be involved in enhancing the alkali stability of AAV-binding proteins, in AVR16 (SEQ ID NO: 65), thereby further enhancing the alkali stability. Specifically, seven types of AAV-binding proteins shown in (a) to (g) below were designed and prepared.
(a) Protein with the Val326Ala, Lys467Gln, and Ser482Thr amino acid substitutions introduced into AVR16 (SEQ ID NO: 83, named "AVR19a"),
(b) Protein with the Lys467Gln, Ser482Thr, and Asn492Asp amino acid substitutions introduced into AVR16 (SEQ ID NO: 85, named "AVR19b"),
(c) Protein with the Lys380Arg, Lys467Gln, and Ser482Thr amino acid substitutions introduced into AVR16 (SEQ ID NO: 87, named "AVR19c"),
(d) Protein with the Val326Ala, Lys467Gln, Ser482Thr, and Asn492Asp amino acid substitutions introduced into AVR16 (SEQ ID NO: 88, named "AVR20a"),
(e) Protein with the Val326Ala, Lys380Arg, Lys467Gln, and Ser482Thr amino acid substitutions introduced into AVR16 (SEQ ID NO: 90, named "AVR20b"),
(f) Protein with the Lys380Arg, Lys467Gln, Ser482Thr, and Asn492Asp amino acid substitutions introduced into AVR16 (SEQ ID NO: 91, named "AVR20c"), and
(g) Protein with the Val326Ala, Lys380Arg, Lys467Gln, Ser482Thr, and Asn492Asp amino acid substitutions introduced into AVR16 (SEQ ID NO: 92, named "AVR21").

Hereinafter, a method of preparing the eight types of AAV-binding proteins shown in (a) to (g) above will be explained.

### (a) AVR19a

This protein is a protein for which Val326Ala, Lys467Gln, and Ser482Thr were selected from among the amino acid substitutions involved in enhancing alkali stability revealed in Example 12, and the amino acid substitutions were aggregated in AVR16 (SEQ ID NO: 65). Specifically, AVR19c was prepared by introducing a mutation resulting in an amino acid substitution of Ser482Thr into a polynucleotide (SEQ ID NO: 69) encoding a protein (named "AVR16_K467Q," SEQ ID NO: 70) with an amino acid substitution of Lys467Gln in AVR16 obtained in Example 12 (named "AVR18"), followed by introducing a mutation resulting in an amino acid substitution of Val326Ala into a polynucleotide encoding the AVR18.
(a-1) PCR was performed in the same manner as in Example 2 (2), except that a vector pET-AVR16_K467Q capable of expressing a polypeptide (SEQ ID NO: 70) comprising AVR16_K467Q was used as a template, and oligonucleotides consisting of the sequences set forth in SEQ ID NO: 73 (5'-TTTT[GGTCTC]AGTGGTCGATACCGATGGCG-3') and SEQ ID NO: 74 (5'-TTTT[GGTCTC]ACCACCGTCAGTCTGAAC-3') (the square brackets in SEQ ID NOS: 73 and 74 indicate the restriction enzyme BsaI recognition sequence) were used as PCR primers (Forward: SEQ ID NO: 73; Reverse: SEQ ID NO: 74).
(a-2) After purifying the PCR products amplified by the method described in Example 2 (3) and (4), a ligation reaction was performed by the method described in Example 2 (5).
(a-3) The E. coli BL21 (DE3) strain was transformed using the obtained ligation product. After the resulting transformant was cultured in an LB medium supplemented with 50 µg/mL kanamycin, the plasmid (expression vector) was extracted from the bacterial cells (transformants) recovered by centrifugation, thereby obtaining a plasmid pET-AVR18 comprising a polynucleotide encoding AVR18 with 18 amino acid substitutions in the wild-type AAV-binding protein.
(a-4) The nucleotide sequence of pET-AVR18 was analyzed and confirmed in the same manner as in Example 3 (7). The amino acid sequence of AVR18 with a signal sequence and a polyhistidine tag is set forth in SEQ ID NO: 81.
(a-5) PCR was performed in the same manner as in Example 2 (2), except that pET-AVR18 obtained in (a-3) was used as a template, and oligonucleotides consisting of the sequences set forth in SEQ ID NO: 75 (5'-TTTT[GGTCTC]ACCGAAGCACGAAGCACAACTG-3') and SEQ ID NO: 76 (5'-TTTT[GGTCTC]ATCGGCAGGGTAATCTGATC-3') (the square brackets in SEQ ID NOS: 75 and 76 indicate the restriction enzyme BsaI recognition sequence) were used as PCR primers (Forward: SEQ ID NO: 75; Reverse: SEQ ID NO: 76).
(a-6) A plasmid (expression vector) pET-AVR19a comprising a polynucleotide encoding AVR19a with 19 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR19a with a signal sequence and a polyhistidine tag is set forth in SEQ ID NO: 83. A PelB signal peptide ranges from methionine (Met) at position 1 to alanine (Ala) at position 22, an AAV-binding protein AVR19a (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 83. In addition, aspartic acid of Val317Asp is present at position 30, histidine of Asn324His is present at position 37, alanine of Val326Ala is present at position 39, valine of Ala330Val is present at position 43, leucine of Gln334Leu is present at position 47, valine of Glu335Val is present at position 48, alanine of Thr341Ala is present at position 54, serine of Tyr342Ser is present at position 55, glutamic acid of Lys362Glu is present at position 75, asparagine of Lys371Asn is present at position 84, tyrosine of Phe379Tyr is present at position 92, alanine of Val381Ala is present at position 94, valine of Ile382Val is present at position 95, serine of Gly390Ser is present at position 103, glutamic acid of Lys399Glu is present at position 112, glutamine of Lys467Gln is present at position 180, arginine of Ser476Arg is present at position 189, threonine of Ser482Thr is present at position 195, and aspartic acid of Asn487Asp is present at position 200 in SEQ ID NO: 83.

### (b) AVR19b

This protein is a protein for which Lys467Gln, Ser482Thr, and Asn492Asp were selected from among the amino acid substitutions involved in enhancing alkali stability revealed in Example 12, and the amino acid substitutions were aggregated in AVR16 (SEQ ID NO: 65). Specifically, AVR19b was prepared by introducing mutations resulting in an amino acid substitution of Asn492Asp into a polynucleotide (SEQ ID NO: 82) encoding AVR18 (SEQ ID NO: 81).
(b-1) PCR was performed in the same manner as in Example 2 (2), except that pET-AVR18 obtained in (a-3) was used as a template, and oligonucleotides consisting of the sequences set forth in SEQ ID NO: 77 (5'-TTTT[GGTCTC]ACACCGCCGATCTTACCGTC-3') and SEQ ID NO: 78 (5'-TTTT[GGTCTC]AGGTGGTGGAGTCGGTTGC-3') (the square brackets in SEQ ID NOS: 77 and 78 indicate the restriction enzyme BsaI recognition sequence) were used as PCR primers (Forward: SEQ ID NO: 77; Reverse: SEQ ID NO: 78).
(b-2) A plasmid (expression vector) pET-AVR19b comprising a polynucleotide encoding AVR19b with 19 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR19b with a signal sequence and a polyhistidine tag is set forth in SEQ ID NO: 85. A PelB signal peptide ranges from methionine (Met) at position 1 to alanine (Ala) at position 22, an AAV-binding protein AVR19b (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 85. In addition, aspartic acid of Val317Asp is present at position 30, histidine of Asn324His is present at position 37, valine of Ala330Val is present at position 43, leucine of Gln334Leu is present at position 47, valine of Glu335Val is present at position 48, alanine of Thr341Ala is present at position 54, serine of Tyr342Ser is present at position 55, glutamic acid of Lys362Glu is present at position 75, asparagine of Lys371Asn is present at position 84, tyrosine of Phe379Tyr is present at position 92, alanine of Val381Ala is present at position 94, valine of Ile382Val is present at position 95, serine of Gly390Ser is present at position 103, glutamic acid of Lys399Glu is present at position 112, glutamine of Lys467Gln is present at position 180, arginine of Ser476Arg is present at position 189, threonine of Ser482Thr is present at position 195, aspartic acid of Asn487Asp is present at position 200, and aspartic acid of Asn492Asp is present at position 205 in SEQ ID NO: 85.

### (c) AVR19c

This protein is a protein for which Lys380Arg, Lys467Gln, and Ser482Thr were selected from among the amino acid substitutions involved in enhancing alkali stability revealed in Example 12, and the amino acid substitutions were aggregated in AVR16 (SEQ ID NO: 65). Specifically, AVR19c was prepared by introducing mutations resulting in an amino acid substitution of Lys380Arg into a polynucleotide (SEQ ID NO: 82) encoding AVR18 (SEQ ID NO: 81).
(c-1) PCR was performed in the same manner as in Example 2 (2), except that pET-AVR18 obtained in (a-3) was used as a template, and oligonucleotides consisting of the sequences set forth in SEQ ID NO: 79 (5'-TTTT[GGTCTC]ATATGAATACCGTGCTGTCGTTG-3') and SEQ ID NO: 80 (5'-TTTT[GGTCTC]ACATACAGACCCGGCGTC-3') (the square brackets in SEQ ID NOS: 79 and 80 indicate the restriction enzyme BsaI recognition sequence) were used as PCR primers (Forward: SEQ ID NO: 79; Reverse: SEQ ID NO: 80).
(c-2) A plasmid (expression vector) pET-AVR19c comprising a polynucleotide encoding AVR19c with 19 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR19c with a signal sequence and a polyhistidine tag is set forth in SEQ ID NO: 87. A PelB signal peptide ranges from methionine (Met) at position 1 to alanine (Ala) at position 22, an AAV-binding protein AVR19c (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 87. In addition, aspartic acid of Val317Asp is present at position 30, histidine of Asn324His is present at position 37, valine of Ala330Val is present at position 43, leucine of Gln334Leu is present at position 47, valine of Glu335Val is present at position 48, alanine of Thr341Ala is present at position 54, serine of Tyr342Ser is present at position 55, glutamic acid of Lys362Glu is present at position 75, asparagine of Lys371Asn is present at position 84, tyrosine of Phe379Tyr is present at position 92, arginine of Lys380Arg is present at position 93, alanine of Val381Ala is present at position 94, valine of Ile382Val is present at position 95, serine of Gly390Ser is present at position 103, glutamic acid of Lys399Glu is present at position 112, glutamine of Lys467Gln is present at position 180, arginine of Ser476Arg is present at position 189, threonine of Ser482Thr is present at position 195, and aspartic acid of Asn487Asp is present at position 200 in SEQ ID NO: 87.

### (d) AVR20a

This protein is a protein for which Val326Ala, Lys467Gln, Ser482Thr, and Asn492Asp were selected from among the amino acid substitutions involved in enhancing alkali stability revealed in Example 12, and the amino acid substitutions were aggregated in AVR16 (SEQ ID NO: 65). Specifically, AVR20a was prepared by introducing mutations resulting in an amino acid substitution of Asn492Asp into a polynucleotide (SEQ ID NO: 84) encoding AVR19a (SEQ ID NO: 83).
(d-1) PCR was performed in the same manner as in (b-1), except that the vector pET-AVR19a capable of expressing the polypeptide (SEQ ID NO: 83) comprising A VR19a prepared in (a) was used as a template.
(d-2) A plasmid (expression vector) pET-AVR20a comprising a polynucleotide encoding AVR20a with 20 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR20a with a signal sequence and a polyhistidine tag is set forth in SEQ ID NO: 88. A PelB signal peptide ranges from methionine (Met) at position 1 to alanine (Ala) at position 22, an AAV-binding protein AVR20a (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 88. In addition, aspartic acid of Val317Asp is present at position 30, histidine of Asn324His is present at position 37, alanine of Val326Ala is present at position 39, valine of Ala330Val is present at position 43, leucine of Gln334Leu is present at position 47, valine of Glu335Val is present at position 48, alanine of Thr341Ala is present at position 54, serine of Tyr342Ser is present at position 55, glutamic acid of Lys362Glu is present at position 75, asparagine of Lys371Asn is present at position 84, tyrosine of Phe379Tyr is present at position 92, alanine of Val381Ala is present at position 94, valine of Ile382Val is present at position 95, serine of Gly390Ser is present at position 103, glutamic acid of Lys399Glu is present at position 112, glutamine of Lys467Gln is present at position 180, arginine of Ser476Arg is present at position 189, threonine of Ser482Thr is present at position 195, aspartic acid of Asn487Asp is present at position 200, and aspartic acid of Asn492Asp is present at position 205 in SEQ ID NO: 88.

### (e) AVR20b

This protein is a protein for which Val326Ala, Lys380Arg, Lys467Gln, and Ser482Thr were selected from among the amino acid substitutions involved in enhancing alkali stability revealed in Example 12, and the amino acid substitutions were aggregated in AVR16 (SEQ ID NO: 65). Specifically, AVR20b was prepared by introducing mutations resulting in an amino acid substitution of Lys380Arg into a polynucleotide (SEQ ID NO: 84) encoding AVR19a (SEQ ID NO: 83).
(e-1) PCR was performed in the same manner as in (c-1), except that the above-described pET-AVR19a prepared in (a) was used as a template.
(e-2) A plasmid (expression vector) pET-AVR20b comprising a polynucleotide encoding AVR20b with 20 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR20b with a signal sequence and a polyhistidine tag is set forth in SEQ ID NO: 90. A PelB signal peptide ranges from methionine (Met) at position 1 to alanine (Ala) at position 22, an AAV-binding protein AVR20b (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 90. In addition, aspartic acid of Val317Asp is present at position 30, histidine of Asn324His is present at position 37, alanine of Val326Ala is present at position 39, valine of Ala330Val is present at position 43, leucine of Gln334Leu is present at position 47, valine of Glu335Val is present at position 48, alanine of Thr341Ala is present at position 54, serine of Tyr342Ser is present at position 55, glutamic acid of Lys362Glu is present at position 75, asparagine of Lys371Asn is present at position 84, tyrosine of Phe379Tyr is present at position 92, arginine of Lys380Arg is present at position 93, alanine of Val381Ala is present at position 94, valine of Ile382Val is present at position 95, serine of Gly390Ser is present at position 103, glutamic acid of Lys399Glu is present at position 112, glutamine of Lys467Gln is present at position 180, arginine of Ser476Arg is present at position 189, threonine of Ser482Thr is present at position 195, and aspartic acid of Asn487Asp is present at position 200 in SEQ ID NO: 90.

### (f) AVR20c

This protein is a protein for which Lys380Arg, Lys467Gln, Ser482Thr, and Asn492Asp were selected from among the amino acid substitutions involved in enhancing alkali stability revealed in Example 12, and the amino acid substitutions were aggregated in AVR16 (SEQ ID NO: 65). Specifically, AVR20c was prepared by introducing mutations resulting in an amino acid substitution of Lys380Arg into a polynucleotide (SEQ ID NO: 86) encoding AVR19b (SEQ ID NO: 85).
(f-1) PCR was performed in the same manner as in (c-1), except that the vector pET-AVR19b capable of expressing the polypeptide (SEQ ID NO: 85) comprising AVR19b prepared in (b) was used as a template.
(f-2) A plasmid (expression vector) pET-AVR20c containing a polynucleotide encoding AVR20c with 20 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The amino acid sequence of AVR20c with a signal sequence and a polyhistidine tag is set forth in SEQ ID NO: 91. A PelB signal peptide ranges from methionine (Met) at position 1 to alanine (Ala) at position 22, an AAV-binding protein AVR20c (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 91. In addition, aspartic acid of Val317Asp is present at position 30, histidine of Asn324His is present at position 37, valine of Ala330Val is present at position 43, leucine of Gln334Leu is present at position 47, valine of Glu335Val is present at position 48, alanine of Thr341Ala is present at position 54, serine of Tyr342Ser is present at position 55, glutamic acid of Lys362Glu is present at position 75, asparagine of Lys371Asn is present at position 84, tyrosine of Phe379Tyr is present at position 92, arginine of Lys380Arg is present at position 93, alanine of Val381Ala is present at position 94, valine of Ile382Val is present at position 95, serine of Gly390Ser is present at position 103, glutamic acid of Lys399Glu is present at position 112, glutamine of Lys467Gln is present at position 180, arginine of Ser476Arg is present at position 189, threonine of Ser482Thr is present at position 195, aspartic acid of Asn487Asp is present at position 200, and aspartic acid of Asn492Asp is present at position 205 in SEQ ID NO: 91.

### (g) AVR21

This protein is a protein for which Val326Ala, Lys380Arg, Lys467Gln, Ser482Thr, and Asn492Asp were selected from among the amino acid substitutions involved in enhancing alkali stability revealed in Example 12, and the amino acid substitutions were aggregated in AVR16 (SEQ ID NO: 65). Specifically, AVR21 was prepared by introducing mutations resulting in an amino acid substitution of Lys380Arg into a polynucleotide (SEQ ID NO: 89) encoding AVR20a (SEQ ID NO: 88).
(g-1) PCR was performed in the same manner as in (c-1), except that the vector pET-AVR20a capable of expressing the polypeptide (SEQ ID NO: 88) containing AVR20a prepared in (d) was used as a template.
(g-2) A plasmid (expression vector) pET-AVR21 containing a polynucleotide encoding AVR21 with 21 amino acid substitutions in the wild-type AAV-binding protein was obtained from the amplified PCR product using the same method as in (a-2) to (a-3). The nucleotide sequence of the plasmid was then analyzed and confirmed using the same method as in Example 3 (7).

The sequence of the polynucleotide encoding a polypeptide containing AVR21 analyzed in (g-2) is set forth in SEQ ID NO: 93. The amino acid sequence of AVR21 with a signal sequence and a polyhistidine tag is set forth in SEQ ID NO: 92. A PelB signal peptide ranges from methionine (Met) at position 1 to alanine (Ala) at position 22, an AAV-binding protein AVR21 (corresponding to a region from position 312 to position 500 in SEQ ID NO: 1) ranges from serine (Ser) at position 25 to aspartic acid (Asp) at position 213, and a tag sequence ranges from histidine (His) at position 214 to histidine (His) at position 219 in SEQ ID NO: 92. In addition, aspartic acid of Val317Asp is present at position 30, histidine of Asn324His is present at position 37, alanine of Val326Ala is present at position 39, valine of Ala330Val is present at position 43, leucine of Gln334Leu is present at position 47, valine of Glu335Val is present at position 48, alanine of Thr341Ala is present at position 54, serine of Tyr342Ser is present at position 55, glutamic acid of Lys362Glu is present at position 75, asparagine of Lys371Asn is present at position 84, tyrosine of Phe379Tyr is present at position 92, arginine of Lys380Arg is present at position 93, alanine of Val381Ala is present at position 94, valine of Ile382Val is present at position 95, serine of Gly390Ser is present at position 103, glutamic acid of Lys399Glu is present at position 112, glutamine of Lys467Gln is present at position 180, arginine of Ser476Arg is present at position 189, threonine of Ser482Thr is present at position 195, aspartic acid of Asn487Asp is present at position 200, and aspartic acid of Asn492Asp is present at position 205 in SEQ ID NO: 92.

### Example 14: Evaluation of Alkali Stability of Aggregate of Amino Acid Substitutions in AVR16

(1) Culture was performed in the same manner as in Example 4 (1) to (3), except that a transformant, which was obtained by transforming the E. coli BL21 (DE3) strain with a plasmid containing a polynucleotide encoding any one of AVR16 [SEQ ID NO: 65] and aggregates of amino acid substitutions in AVR16, i.e., five types of AAV-binding proteins (AVR16_K467Q [SEQ ID NO: 70] and proteins each comprising AVR16 [SEQ ID NO: 65] with an amino acid substitution of Val326Ala [named "AVR16_V326A," SEQ ID NO: 67], Lys380Arg [named "AVR16_K380R," SEQ ID NO: 68], Ser482Thr [named "AVR16_S482T," SEQ ID NO: 71], or Asn492Asp [named "AVR16_N492D," SEQ ID NO: 72]) obtained in Example 12 and seven types of AAV-binding proteins (AVR19a [SEQ ID NO: 83], AVR19b [SEQ ID NO: 85], AVR19c [SEQ ID NO: 87], AVR20a [SEQ ID NO: 89], AVR20b [SEQ ID NO: 90], AVR20c [SEQ ID NO: 91], and AVR21 [SEQ ID NO: 92] obtained in Example 13), was used as a transformant capable of expressing an AAV-binding protein.
(2) After the culture, bacterial cells were collected using the method described in Example 4 (4), and an AAV-binding protein was prepared from the collected bacterial cells using the method described in Examples 4 (5) to (7).
(3) The binding activity between the AAV-binding protein prepared in (2) and the VLP2 prepared in Example 8 was measured using the ELISA method described in Example 3 (4). Based on the absorbance at 450 nm as the measurement result, the AAV-binding protein obtained in (2) was diluted with pure water such that the measurement value would be the same.
(4) Each AAV-binding protein solution diluted in (3) was divided into two fractions. One of the fractions was mixed with an equal amount of a 0.5 M aqueous sodium hydroxide solution and left standing at 30°C for 1 hour for alkali treatment. The other fraction was not subjected to the alkali treatment (corresponding to the "starting time" of the alkali treatment).
(5) The fraction after the treatment in (4) was mixed with 0.5M MES buffer (pH 6.0) at a ratio of 8:2 to adjust the pH to around 6. The binding activity with VLP2 was then measured by the ELISA method described in Example 3 (4).
(6) The residual activity was calculated by dividing the absorbance at 450 nm when the alkali treatment in (4) was performed by the absorbance at 450 nm when the treatment time was 0 hours.

Table 13 shows the results. It is understood that each aggregate of amino acid substitutions in AVR16 obtained in this Example has higher residual activity and enhanced alkali stability even compared with AVR16 (SEQ ID NO: 65) with more enhanced alkali stability than AVR11a (SEQ ID NO: 19).

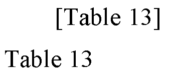

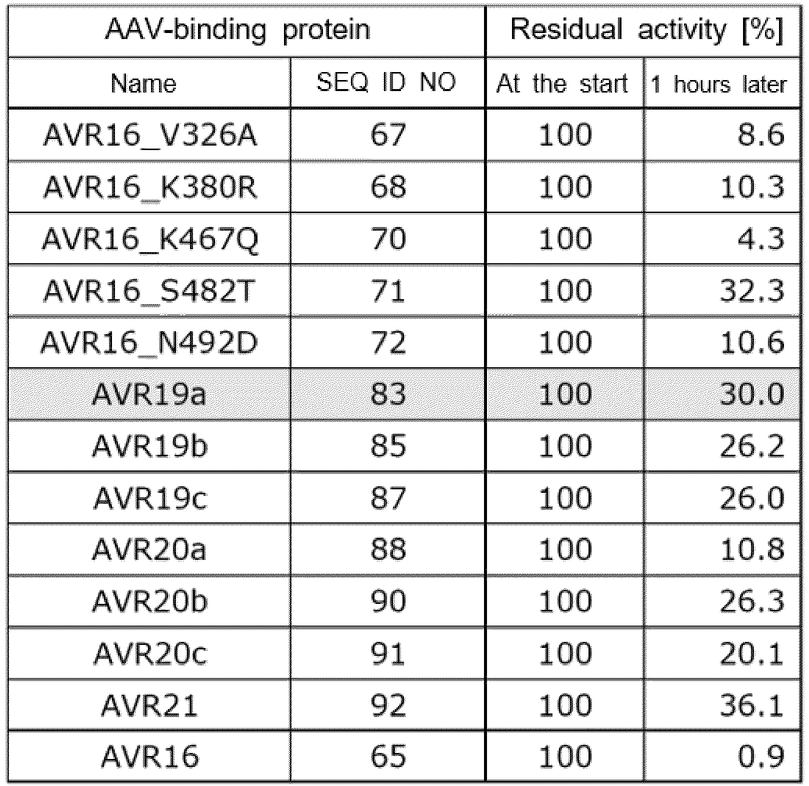

### Example 15: Preparation of Adeno-Associated Virus (AAV) Vector

(1) A nucleotide sequence (SEQ ID NO: 96) was designed, in which a restriction enzyme EcoRI recognition sequence (GAATTC) was added to the 5' end of a polynucleotide encoding an enhanced green fluorescent protein (EGFP) consisting of the amino acid sequence set forth in SEQ ID NO: 95 and a stop codon (TAG) and a BamHI recognition sequence (GGATCC) were added to the 3' end of the same.
(2) A polynucleotide consisting of the sequence set forth in SEQ ID NO: 96 was totally synthesized and cloned into a plasmid (consigned to Fasmac Co., Ltd., named "pUC-EGFP"). The E. coli JM109 strain was transformed with pUC-EGFP, and the resulting transformant was cultured. pUC-EGFP was extracted from the culture solution using a QIAprep Spin Miniprep Kit (manufactured by QIAGEN).
(3) pUC-EGFP obtained in (2) was digested with restriction enzymes EcoRI and BamHI and then ligated to an expression vector pAAV-CMV (manufactured by Takara Bio Inc.), which had previously been digested with restriction enzymes EcoRI and BamHI. The E. coli JM109 strain was transformed using the ligation product.
(4) The transformant obtained in (3) was subjected to shaking culture overnight at 37°C in a 5-L baffled flask containing 1L of a 2YT medium (1.6% (w/v) tryptone, 1% (w/v) yeast extract, 0.5% (w/v) sodium chloride) containing 100 µg/mL carbenicillin. After the culture was completed, the cells were collected by centrifugation, and a vector pAAV-EGFP expressing EGFP was prepared in a large quantity from the collected cells using a Plasmid Mega Kit (manufactured by QIAGEN).
(5) The E. coli JM109 strain was transformed with a pHelper Vector (manufactured by Takara Bio Inc.) and a plasmid containing a polynucleotide encoding any one of a capsid region VP1 of serotype 2 AAV (AAV2) (UniProt Accession No.: P03135, SEQ ID NO: 99) and amino acid substitution products of the VP1 shown in the following (a) to (m) (hereinafter, also collectively referred to as simply"AAV2") (hereinafter, also collectively referred to as "pRC2 Vector"). The pRC2 Vector and pHelper were prepared in large quantities by performing the same operation as in (4) using the obtained transformant:
   (a) glutamine at position 263 of SEQ ID NO: 99 is substituted with alanine (hereinafter also referred to as "AAV2 Q263A");
   (b) serine at position 264 of SEQ ID NO: 99 is substituted with alanine (hereinafter also referred to as "AAV2 S264A");
   (c) serine at position 267 of SEQ ID NO: 99 is substituted with alanine or threonine (hereinafter also referred to as "AAV2 S267A" and "AAV2 S267T," respectively);
   (d) histidine at position 271 in SEQ ID NO: 99 is substituted with any one of alanine, phenylalanine, glutamine, and threonine (hereinafter also referred to as "AAV2 H271A," "AAV2 H271F," "AAV2 H271Q," and "AAV2 H271T," respectively);
   (e) asparagine at position 382 of SEQ ID NO: 99 is substituted with alanine (hereinafter also referred to as "AAV2 N382A");
   (f) glycine at position 383 of SEQ ID NO: 99 is substituted with alanine (hereinafter also referred to as "AAV2 G383A");
   (g) serine at position 384 of SEQ ID NO: 99 is substituted with alanine (hereinafter also referred to as "AAV2 S384A");
   (h) glutamine at position 385 of SEQ ID NO: 99 is substituted with alanine (hereinafter also referred to as "AAV2 Q385A");
   (i) arginine at position 471 of SEQ ID NO: 99 is substituted with alanine (hereinafter also referred to as "AAV2 R471A");
   (j) glutamic acid at position 499 of SEQ ID NO: 99 is substituted with alanine (hereinafter also referred to as "AAV2 E499A");
   (k) threonine at position 503 of SEQ ID NO: 99 is substituted with serine (hereinafter also referred to as "AAV2 T503 S");
   (l) aspartic acid at position 528 of SEQ ID NO: 99 is substituted with alanine (hereinafter also referred to as "AAV2 D528A"); and
   (m) aspartic acid at position 529 of SEQ ID NO: 99 is substituted with alanine or glutamic acid (hereinafter also referred to as "AAV2 D529A" and "AAV2 D529E," respectively).

The infectivity of the amino acid substitution products shown in (a) to (m) above relative to the wild type (SEQ ID NO: 99) is summarized in Table 14 (Source: Lochrie, M.A., et al., Journal of Virology, 80(2), 821).

### [Table 14]

**Table 14**

| AAV | Rate of infectivity to AAV2 (wild type) [%] |
|---|---|
| AAV2(wild type) | 100 |
| AAV2 Q263A | 39 |
| AAV2 5264A | 72 |
| AAV2 S267A | 378 |
| AAV2 S267T | 864 |
| AAV2 H271A | 10 |
| AAV2 H271F | 0.02 |
| AAV2 H271Q | 2 |
| AAV2 H271T | 0.06 |
| AAV2 N382A | 3 |
| AAV2 G383A | 0.1 |
| AAV2 S384A | 25 |
| AAV2 Q385A | 14 |
| AAV2 R471A | 318 |
| AAV2 E499A | 40 |
| AAV2 T503S | 26 |
| AAV2 D528A | 2 |
| AAV2 D529A | 0.002 |
| AAV2 D529E | 20 |

(6) Viral Production Cells (manufactured by Thermo Fisher Scientific) were subjected to shaking culture at 120 rpm in 8% (v/v) carbon dioxide and 37°C until reaching 3.0 × 10⁶ cells/mL in a 250-mL Erlenmeyer flask (manufactured by Corning) containing 60 mL of Viral Production Medium (manufactured by Thermo Fisher Scientific).

(7) Gene introduction was carried out by adding a mixture of AAV-MAX Enhancer (manufactured by Thermo Fisher Scientific), pAAV-EGFP prepared in (4), pRC2 Vector and pHelper prepared in (5), and AAV-MAX Transfection Reagent, Viral-Plex Complexation, and AAV-MAX Transfection Booster (all manufactured by Thermo Fisher Scientific) to the culture solution obtained in (6), followed by shaking culture at 120 rpm for 3 days in 8% (v/v) carbon dioxide and 37°C.

(8) To the culture solution obtained in (7), AAV-MAX Lysis Buffer (manufactured by Thermo Fisher Scientific) was added in a 1/10 amount of the medium, Benzonase (manufactured by Merck Millipore) was added at a final concentration of 1U/mL, and magnesium chloride was added at a final concentration of 2 mM, followed by shaking at 120 rpm at 37°C for 2.0 hours for bacteriolytic treatment.

(9) The solution after the bacteriolytic treatment was centrifuged at 8,000 × g and 4°C for 10 minutes, and the supernatant was passed through a filter with a pore size of 0.22 µm to remove suspended matter, thereby obtaining a solution containing AAV2.

(10) The concentration of AAV2 in the solution obtained in (9) was quantified using an AAV2 Titration ELISA Kit (manufactured by PROGEN).

As a result, all solutions contained AAV2 at 8.2 × 10⁹ cp/mL (cp indicates the number of AAV particles) or more. A solution that is sufficient to confirm the peak when detected using HPLC M40A (manufactured by Shimadzu Corporation) with a fluorescence intensity of 350 nm against excitation light of 280 nm could be obtained.

### Example 16: Preparation of AAV-Binding Protein (Part 1)

(1) A transformant capable of expressing A VR10s, which was obtained by transforming the E. coli BL21 (DE3) strain with a plasmid pET-AVR10s containing a polynucleotide (SEQ ID NO: 98) encoding a polypeptide comprising the AAV-binding protein A VR10s consisting of the amino acid sequence set forth in SEQ ID NO: 97, was inoculated into 3 mL of a 2YT liquid medium containing 50 µg/mL of kanamycin and precultured by aerobic shaking culture at 37°C overnight. A PelB signal peptide ranges from methionine (Met) at position 1 to alanine (Ala) at position 22, an AAV-binding protein AVR10s ranges from serine (Ser) at position 25 to aspartic acid (Asp) at position 213, and a cysteine tag sequence serving as an immobilization tag ranges from cysteine (Cys) at position 220 to glycine (Gly) at position 226 in SEQ ID NO: 97. AVR10s is a polypeptide with the following amino acid substitutions <I> to <X> in the extracellular domain 1 (PKD1) and the domain 2 (PKD2) (amino acid residue from serine at position 312 to aspartic acid at position 500 of SEQ ID NO: 94) of AAV receptor KIAA0319L:
   <I> a substitution of valine with aspartic acid at position 317 of SEQ ID NO: 94 (position 30 of SEQ ID NO: 97),
   <II> a substitution of tyrosine with serine at position 342 of SEQ ID NO: 94 (position 55 of SEQ ID NO: 97),
   <III> a substitution of lysine with glutamic acid at position 362 of SEQ ID NO: 94 (position 75 of SEQ ID NO: 97),
   <IV> a substitution of lysine with asparagine at position 371 of SEQ ID NO: 94 (position 84 of SEQ ID NO: 97),
   <V> a substitution of valine with alanine at position 381 of SEQ ID NO: 94 (position 94 of SEQ ID NO: 97),
   <VI> a substitution of isoleucine with valine at position 382 of SEQ ID NO: 94 (position 95 of SEQ ID NO: 97),
   <VII> a substitution of glycine with serine at position 390 of SEQ ID NO: 94 (position 103 of SEQ ID NO: 97),
   <VIII> a substitution of lysine with glutamic acid at position 399 of SEQ ID NO: 94 (position 112 of SEQ ID NO: 97),
   <IX> a substitution of serine with arginine at position 476 of SEQ ID NO: 94 (position 189 of SEQ ID NO: 97), and
   <X> a substitution of asparagine with aspartic acid at position 487 of SEQ ID NO: 94 (position 200 of SEQ ID NO: 97).
(2) The preculture solution from (1) was inoculated at 2 mL into 200 mL of a 2YT liquid medium supplemented with 50 µg/mL kanamycin in a 1-L baffled flask and subjected to aerobic shaking culture at 37°C.
(3) After 2.0 hours from the start of the culture, the flask was cooled on ice, isopropyl-β-d-thiogalactopyranoside (IPTG) was added to a final concentration of 0.1 mM, and then the aerobic shaking culture was continued at 25°C overnight.
(4) After the culture was completed, bacterial cells were collected by centrifuging the culture solution at 4°C and 8000 rpm for 20 minutes.
(5) The bacterial cells collected in (4) were suspended in 20 mM Tris-HCl buffer (pH 7.4) containing 150 mM sodium chloride and 20 mM imidazole to 5 mL/1 g (bacterial cells). following which the bacterial cells were disrupted at 8°C for about 10 minutes with an output of about 150 W using an ultrasonic generator (Insonator 201M [manufactured by KUBOTA CORPORATION]). The cell disruption solutions were centrifuged twice at 8000 rpm for 20 minutes at 4°C, and the supernatant was collected.
(6) The supernatant obtained in (5) was applied to an XK26/20 column (manufactured by Cytiva) packed with 50 mL of Ni Sepharose 6 Fast Flow (manufactured by Cytiva), which had previously been equilibrated with tris-HCl buffer (pH 7.4) containing 150 mM sodium chloride and 20mM imidazole (hereinafter also referred to as "equilibration solution B"). After washing with the equilibration solution B, elution was performed with 20 mM Tris-HCl buffer (pH 7.4) containing 0.5 M imidazole and 150 mM sodium chloride.
(7) The eluate obtained in (6) was dialyzed against 20 mM tris buffer (pH 7.4) containing 150 mM sodium chloride, thereby preparing the AVR10s protein in an amount required for AAV adsorbent production.

### Example 17: Preparation of AAV Adsorbent (Part 1)

(1) A hydrophilic vinyl polymer for separation agent (TOYOPEARL , manufactured by Tosoh Corporation) was prepared as an insoluble carrier, and a carrier with iodoacetamide groups introduced was prepared by chemically modifying the hydroxy groups on the surface of the polymer.
(2) To 7 g (wet weight) of the carrier prepared in (1), 14 mg of the AAV-binding protein AVR10s prepared in Example 16, and tris(2-carboxyethyl) phosphine (TCEP) at a final concentration of 0.3 mM as a reducing agent were added. The reaction was carried out by shaking at pH 8.1 and 25°C for 3 hours. Thus, an AVR10s immobilization gel serving as an AAV adsorbent with AVRlOs immobilized thereon was prepared.

### Example 18: AAV Analysis using AAV Adsorbent (Part 1)

(1) Stainless steel columns (ϕ4.6 mm × 75 mm, manufactured by Tosoh Corporation) were each packed with 1.25 mL of the AAV adsorbent AVR10s immobilization gel prepared in Example 17, thereby preparing columns (named "AVR10s columns").
(2) The AVRlOs column was connected to HPLC M40A (manufactured by Shimadzu Corporation) and equilibrated with 10 mM glycine buffer (pH 4.5) containing 50 mM calcium chloride and 15 mM sodium acetate (hereinafter also referred to as "equilibration solution C").
(3) To the AVR10s columns equilibrated in (2), 0.01 mL of the solutions containing AAV2 obtained in Example 15 were applied.
(4) After washing for 30 minutes with the equilibration solution C at a flow rate of 0.5 mL/min, 10 mM glycine buffer (pH 2.2) containing 50 mM calcium chloride and 15 mM sodium acetate (hereinafter also referred to as "eluate A") and the equilibration solution C were fed to the AVR10s columns (flow rate: 0.5 mL/min), thereby creating a linear gradient in which the concentration of eluate A ranged from 0% to 100% in 60 minutes. After completing the linear gradient, the eluate A was fed for 5 minutes, and then equilibration solution C was fed again to the AVR10s columns for re-equilibration.
(5) AAV2 eluted from each AVR10s column was detected at 350 nm fluorescence intensity against 280 nm excitation light, and the time at the top of the elution peak corresponding to AAV2 was defined as the elution time of the AAV2.

Table 15 shows the elution time of each AAV2 using the AVR10s column. In addition, FIG. 1 shows the results of plotting the infectivity (relative value to wild type AAV2 (SEQ ID NO: 99)) of each AAV2 on the vertical axis and the elution time of the AAV2 on the horizontal axis. AAV2s that were not adsorbed to the AVR10s immobilization gel and passed through the AVR10s column (AAV2 G383A, AAV2 H271F, and AAV2 D529A) were excluded from the plot in FIG. 1.

From Table 15 and FIG. 1, it is understood that AAV with strong infectivity has a delayed elution time (i.e., it has a strong binding force to the AVR10s immobilization gel). Therefore, it is understood that by analyzing AAV contained in a sample using an AAV adsorbent containing an insoluble carrier and a polypeptide comprising at least an amino acid sequence corresponding to PKD1 or PKD2 of KIAA0319L immobilized on the carrier, the strength of infectivity can be analyzed.

### [Table 15]

**Table 15**

| AAV | Rate of infectivity to AAV2 (wild type) [%] | AVR10s column elution time [min] |
|---|---|---|
| AAV2 S267T | 864 | 54.2 |
| AAV2 S267A | 378 | 52.9 |
| AAV2 R471A | 318 | 53.4 |
| AAV2(wild type) | 100 | 54.4 |
| AAV2 S264A | 72 | 53.7 |
| AAV2 E499A | 40 | 53.1 |
| AAV2 Q263A | 39 | 54.4 |
| AAV2 T503S | 26 | 48.0 |
| AAV2 S384A | 25 | 52.1 |
| AAV2 D529E | 20 | 47.0 |
| AAV2 Q385A | 14 | 50.9 |
| AAV2 H271A | 10 | 46.7 |
| AAV2 N382A | 3 | 46.4 |
| AAV2 H271Q | 2 | 46.4 |
| AAV2 D528A | 2 | 47.5 |
| AAV2 H271T | 0.6 | 42.6 |
| AAV2 G383A | 0.1 | Not detected |
| AAV2 H271F | 0.02 | Not detected |
| AAV2 D529A | 0.002 | Not detected |

| | | |
|---|---|---|
| 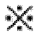 Not detected: No elution peak corresponding to AAV detected | | |

### Comparative Example 1: AAV Analysis using AAV Adsorbent (Part 2)

AAV2 was analyzed in the same manner as in Example 18, except that 1.25 mL of a POROS Capture Select AAVX Affinity Resin (manufactured by Thermo Fisher Scientific), which is an AAV adsorbent different from the AVR10s immobilization gel, was used as the gel packed into the stainless steel columns in Example 18 (1).

Table 16 shows the elution time of each AAV2 using a stainless steel column (hereinafter also referred to as "AAVX column") packed with the POROS Capture Select AAVX Affinity Resin. In addition, FIG. 2 shows the results of plotting the infectivity (relative value to wild type AAV2 (SEQ ID NO: 99)) of each AAV2 on the vertical axis and the elution time of the AAV2 on the horizontal axis.

It is understood from Table 16 and FIG. 2 that AAV2 is eluted at a nearly constant elution time regardless of the strength of infectivity. It is understood from the above results that the AAVX column cannot be used to analyze AAV based on the strength of infectivity.

### [Table 16]

**Table 16**

| AAV | Rate of infectivity to AAV2 (wild type) [%] | AAVX column elution time [min] |
|---|---|---|
| AAV2 S267T | 864 | 48.0 |
| AAV2 S267A | 378 | 48.0 |
| AAV2 R471A | 318 | 48.1 |
| AAV2(wild type) | 100 | 48.2 |
| AAV2 S264A | 72 | 48.0 |
| AAV2 E499A | 40 | 47.8 |
| AAV2 Q263A | 39 | 48.0 |
| AAV2 T503S | 26 | 48.0 |
| AAV2 S384A | 25 | 48.0 |
| AAV2 D529E | 20 | 48.0 |
| AAV2 Q385A | 14 | 47.9 |
| AAV2 H271A | 10 | 48.2 |
| AAV2 N382A | 3 | 47.9 |
| AAV2 H271Q | 2 | 48.1 |
| AAV2 D528A | 2 | 47.8 |
| AAV2 H271T | 0.6 | 47.9 |
| AAV2 G383A | 0.1 | 48.1 |
| AAV2 H271F | 0.02 | 48.11 |
| AAV2 D529A | 0.002 | 47.8 |

### Example 19: Preparation of AAV-Binding Protein

AAV-binding proteins were prepared in the same manner as in Example 16, except that one of the following was used as a plasmid containing a polynucleotide encoding a polypeptide containing an AAV-binding protein:
plasmid pET-AVR11a containing a polynucleotide (SEQ ID NO: 101) encoding a polypeptide comprising the AAV-binding protein AVR11a consisting of the amino acid sequence set forth in SEQ ID NO: 100; and
plasmid pET-AVR21 containing a polynucleotide (SEQ ID NO: 103) encoding a polypeptide comprising the AAV-binding protein AVR21 consisting of the amino acid sequence set forth in SEQ ID NO: 102.

A PelB signal peptide ranges from methionine (Met) at position 1 to alanine (Ala) at position 22, an AAV-binding protein (AVR11a or AVR21) ranges from serine (Ser) at position 25 to aspartic acid (Asp) at position 213, and a cysteine tag sequence serving as an immobilization tag ranges from cysteine (Cys) at position 220 to glycine (Gly) at position 226 in SEQ ID NOS: 100 and 102. AVR11a is a polypeptide with the following amino acid substitutions <I> to <XI> in the extracellular domain 1 (PKD1) and the domain 2 (PKD2) (amino acid residue from serine at position 312 to aspartic acid at position 500 of SEQ ID NO: 94) of AAV receptor KIAA0319L, and AVR21 is a polypeptide with the following amino acid substitutions <I> to <XXI> in PKD1 and PKD2 above:
<I> a substitution of valine with aspartic acid at position 317 of SEQ ID NO: 94 (position 30 of SEQ ID NOS: 100 and 102),
<II> a substitution of tyrosine with serine at position 342 of SEQ ID NO: 94 (position 55 of SEQ ID NOS: 100 and 102),
<III> a substitution of lysine with glutamic acid at position 362 of SEQ ID NO: 94 (position 75 of SEQ ID NOS: 100 and 102),
<IV> a substitution of lysine with asparagine at position 371 of SEQ ID NO: 94 (position 84 of SEQ ID NOS: 100 and 102),
<V> a substitution of valine with alanine at position 381 of SEQ ID NO: 94 (position 94 of SEQ ID NOS: 100 and 102),
<VI> a substitution of isoleucine with valine at position 382 of SEQ ID NO: 94 (position 95 of SEQ ID NOS: 100 and 102),
<VII> a substitution of glycine with serine at position 390 of SEQ ID NO: 94 (position 103 of SEQ ID NOS: 100 and 102),
<VIII> a substitution of lysine with glutamic acid at position 399 of SEQ ID NO: 94 (position 112 of SEQ ID NOS: 100 and 102),
<IX> a substitution of serine with arginine at position 476 of SEQ ID NO: 94 (position 189 of SEQ ID NOS: 100 and 102),
<X> a substitution of asparagine with aspartic acid at position 487 of SEQ ID NO: 94 (position 200 of SEQ ID NOS: 100 and 102).
(XI) a substitution of alanine with valine at position 330 of SEQ ID NO: 94 (position 43 of SEQ ID NOS: 100 and 102).
<XII> a substitution of asparagine with histidine at position 324 of SEQ ID NO: 94 (position 37 of SEQ ID NO: 102),
<XIII> a substitution of valine with alanine at position 326 of SEQ ID NO: 94 (position 39 of SEQ ID NO: 102),
<XIV> a substitution of glutamine with leucine at position 334 of SEQ ID NO: 94 (position 47 of SEQ ID NO: 102),
<XV> a substitution of glutamic acid with valine at position 335 of SEQ ID NO: 94 (position 48 of SEQ ID NO: 102),
<XVI> a substitution of threonine with alanine at position 341 of SEQ ID NO: 94 (position 54 of SEQ ID NO: 102),
<XVII) a substitution of phenylalanine with tyrosine at position 379 of SEQ ID NO: 94 (position 92 of SEQ ID NO: 102),
<XVIII> a substitution of lysine with arginine at position 380 of SEQ ID NO: 94 (position 93 of SEQ ID NO: 102),
<XIX> a substitution of lysine with glutamine at position 467 of SEQ ID NO: 94 (position 180 of SEQ ID NO: 102),
<XX> a substitution of serine with threonine at position 482 of SEQ ID NO: 94 (position 195 of SEQ ID NO: 102), and
<XXI> a substitution of asparagine with aspartic acid at position 492 of SEQ ID NO: 94 (position 205 of SEQ ID NO: 102).

### Example 20: Preparation of AAV Adsorbent (Part 2)

An AAV adsorbent was prepared in the same manner as in Example 17, except that AAV1 1a or AAV21 prepared in Example 19 was used as the AAV-binding protein. The AAV adsorbent with immobilized AVR11a is named "AVR11a immobilization gel," and the AAV adsorbent with immobilized AVR21 is named "AVR21 immobilization gel."

### Example 21: AAV Analysis using AAV Adsorbent (Part 3)

(1) Stainless steel columns (ϕ4.6 mm × 75 mm, manufactured by Tosoh Corporation) were each packed with 1.25 mL of the AAV adsorbent (AVR11a immobilization gel or AVR21 immobilization gel) prepared in Example 20, thereby preparing columns. The column packed with the AVR11a immobilization gel is named "AVR11a column," and the column packed with the AVR21 immobilization gel is named "AVR21 column."
(2) AAV2 was analyzed in the same manner as in Example 18 (2) to (5), except that the AVR11a column or AVR21 column was used as the column, 10 mM glycine buffer (pH 6.0) containing 50 mM calcium chloride and 15 mM sodium acetate was used as the equilibration solution C, and 10 mM glycine buffer (pH 2.0) containing 50 mM calcium chloride and 15 mM sodium acetate was used as the eluate A.

Table 17 shows the elution time of each AAV2 using the AVR11a column and AAV21 column. In addition, FIGS. 3 and 4 show the results of plotting the infectivity (relative value to wild type AAV2 (SEQ ID NO: 99)) of each AAV2 on the vertical axis and the elution time of the AAV2 on the horizontal axis. AAV2s that were not adsorbed to the AVR11a immobilization gel and passed through the AVR11a column (AAV2 H271A, AAV2 H271Q, AAV2 H271T, AAV2 G383A, AAV2 H271F, and AAV2 D529A) were excluded from the plot in FIG. 3. AAV2s that were not adsorbed to the AVR21 immobilization gel and passed through the AVR21 column (AAV2 G383A, AAV2 H271F, and AAV2 D529A) were excluded from the plot in FIG. 4.

From Table 17 and FIGS. 3 and 4, it is understood that AAV with strong infectivity has a delayed elution time (i.e., it has a strong binding force to the AVR11a immobilization gel or the AVR21 immobilization gel). Therefore, it is understood that by analyzing AAV contained in a sample using an AAV adsorbent containing an insoluble carrier and a polypeptide comprising at least an amino acid sequence corresponding to PKD1 or PKD2 of KIAA0319L immobilized on the carrier, the strength of infectivity can be analyzed.

### [Table 17]

**Table 17**

| AAV | Rate of infectivity to AAV2 (wild type) [%] | AVR11a column elution time [min] | AVR21 column elution time [min] |
|---|---|---|---|
| AAV2 S267T | 864 | 59.2 | 60.5 |
| AAV2 S267A | 378 | 59.4 | 61.2 |
| AAV2 R471A | 318 | 60.3 | 61.7 |
| AAV2(wild type) | 100 | 59.8 | 60.9 |
| AAV2 S264A | 72 | 59.6 | 61.3 |
| AAV2 E499A | 40 | 59.3 | 60.9 |
| AAV2 Q263A | 39 | 60.2 | 61.6 |
| AAV2 T503S | 26 | 55.2 | 58.5 |
| AAV2 S384A | 25 | 58.6 | 60.3 |
| AAV2 D529E | 20 | 54.5 | 56.8 |
| AAV2 Q385A | 14 | 57.7 | 59.1 |
| AAV2 H271A | 110 | Not detected | 57.1 |
| AAV2 N382A | 3 | 53.5 | 57.3 |
| AAV2 H271Q | 2 | Not detected | 56.0 |
| AAV2 D528A | 2 | 55.5 | 58.0 |
| AAV2 H271T | 0.6 | Not detected | 55.9 |
| AAV2 G383A | 0.1 | Not detected | Not detected |
| AAV2 H271F | 0.02 | Not detected | Not detected |
| AAV2 D529A | 0.002 | Not detected | Not detected |

| | | | |
|---|---|---|---|
| 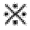 Not detected: No elution peak corresponding to AAV detected | | | |

### Comparative Example 2: AAV Analysis using AAV Adsorbent (Part 4)

AAV2 was analyzed in the same manner as in Example 20, except that 1.25 mL of a POROS Capture Select AAVX Affinity Resin (manufactured by Thermo Fisher Scientific), which is an AAV adsorbent different from AVR11a immobilization gel and AVR21 immobilization gel, was used as the gel packed into the stainless steel columns in Example 21 (1).

Table 18 shows the elution time of each AAV2 using a stainless steel column (hereinafter also referred to as "AAVX column") packed with the POROS Capture Select AAVX Affinity Resin. In addition, FIG. 5 shows the results of plotting the infectivity (relative value to wild type AAV2 (SEQ ID NO: 99)) of each AAV2 on the vertical axis and the elution time of the AAV2 on the horizontal axis.

It is understood from Table 18 and FIG. 5 that AAV2 is eluted at a nearly constant elution time regardless of the strength of infectivity. It is understood from the above results that the AAVX column cannot be used to analyze AAV based on the strength of infectivity.

### [Table 18]

**Table 18**

| AAV | Rate of infectivity to AAV2 (wild type) [%] | AAVX column elution time [min] |
|---|---|---|
| AAV2 S267T | 864 | 59.2 |
| AAV2 S267A | 378 | 59.,3 |
| AAV2 R471A | 318 | 59.4 |
| AAV2(wild type) | 100 | 59.3 |
| AAV2 S264A | 72 | 59.2 |
| AAV2 E499A | 40 | 59.3 |
| AAV2 Q263A | 39 | 59.3 |
| AAV2 T503S | 26 | 59.4 |
| AAV2 S384A | 25 | 59.3, |
| AAV2 D529E | 20 | 59.3 |
| AAV2 Q385A | 14 | 59.3, |
| AAV2 H271A | 10 | 59.4 |
| AAV2 N382A | 3 | 59.2 |
| AAV2 H271Q | 2 | 59.3 |
| AAV2 D528A | 2 | 59.1 |
| AAV2 H271T | 0.6 | 59.3 |
| AAV2 G383A | 0.1 | 59.3 |
| AAV2 H271F | 0.02 | 59.3 |
| AAV2 D529A | 0.002 | 59.3 |

## Claims

1. An adeno-associated virus (AAV)-binding protein, which is selected from any of the following (i) to (iii):
(i) an AAV-binding protein comprising at least amino acid residues from serine at position 312 to aspartic acid at position 500 of the amino acid sequence set forth in SEQ ID NO: 1, wherein at least any one of the following amino acid substitutions (1) to (5) is present at the amino acid residues from position 312 to position 500;
(1) a substitution of alanine with valine at position 330 of SEQ ID NO: 1,
(2) a substitution of alanine with any one of cysteine, phenylalanine, leucine, arginine, tryptophan, or tyrosine at position 330 of SEQ ID NO: 1,
(3) a substitution of tyrosine with cysteine at position 331 of SEQ ID NO: 1,
(4) a substitution of valine with tryptophan or tyrosine at position 332 of SEQ ID NO: 1,
(5) a substitution of leucine with cysteine at position 333 of SEQ ID NO: 1;
(ii) an AAV-binding protein comprising at least amino acid residues from serine at position 312 to aspartic acid at position 500 of the amino acid sequence set forth in SEQ ID NO: 1, wherein the at least any one of the amino acid substitutions (1) to (5) is present at the amino acid residues from position 312 to position 500, wherein one or more substitutions, deletions, insertions, and additions of one or several amino acid residues are further present at one or several positions other than the amino acid substitutions set forth in (1) to (5), and wherein the AAV-binding protein has AAV-binding activity; and
(iii) an AAV-binding protein comprising an amino acid sequence having 70% or more homology to an entire amino acid sequence in which the at least any one of the amino acid substitutions (1) to (5) is present in an amino acid sequence ranging from serine at position 312 to aspartic acid at position 500 of the amino acid sequence set forth in SEQ ID NO: 1, wherein the at least any one of the amino acid substitutions remains in the amino acid sequence, and wherein the AAV-binding protein has AAV-binding activity.

2. The AAV-binding protein according to claim 1, which is selected from any of the following (iv) to (vi):
(iv) an AAV-binding protein comprising at least amino acid residues from serine at position 312 to aspartic acid at position 500 of the amino acid sequence set forth in SEQ ID NO: 1, wherein at least the following amino acid substitution (1) is present at the amino acid residues from position 312 to position 500:
(1) a substitution of alanine with valine at position 330 of SEQ ID NO: 1;
(v) an AAV-binding protein comprising at least amino acid residues from serine at position 312 to aspartic acid at position 500 of the amino acid sequence set forth in SEQ ID NO: 1, wherein the at least amino acid substitution (1) is present at the amino acid residues from position 312 to position 500, wherein one or more substitutions, deletions, insertions, and additions of one or several amino acid residues are further present at one or several positions other than the amino acid substitution set forth in (1), and wherein the AAV-binding protein has AAV-binding activity; and
(vi) an AAV-binding protein comprising an amino acid sequence having 70% or more homology to an entire amino acid sequence in which the at least the amino acid substitution (1) is present in an amino acid sequence ranging from serine at position 312 to aspartic acid at position 500 of the amino acid sequence set forth in SEQ ID NO: 1, wherein the amino acid substitution (1) remains in the amino acid sequence, and wherein the AAV-binding protein has AAV-binding activity.

3. The AAV-binding protein according to claim 1, wherein the following 10 amino acid substitutions are further present:
a substitution of valine with aspartic acid at position 317 of SEQ ID NO: 1,
a substitution of tyrosine with serine at position 342 of SEQ ID NO: 1,
a substitution of lysine with glutamic acid at position 362 of SEQ ID NO: 1,
a substitution of lysine with asparagine at position 371 of SEQ ID NO: 1,
a substitution of valine with alanine at position 381 of SEQ ID NO: 1,
a substitution of isoleucine with valine at position 382 of SEQ ID NO: 1,
a substitution of glycine with serine at position 390 of SEQ ID NO: 1,
a substitution of lysine with glutamic acid at position 399 of SEQ ID NO: 1,
a substitution of serine with arginine at position 476 of SEQ ID NO: 1, and
a substitution of asparagine with aspartic acid at position 487 of SEQ ID NO: 1.

4. The AAV-binding protein according to claim 3, which is selected from any of the following (vii) to (ix):
(vii) an AAV-binding protein comprising at least amino acid residues from serine at position 25 to aspartic acid at position 213 of the amino acid sequence set forth in any one of SEQ ID NOS: 15 to 25;
(viii) an AAV-binding protein comprising at least amino acid residues from serine at position 25 to aspartic acid at position 213 of the amino acid sequence set forth in any one of SEQ ID NOS: 15 to 25, wherein one or more substitutions, deletions, insertions, and additions of one or several amino acid residues are further present at one or several positions other than said amino acid substitution(s) of said amino acid sequence in the amino acid residues from position 25 to position 213, and wherein the AAV-binding protein has AAV-binding activity; and
(ix) an AAV-binding protein comprising at least amino acid residues from serine at position 25 to aspartic acid at position 213 of the amino acid sequence set forth in any one of SEQ ID NOS: 15 to 25, except for having 70% or more homology to said amino acid sequence from serine at position 25 to aspartic acid at position 213, wherein said amino acid substitution(s) of said amino acid sequence remain, and wherein the AAV-binding protein has AAV-binding activity.

5. The AAV-binding protein according to claim 3, wherein at least any one of the following amino acid substitutions (I) to (LXXXVII) is further present:
(I) a substitution of serine with proline at position 312 of SEQ ID NO: 1,
(II) a substitution of alanine with valine at position 313 of SEQ ID NO: 1,
(III) a substitution of glutamic acid with glycine or valine at position 315 of SEQ ID NO: 1,
(IV) a substitution of glutamine with arginine at position 318 of SEQ ID NO: 1,
(V) a substitution of isoleucine with valine at position 319 of SEQ ID NO: 1,
(VI) a substitution of lysine with methionine or asparagine at position 323 of SEQ ID NO: 1,
(VII) a substitution of asparagine with histidine at position 324 of SEQ ID NO: 1,
(VIII) a substitution of glutamic acid with lysine at position 325 of SEQ ID NO: 1,
(IX) a substitution of valine with glutamic acid or alanine at position 326 of SEQ ID NO: 1,
(X) a substitution of glutamine with leucine at position 327 of SEQ ID NO: 1,
(XI) a substitution of leucine with any one of glutamine, arginine and proline at position 328 of SEQ ID NO: 1,
(XII) a substitution of asparagine with tyrosine or serine at position 329 of SEQ ID NO: 1,
(XIII) a substitution of valine with glutamic acid or alanine at position 332 of SEQ ID NO: 1,
(XIV) a substitution of leucine with proline or methionine at position 333 of SEQ ID NO: 1,
(XV) a substitution of glutamine with leucine or histidine at position 334 of SEQ ID NO: 1,
(XVI) a substitution of glutamic acid with valine at position 335 of SEQ ID NO: 1,
(XVII) a substitution of proline with glutamine at position 337 of SEQ ID NO: 1,
(XVIII) a substitution of lysine with glutamic acid or arginine at position 338 of SEQ ID NO: 1,
(XIX) a substitution of glycine with glutamic acid at position 339 of SEQ ID NO: 1,
(XX) a substitution of glutamic acid with aspartic acid at position 340 of SEQ ID NO: 1,
(XXI) a substitution of threonine with alanine at position 341 of SEQ ID NO: 1,
(XXII) a substitution of threonine with any one of proline, alanine and serine at position 343 of SEQ ID NO: 1,
(XXIII) a substitution of aspartic acid with glycine at position 345 of SEQ ID NO: 1,
(XXIV) a substitution of isoleucine with threonine at position 349 of SEQ ID NO: 1,
(XXV) a substitution of threonine with alanine at position 350 of SEQ ID NO: 1,
(XXVI) a substitution of aspartic acid with valine at position 354 of SEQ ID NO: 1,
(XXVII) a substitution of tyrosine with histidine at position 355 of SEQ ID NO: 1,
(XXVIII) a substitution of methionine with any one of isoleucine, leucine and valine at position 359 of SEQ ID NO: 1,
(XXIX) a substitution of glutamic acid with aspartic acid at position 360 of SEQ ID NO: 1,
(XXX) a substitution of glycine with glutamic acid at position 361 of SEQ ID NO: 1,
(XXXI) a substitution of isoleucine with threonine or proline at position 366 of SEQ ID NO: 1,
(XXXII) a substitution of leucine with valine at position 367 of SEQ ID NO: 1,
(XXXIII) a substitution of lysine with any one of asparagine, arginine and glutamic acid at position 368 of SEQ ID NO: 1,
(XXXIV) a substitution of leucine with glutamine or proline at position 369 of SEQ ID NO: 1,
(XXXV) a substitution of leucine with proline at position 376 of SEQ ID NO: 1,
(XXXVI) a substitution of phenylalanine with tyrosine at position 379 of SEQ ID NO: 1,
(XXXVII) a substitution of lysine with asparagine or arginine at position 380 of SEQ ID NO: 1,
(XXXVIII) a substitution of glutamic acid with valine at position 384 of SEQ ID NO: 1,
(XXXIX) a substitution of alanine with threonine at position 388 of SEQ ID NO: 1,
(XL) a substitution of histidine with any one of asparagine, aspartic acid, arginine and leucine at position 389 of SEQ ID NO: 1,
(XLI) a substitution of glycine with arginine at position 392 of SEQ ID NO: 1,
(XLII) a substitution of valine with alanine or aspartic acid at position 394 of SEQ ID NO: 1,
(XLII) a substitution of asparagine with aspartic acid at position 395 of SEQ ID NO: 1,
(XI,III) a substitution of valine with glycine at position 396 of SEQ ID NO: 1,
(XLIV) a substitution of valine with alanine at position 398 of SEQ ID NO: 1,
(XLV) a substitution of arginine with serine or histidine at position 403 of SEQ ID NO: 1,
(XLVI) a substitution of lysine with alanine at position 404 of SEQ ID NO: 1,
(XLVII) a substitution of arginine with serine at position 406 of SEQ ID NO: 1,
(XLVIII) a substitution of isoleucine with asparagine at position 409 of SEQ ID NO: 1,
(XLIX) a substitution of isoleucine with threonine or leucine at position 411 of SEQ ID NO: 1,
(L) a substitution of valine with isoleucine at position 412 of SEQ ID NO: 1,
(LI) a substitution of phenylalanine with serine at position 416 of SEQ ID NO: 1,
(LII) a substitution of isoleucine with phenylalanine or valine at position 419 of SEQ ID NO: 1,
(LIII) a substitution of serine with threonine at position 420 of SEQ ID NO: 1,
(LIV) a substitution of leucine with proline at position 421 of SEQ ID NO: 1,
(LV) a substitution of proline with serine at position 422 of SEQ ID NO: 1,
(LVI) a substitution of threonine with alanine at position 423 of SEQ ID NO: 1,
(LVII) a substitution of serine with glycine at position 425 of SEQ ID NO: 1,
(LVIII) a substitution of isoleucine with valine at position 428 of SEQ ID NO: 1,
(LIX) a substitution of glycine with alanine at position 430 of SEQ ID NO: 1,
(LX) a substitution of threonine with alanine at position 434 of SEQ ID NO: 1,
(LXI) a substitution of aspartic acid with asparagine at position 437 of SEQ ID NO: 1,
(LXII) a substitution of valine with alanine at position 440 of SEQ ID NO: 1,
(LXIII) a substitution of histidine with tyrosine or arginine at position 443 of SEQ ID NO: 1,
(LXIV) a substitution of glutamic acid with aspartic acid at position 446 of SEQ ID NO: 1,
(LXV) a substitution of lysine with glutamic acid or asparagine at position 448 of SEQ ID NO: 1,
(LXVI) a substitution of leucine with isoleucine at position 451 of SEQ ID NO: 1,
(LXVII) a substitution of glutamic acid with lysine at position 453 of SEQ ID NO: 1,
(LXVIII) a substitution of glutamic acid with glycine at position 454 of SEQ ID NO: 1,
(LXIX) a substitution of lysine with glutamic acid or arginine at position 455 of SEQ ID NO: 1,
(LXX) a substitution of lysine with asparagine or glutamic acid at position 464 of SEQ ID NO: 1,
(LXXI) a substitution of lysine with glutamine or glutamic acid at position 467 of SEQ ID NO: 1,
(LXXII) a substitution of valine with glutamic acid at position 469 of SEQ ID NO: 1,
(LXXIII) a substitution of glycine with cysteine at position 471 of SEQ ID NO: 1,
(LXXIV) a substitution of asparagine with tyrosine or aspartic acid at position 472 of SEQ ID NO: 1,
(LXXV) a substitution of tyrosine with histidine at position 473 of SEQ ID NO: 1,
(LXXVI) a substitution of threonine with alanine at position 478 of SEQ ID NO: 1,
(LXXVII) a substitution of valine with alanine at position 479 of SEQ ID NO: 1,
(LXXVIII) a substitution of valine with aspartic acid or alanine at position 480 of SEQ ID NO: 1,
(LXXIX) a substitution of serine with threonine at position 482 of SEQ ID NO: 1,
(LXXX) a substitution of glycine with serine at position 484 of SEQ ID NO: 1,
(LXXXI) a substitution of threonine with serine at position 486 of SEQ ID NO: 1,
(LXXXII) a substitution of serine with threonine or phenylalanine at position 488 of SEQ ID NO: 1,
(LXXXIII) a substitution of threonine with alanine or serine at position 489 of SEQ ID NO: 1,
(LXXXIV) a substitution of alanine with serine at position 491 of SEQ ID NO: 1,
(LXXXV) a substitution of asparagine with aspartic acid at position 492 of SEQ ID NO: 1,
(LXXXVI) a substitution of asparagine with tyrosine or aspartic acid at position 496 of SEQ ID NO: 1, and
(LXXXVII) a substitution of aspartic acid with glycine at position 500 of SEQ ID NO: 1.

6. The AAV-binding protein according to claim 5, which is selected from any of the following (x) to (xii):
(x) an AAV-binding protein comprising at least amino acid residues from serine at position 25 to aspartic acid at position 213 of the amino acid sequence set forth in any one of SEQ ID NOS: 54 to 63, 65, 83, 85, 87, 88, and 90 to 92;
(xi) an AAV-binding protein comprising at least amino acid residues from serine at position 25 to aspartic acid at position 213 of the amino acid sequence set forth in any one of SEQ ID NOS: 54 to 63, 65, 83, 85, 87, 88, and 90 to 92, wherein one or more substitutions, deletions, insertions, and additions of one or several amino acid residues are further present at one or several positions other than said amino acid substitution(s) of said amino acid sequence in the amino acid residues from position 25 to position 213, and wherein the AAV-binding protein has AAV-binding activity; and
(xii) an AAV-binding protein comprising at least amino acid residues from serine at position 25 to aspartic acid at position 213 of the amino acid sequence set forth in any one of SEQ ID NOS: 54 to 63, 65, 83, 85, 87, 88, and 90 to 92, except for having 70% or more homology to said amino acid sequence from serine at position 25 to aspartic acid at position 213, wherein said amino acid substitution(s) of said amino acid sequence remain, and wherein the AAV-binding protein has AAV-binding activity.

7. A polynucleotide encoding the AAV-binding protein according to any of claims 1 to 6.

8. An expression vector comprising the polynucleotide according to claim 7.

9. A transformant obtained by transforming *Escherichia coli* with the expression vector according to claim 8.

10. A method for producing an AAV-binding protein, comprising a step of allowing an AAV-binding protein to be expressed by culturing the transformant according to claim 9 and a step of recovering the AAV-binding protein expressed from the obtained culture.

11. An AAV adsorbent comprising an insoluble carrier and the AAV-binding protein according to any one of claims 1 to 6 which is immobilized on the carrier.

12. A column comprising the AAV adsorbent according to claim 11.

13. A method for purifying AAV, comprising a step of adding a solution containing AAV to the column according to claim 12 to allow the AAV to be adsorbed by said adsorbent and a step of eluting the AAV adsorbed by said adsorbent using an eluate.

14. A method for analyzing AAV contained in a sample based on the strength of the infectivity, comprising a step of adding the sample containing AAV to the column according to claim 12 to allow the AAV to be adsorbed by said adsorbent, and a step of eluting the AAV adsorbed by said adsorbent using an eluate.
